# FASCICULE DE BREVET EUROPEEN

(11) **EP 0 571 253 B1**
(45) Date de publication et mention de la délivrance du brevet: **04.11.1998**
(21) Numéro de dépôt: 93401239.4
(22) Date de dépôt: 14.05.1993
(51) Int. Cl.: C07D 487/04, A61K 31/415, A61K 31/505

(54) **Dérivés du benzimidazole à activité antidiabétique et antiagrégante plaquettaire**
Benzimidezolderivate mit antidiabetischer und Anti-Plättchenklumpungswirkung
Benzimidazole derivatives with antidiabetic and antiplatelet aggregation activity

(30) Priorité: 19.05.1992 FR 9206036; 31.07.1992 FR 9209488
(43) Date de publication de la demande: 24.11.1993
(73) Titulaire: ADIR ET COMPAGNIE, 92415 Courbevoie Cédex (FR)
(72) Inventeur: Anisimova, Vera Alekseevna, Rostov-on-Don, 344010 (RU); Levchenko, Margarita Valentinovna, Tostov-on-Don (RU); Korochina, Tatyana Borisovna, Rostov-on-Don, 344101 (RU); Spasov, Alexander Alexeyevich, F-92415 Courbevoie Cedex (RU); Kovalev, Sergei Gennadyevich, Volgograd, 400048 (RU); Dudchenko, Galina Petrovna, Volgograd, 400033 (RU)

(56) Documents cités:
- US-A- 3 989 709
- CHEMICAL ABSTRACTS, vol. 110, 1989, Columbus, Ohio, US; abstract no. 51270k, ANISIMOVA, V. A. ET AL. 'Synthesis and pharmacological activity of N-substituted 1-aminoethyl-2,3-dihydroimidazoÄ1,2-aÜbenz imidazoles' page 71 ;
- CHEMICAL ABSTRACTS, vol. 108, 1988, Columbus, Ohio, US; abstract no. 5879b, ANISIMOVA, V. A. ET AL. 'Synthesis and pharmacological activity of some 2,3-dihydroimidazoÄ1,2-aÜbenzimidazoles and their intermediates' page 558 ;
- CHEMICAL ABSTRACTS, vol. 114, 1991, Columbus, Ohio, US; abstract no. 156579d, A.A. SPAASOV ET AL. 'Effects of imidazo(1,2-a)benzimidazole derivatives on gastric secretion and the antiulcer action' page 14 ;
- CHEMICAL ABSTRACTS, vol. 99, 1983, Columbus, Ohio, US; abstract no. 212451s, V. A. ANISIMOVA ET AL. 'New method for the synthesis of 9-substituted derivatives of 2,3-dihydroimidazo(1,2-a)benzimidazole' page 638 ;
- CHEMICAL ABSTRACTS, vol. 70, 1969, Columbus, Ohio, US; abstract no. 96705c, P.M. KOCHERGIN ET AL. 'Imidazoles. XXX. Synthesis of derivatives of imidazolino(1,2-a)benzimidazole and tetrahydropyrimido(1,2-a)benzimidazole' page 340 ;
- CHEMICAL ABSTRACTS, vol. 88, 1978, Columbus, Ohio, US; abrégé no. 69332U, SIMONOV ET AL. 'Water-soluble salts of 2-substituted 9-diethylaminoethylimidazo[1,2-a]benzimidaz ole with hypotensive and antiphlogistic properties'

## Description

La présente invention concerne de nouveaux dérivés tricycliques du benzimidazole, leur procédé de préparation et les compositions pharmaceutiques qui les contiennent.

Des composés à structure imidazo[1,2-a]benzimidazole et tétrahydropyrimidino[1,2-a]benzimidazole sont décrits dans la littérature (Khim. Geterotsikl. Soedin., 1983, 3, 419 et 1967, Sb.1, 137) sans qu'aucunes propriétés pharmacologiques ne leur soit attribuées. On connait également des imidazo[1,2-a]benzimidazoles qui possèdent des propriétés antihypertensives (Othrytiya, Isobret., Prom. Obraztsy, Tovarnye Znaki, 1977, 54, 13) parfois associées à des effets dépresseurs du système nerveux central (Khim. Farm. Zh., 1979, 13, 87) ou bien inhibitrices d'AMP cyclique phosphodiestérase (Khim. Farm. Zh., 1987, 21, 313). De tels dérivés ont aussi été étudiés pour leur action antiulcéreuse (Farmakol. Toksikol., 1990, 53, 30) ou antidiabétique et antioxydante (Khim. Farm. Zh., 1988, 22, 1212).

La demanderesse a présentement découvert des dérivés tricycliques du benzimidazole qui possèdent simultanément une activité antidiabétique et une importante activité antiagrégante plaquettaire. L'association des activités antidiabétique et antiagrégante plaquettaire dans une seule et même molécule est à ce jour originale et particulièrement intéressante en thérapeutique. En effet, l'homme de métier sait qu'outre les problèmes métaboliques liés au diabète, le pronostic de cette maladie est aggravé par un risque important de complications vasculaires (Gooodman and Gilman's - The pharmacological basis of Therapeutics, 8th edition, p 1471). Celles-ci doivent faire l'objet d'une surveillance importante et d'un traitement séparé. Par leur activité antiagrégante plaquettaire inconnue actuellement chez des dérivés doués d'une activité antidiabétique, les dérivés de l'invention, tout en traitant les troubles métaboliques qui sont la cause du diabète, ont également un effet préventif et curatif des troubles cardiovasculaires qui en sont une conséquence inévitable. De par là même, les dérivés de l'invention réalisent un progrès décisif dans la thérapeutique du diabète par rapport à tous les antidiabétiques connus dans l'art antérieur. Enfin, il est important de signaler que les dérivés de la présente invention sont dépourvus d'activité sur la pression artérielle contrairement à ce que pourrait laisser supposer la structure β-phényléthylaminique de certains dérivés de l'invention ainsi que des résultats de l'art antérieur précédemment cités.

Plus particulièrement la présente invention concerne les dérivés de formule générale (I) : 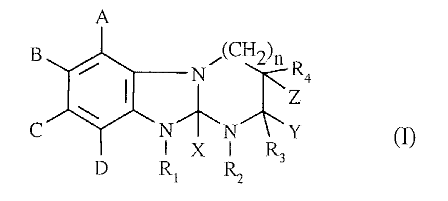 pour lesquels :
ou bien :
**1.**
   n = 0
   A, B, C, D identiques ou différents représentent un atome d'hydrogène, d'halogène, un groupement alkyle inférieur, alkoxy inférieur, hydroxyle, trifluorométhyle, ou hydroxyalkyle inférieur,
   Y et Z représentent chacun un atome d'hydrogène ou forment ensemble une liaison, et alors soit :
      **1.A**.
         X et R₂ forment ensemble une liaison, et dans ce cas alors :
         - R₁: représente un groupement G₁,
         G₁ représente le groupement 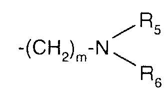 avec m nombre entier compris inclusivement entre 1 et 6 et R₅ et R₆ identiques ou différents représentent :
         a/ indépendamment l'un de l'autre un atome d'hydrogène, un groupement alkyle inférieur, un groupement arylalkyle inférieur, ou un groupement arylalkyle inférieur substitué ;
         b/ ou R₅ et R₆ forment simultanément avec l'atome d'azote qui les porte un système morpholinique, pipéridine, pyrrolidine ou pipérazine éventuellement substitué sur l'atome d'azote en 4 par un groupement alkyle inférieur, aryle, aryle substitué, arylalkyl inférieur, arylalkyl inférieur substitué,
         ou bien
         - R₁: représente un groupement G₂, G₂ signifiant un groupement (CH₂)ₘCOR₇ ou G₃, G₃ signifiant un groupement (CH₂)ₘCHOHR₇, m ayant la même signification que précédemment et R₇ signifiant un groupement aryle ou aryle substitué,
         - R₃: représente un atome d'hydrogène, un groupement alkyle inférieur, un noyau phényl substitué par un groupement hydroxy ou hydroxyalkyle ou par deux à cinq groupements choisis parmi alkyle inférieur, alkoxy inférieur, halogène, trifluorométhyle, hydroxy, hydroxyalkyle, ou bien R₃ représente un noyau naphtyle éventuellement subsitué, ou un groupement hétéroaryale éventuellement substitué,
         - R₄: représente un atome d'hydrogène, un groupement G₁ où G₁ a la même définition que précédemment, un groupement G₄, c'est-à-dire un groupement de formule :

         -COO-G₁

         où G₁ a la même définition que précédemment, ou un groupement G₅ c'est-à-dire -COR₉ où R₉ représente un groupement aryle, un groupement aryle substitué, un groupement hétéroaryle, un groupement hétéroaryle substitué,
         à l'exception des dérivés pour lesquels simultanément R₄ représente un atome d'hydrogène et R₁ représente un groupement: 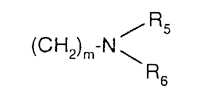 avec m = 2,
         et R₅ et R₆ représentent simultanément un groupement éthyle tandis que R₃ représente un groupement alkyle inférieur ou naphtyle,
         et des dérivés pour lesquels R₅ et R₆ forment simultanément avec l'atome d'azote qui les porte un système pipéridine, pyrrolidine ou pipérazine éventuellement substitué sur l'azote en 4 par un groupement alkyle inférieur, aryle, arylalkyle inférieur, aryle substitué, arylalkyle inférieur substitué tandis que R₃ représente un atome d'hydrogène.
         ou
      **1.B**
         X et R₁ forment ensemble une liaison,
         R₂ représente un groupement G₁, G₂ ou G₃ avec G₁, G₂ ou G₃ ayant la même définition que précédemment, et alors R₃ représente un atome d'hydrogène, un groupement alkyle inférieur, un groupement phényle substitué, un groupement naphtyle, un groupement naphtyle substitué, un groupement hétéroaryle, un groupement hétéroaryle substitué, tandis que R₄ représente un atome d'hydrogène ou un groupement G₁, G₄ ou G₅ où G₄ et G₅ ont la même définition que précédemment,
         étant entendu que lorsque R₂ représente un groupement G₁, G₁ ayant la même définition que précédemment, alors R₃ est différent d'un atome d'hydrogène,
   ou
**2.**
   n = 1,
   A, B, C, D identiques ou différents représentent un atome d'hydrogène, d'halogène, un groupement alkyle inférieur ou trifluorométhyle,
      avec X et R₁ forment une liaison ou bien X et R₂ forment une liaison l'une au moins de ces deux liaisons devant être présente dans la molécule,
      Y et Z représentent chacun un atome d'hydrogène, ou forment ensemble une liaison,
   R₁ ou R₂ - selon que X et R₂ ou X et R₁ forment une liaison - représentent alors un groupement méthyle ou G₁ ou G₂ ou G₃, ou G₁, G₂ et G₃ ayant la même définition que précédemment,
   R₃ représente, quelles que soient les valeurs de R₁ ou R₂, un atome d'hydrogène, un groupement alkyle inférieur, un groupement aryle, un groupement aryle substitué, un groupement hétéroaryle, un groupement hétéroaryle substitué,
   et R₄ représente un atome d'hydrogène ou un groupement G₁ ou G₄ ou G₅, G₁, G₄ et G₅ gardant la même définition que précédemment,
      leurs isomères, énantiomères, diastéréoisomères, ainsi que leurs sels d'addition à un acide pharmaceutiquement acceptable,
      étant entendu que par groupement alkyle inférieur ou alkoxy inférieur, on entend des groupements linéaire ou ramifié comprenant de 1 à 6 atomes de carbone,
      que par groupement aryle, on entend un groupement phényle ou naphtyle, que par groupement hétéroaryle, on entend un groupement furyle, thiényle, pyridyle, pyrrolyle, imidazolyle, benzimidazolyle, benzofuryle, benzothiényle, quinolyle, isoquinolyle, ou indolyle,
      que le terme substitué affectant les expressions arylalkyle, aryle, phényle, naphtyle, hétéroaryle signifie, sauf précision contraire, que les groupements concernés sont substitués sur leur partie cyclique par un à trois radicaux choisis parmi halogène, trifluorométhyle, hydroxy, alkoxy inférieur, hydroxy alkyle inférieur, alkyle inférieur,
      et que s'il existe dans une même molécule plusieurs groupements G₁ ou groupements R₅ et R₆, ceux-ci peuvent être identiques ou différents.

Parmi les acides pharmaceutiquement acceptables que l'on peut utiliser pour former un sel d'addition des composés de formule (I), on peut citer à titre non limitatif, les acides chlorhydrique, sulfurique, tartrique, maléique, fumarique, oxalique, éthane sulfonique, camphonique, éthane sulfonique, citrique, etc...

L'invention s'étend aussi au procédé de préparation des dérivés de formule générale (I) caractérisé :
**A** - lorsque dans le dérivé de formule (I) que l'on souhaite obtenir, X et R₂ forment ensemble une liaison, en ce que l'on utilise comme matière première un dérivé de formule (II) : 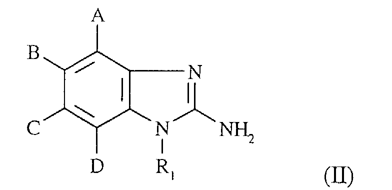 où A, B, C, D et R₁ ont la même définition que précédemment,
   dérivé de formule (II) obtenu comme décrit dans la littérature, (citons, à titre d'exemple et de façon non limitative, Tetrahedron 1976, 32(7), 839-842 ou Khim Heterosikl, Soedin 1969(5), 869-873),
   que l'on traite :
   * lorsque dans le dérivé de formule (I) que l'on souhaite obtenir Y et Z représentent chacun un atome d'hydrogène, par un dérivé de formule (III/A) :

      Hal_{A} - (CH₂)ₙ - CHR₄- CHR₃ - P (III/A)

      où Hal_{A} représente un atome d'halogène, n, R₃ et R₄ ont la même définition que précédemment et P représentant un groupe partant choisi parmi halogène ou hydroxyle,
      pour conduire à un dérivé de formule (IV/A): 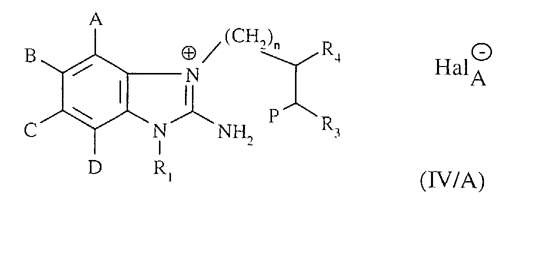 où A, B, C, D, R₁ ont la même définition que dans la formule (I) et n, R₃, R₄ et P ont la même définition que précédemment,
      qui, par chauffage, précédé, lorsque P représente un groupement hydroxyle par un traitement par un agent d'halogénation,
      conduit à un dérivé de formule (I/B): 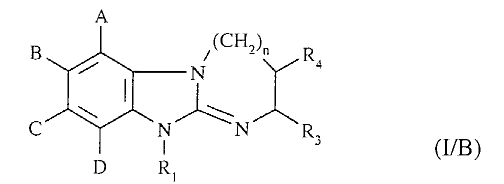 cas particulier des dérivés de formule (I) pour lesquels A, B, C, D, R₁, R₃, R₄ et n ont la même définition que précédemment,
      X et R₂ forment ensemble une liaison et Y et Z représentent chacun un atome d'hydrogène,
   * lorsque dans le dérivé de formule (I) que l'on souhaite obtenir Y et Z forment ensemble une liaison,
      par un dérivé de formule (III) 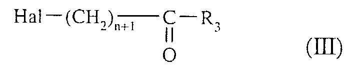 où n a la même définition que précédemment et R₃ la même définition que dans la formule (I) en fonction des valeurs de R₁ et Hal représente un atome d'halogène,
      pour conduire à un dérivé de formule (IV) : 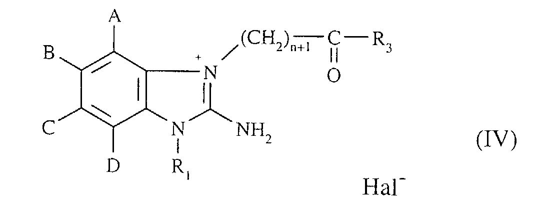 où Hal, A, B, C, D, R₁, n, R₃ ont la même définition que précédemment,
      qui, par chauffage, conduit à un dérivé de formule (I/A) : 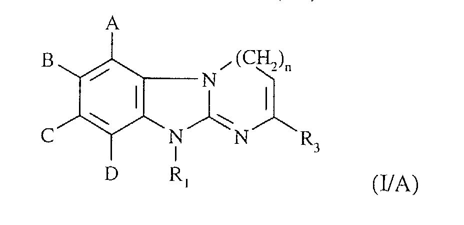 cas particulier des dérivés de formule (I) pour lesquels A, B, C, D, R₁, R₃ et n ont la même définition que précédemment, et X et R₂ d'une part, et Y et Z d'autre part forment ensemble une liaison et R₄ représente un atome d'hydrogène,
      dérivé de formule (I/A) qui, lorsque dans le dérivé de formule (I) que l'on souhaite obtenir, R₄ est différent de l'hydrogène est traité :
      - lorsque dans le dérivé de formule (I) que l'on souhaite obtenir, R₄ représente un groupement G₄ et Y et Z représentent une liaison,
         par un dérivé de formule (V/A) :

         Hal'₃ - C - CO - Hal" (V/A)

         où Hal' et Hal" identiques ou différents représentent chacun un atome d'halogène, pour conduire à un dérivé de formule (VI) : 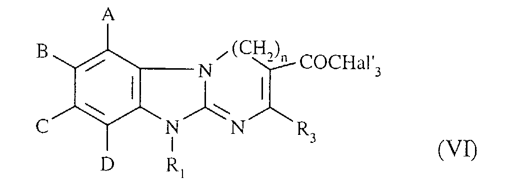 où A, B, C, D, R₁, n, R₃ et Hal'₃ ont la même définition que précédemment, dérivé de formule (VI) que l'on traite par un composé de formule (VII) : 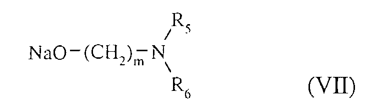 où m, R₅ et R₆ ont la même signification que dans la formule du groupement G₄ tel que défini dans la formule (I),
      pour conduire à un dérivé de formule (I/C): 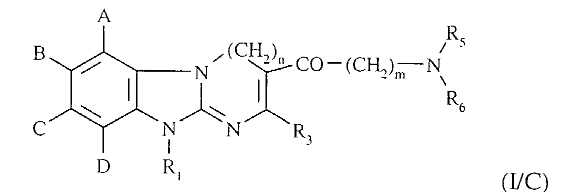 où A, B, C, D, R₁, R₃, R₅ et R₆ et n ont la même définition que dans la formule (I),
      Y et Z d'une part et X et R₂ d'autre part forment ensemble une double liaison et R₄ représente un groupement G₄ :
   * lorsque dans le dérivé de formule (I) que l'on souhaite obtenir R₄ représente un groupement G₄, Y et Z représentent une liaison par un dérivé de formule (VIII) :

      Hal"'-(CH₂)ₘ-Hal₄ (VIII)

      ou Hal''' et Hal₄ identiques ou différents représentent un atome d'halogène et m a la même définition que précédemment, pour conduire à un dérivé de formule (IX) : 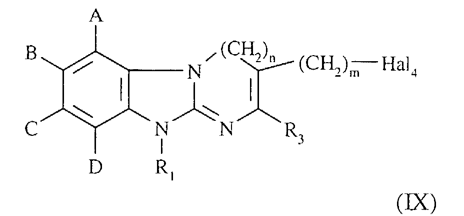 ou A, B, C, D, R₁, n, p, R₃ et Hal₄ ont la même définition que précédemment,
      que l'on traite alors par un dérivé de formule (X) : 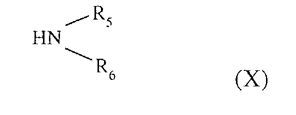 où R₅ et R₆ ont la même définition que précédemment,
      pour conduire à un dérivé de formule (I/D) : 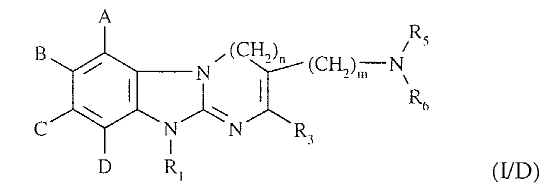 cas particulier des dérivés de formule (I) où A, B, C, D, R₁, n ont la même définition que dans la formule (I) et X et R₂ d'une part, et Y et Z d'autre part, forment ensemble une liaison et R₄ forme un groupement G₁ tel que défini dans la formule (II),
   * lorsque dans le dérivé de formule (I) que l'on souhaite obtenir R₄ représente un groupement G₅ ou G₇ et Y et Z représentent une liaison, par un dérivé de formule (XI) :

      Cl-CO-R₉ ou Cl-CO-R₁₀ (XI)

      selon le dérivé que l'on souhaite obtenir où R₉ et R₁₀ ont la même définition que précédemment,
      pour conduire à un dérivé de formule (I/E): 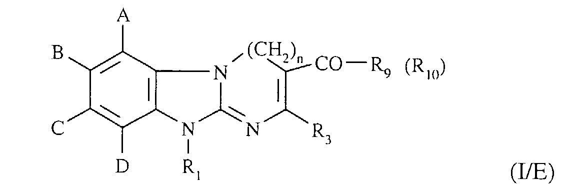 cas particulier des dérivés de formule (I) pour lesquels A, B, C, D, n, R₃ ont la même définition que précédemment, X et R₂ d'une part, et Y et Z d'autre part, forment ensemble une liaison et R₄ représente un groupement G₅ ou G₇ tels que défini précédemment.
**B.**
   lorsque dans le dérivé de formule (I) que l'on souhaite obtenir X et R₁ forment ensemble une liaison,
   en ce que l'on utilise comme matière première un dérivé de formule (XII) : 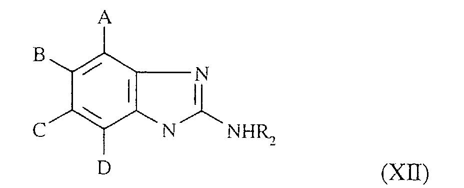 où A, B, C, D et R₂ ont la même définition que précédemment,
   * que l'on traite lorsque dans le dérivé de formule (I) que l'on souhaite obtenir Y et Z représentent chacun un atome d'hydrogène,
      par un dérivé de formule (III/A) tel que défini précédemment pour conduire après chauffage à un dérivé de formule (I/G) : 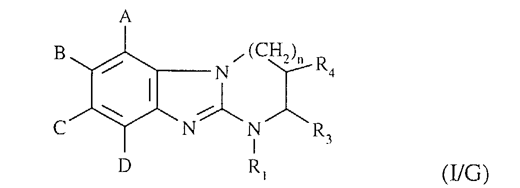 cas particulier des dérivés de formule (I) où A, B, C, D, n, R₁, R₃, R₄ ont la même définition que précédemment,
      X et R₁ formant ensemble une liaison et Y et Z représentent chacun un atome d'hydrogène,
   * lorsque dans le dérivé de formule (I) que l'on souhaite obtenir Y et Z forment une liaison,
      par un dérivé de formule (III) telle que défini précédemment,
      pour conduire après chauffage à un dérivé de formule (I/F): 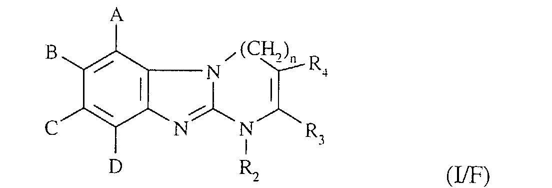 cas particulier des dérivés de formule (I) pour lesquels A, B, C, D, n, R₂, R₃ ont la même définition que précédemment,
      X et R₁ d'une part et Y et Z d'autre part forment ensemble une liaison et R₄ représente un atome d'hydrogène,
      dérivé de formule (I/F) que l'on peut en fonction de la valeur de R₄ dans le dérivé de formule (I) que l'on souhaite obtenir traiter :
      - successivement comme indiqué plus haut par un dérivé de formule (V/A) tel que défini précédemment, puis par un dérivé de formule (VII) tel que défini précédemment,
      - successivement comme indiqué plus haut par un dérivé de formule (VIII) tel que défini précédemment puis par un dérivé de formule (X) tel que définit précédemment,
      - ou bien encore par un dérivé de formule (XI) tel que défini précédemment,
      pour obtenir un dérivé de formule (I/H): 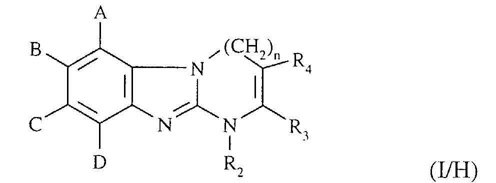 cas particulier des dérivés de formule (I) pour lesquels A, B, C, D, n, R₂, R₃, R₄ ont la même définition que dans la formule (I),
      X et R₁ d'une part et Y et Z d'autre part formant chacun une liaison,
      dérivés de formule (I), (I/A), (I/B), (I/C), (I/D), (I/E), (I/F), (I/G), (I/H) que l'on purifie, si nécessaire, par une technique choisie parmi chromatographie et/ou cristallisation et que l'on transforme, si on le désire, par un acide, en sel pharmaceutiquement acceptable.

Le procédé de synthèse des dérivés de formule (I) indiqué ci-dessus est très général et est susceptible de subir les modifications que l'homme de l'art jugera appropriées afin de réaliser une synthèse parfaitement adaptée à chaque produit de formule (I) que l'on souhaite obtenir, celle-ci étant bien évidemment fonction de la nature des substituants A, B, C, D, R₁, R₂, R₃, R₄. On trouvera ci-dessous un certain nombre de variantes non limitatives.

L'une des variantes consiste à soumettre à réduction par un hydrure mixte de métal alcalin un dérivé de formule (IV/B) : 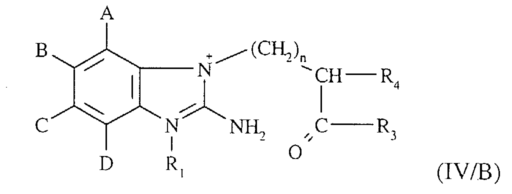 obtenu par condensation d'un dérivé de formule (Il) sur un dérivé de formule (III/B) : 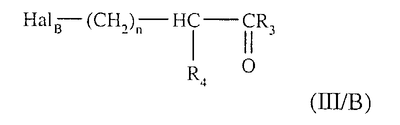 où Hal_{B} représente un atome d'halogène et n, R₃, R₄ ont la même définition que précédemment,
en dérivé de formule (IV/A) tel que défini précédemment pour lequel le groupement P représente un groupement hydroxyle, lequel traité par un agent d'halogénation puis chauffé conduit à un dérivé de formule (I/B) tel que défini précédemment.

Une autre variante concerne l'obtention des dérivés de formule (I/B) tel que défini précédemment pour lesquels R₁ représente un groupement G₂ ou G₃, et Y et Z forment un atome d'hydrogène.

Dans ce cas, on utilise comme matière première un dérivé de formule (XIII) : 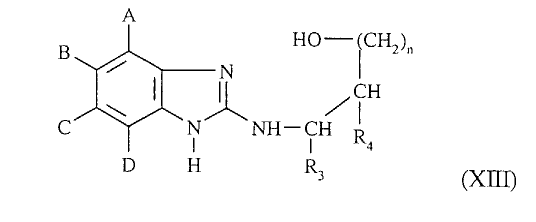 où A, B, C, D et n ont la même définition que précédemment,
que l'on traite par un agent d'halogénation puis par chauffage,
pour conduire à un dérivé de formule (XIV): 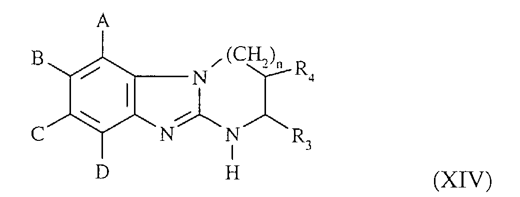 dans laquelle A, B, C, D, R₃, R₄ et n ont la même définition que précédemment,
que l'on traite par un dérivé de formule (XV): 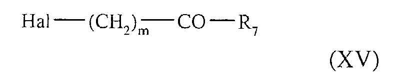 où m et R₇ ont la même définition que dans la formule (I) et Hal représente un atome d'halogène,
pour conduire à un dérivé de formule (I/J) : 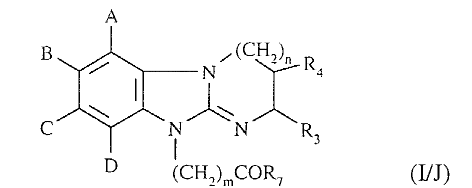 dans lequel A, B, C, D, m, n, R₃, R₄ et R₇ ont la même définition que précédemment, Y et Z représentant un atome d'hydrogène, et X et R₂ formant une liaison et R₁ représentant un groupement G₂,
dérivé de formule (I/J) que l'on peut soumettre à l'action d'un hydrure mixte de métal alcalin pour conduire à un dérivé de formule (I/K): 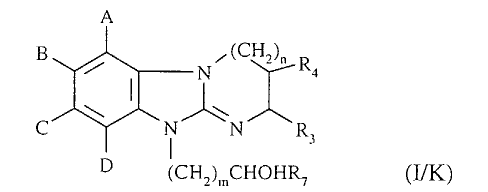 où A, B, C, D, m, n, R₃, R₄ et R₇ ont la même définition que précédemment, Y et Z représentent chacun un atome d'hydrogène et X et R₂ forment ensemble une liaison, et R₁ représente un groupement G₃,
dérivés de formule (I/J) ou (I/K) que l'on purifie si nécessaire et que l'on transforme, si on le désire, par un acide en un sel pharmaceutiquement acceptable.

Une autre voie d'accès aux dérivés de formule (I/J) consiste à traiter le dérivé de formule (XIII) tel que défini précédemment par le dérivé de formule (XV) tel que défini précédemment pour permettre l'obtention d'un dérivé de formule (XVI) : 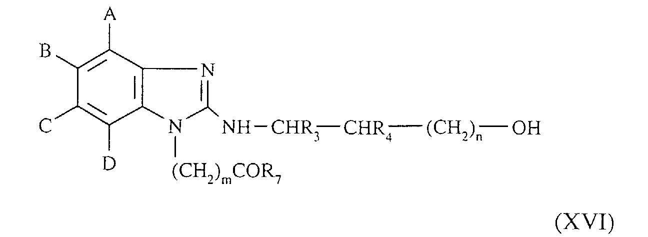 où A, B, C, D, R₃, R₄, n, m et R₇ ont la même définition que précédemment,
qui traite par un agent d'halogénation et par chauffage conduit à un dérivé de formule (I/J) tel que défini précédemment.

Une autre voie d'accès au dérivé de formule (I/J) ou (I/K) consiste à utiliser comme matière première un dérivé de formule (XIV) (et non plus un dérivé de formule XIII), que l'on traite comme décrit précédemment.

Une autre voie d'accès au dérivé de formule (I/K) consiste à traiter le dérivé de formule (XIV) tel que défini précédemment par un dérivé de formule (XVII) :

Hal - (CH₂)ₘ - CHOH - R₇ (XVII)

où Hal, m et R₇ ont la même définition que dans la formule (I).

Il est également possible d'utiliser comme matière première des dérivés décrits dans la littérature et de formule générale : 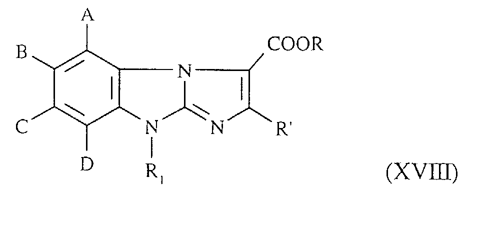 où A, B, C, D, R₁ ont la même définition que précédemment, R un groupement alkyle inférieur ou un atome d'hydrogène et R' un groupement alkyle inférieur éventuellement substitué par un atome d'halogène ou un groupement hydroxyle.

De tels dérivés sont décrits dans Khim. Farm., Zh, 1988, 22(7), 815-819 ou dans Khim. Geterotskl. Soedin., 1973(1), 111-114 ou peuvent être obtenus en traitant un dérivé de formule (IV), tel que défini précédemment, pour lequel n vaut 0 et R₃ représente un groupement OR,
par un dérivé de formule (XIX) : 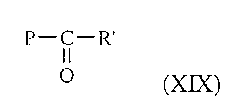 où P constitue un groupe partant choisi parmi halogène, hydroxy, (C₁-C₆) alkoxy inférieur et R' a la même définition que précédemment,
pour conduire après chauffage à un dérivé de formule (XVIII).

Lorsque R' représente un groupement méthyle, les dérivés (XVIII) sont traités par la N-bromosuccinimide,
pour conduire à un dérivé (XVIII') où R' représente un groupement CH₂Br.

Lorsque R' représente un groupement alkyle inférieur substitué par un groupement hydroxyle un traitement par un agent d'halogénation permet d'obtenir un dérivé (XVIII") pour lequel R' représente un groupement alkyle substitué par un atome d'halogène.

Ces dérivés (XVIII) (lorsque R' représente un alkyl inférieur substitué par un halogène), (XVIII') ou (XVIII") peuvent être traités par une amine de formule HNR₅R₆, pour conduire après chauffage en milieu acide à un dérivé de formule (I/L) : 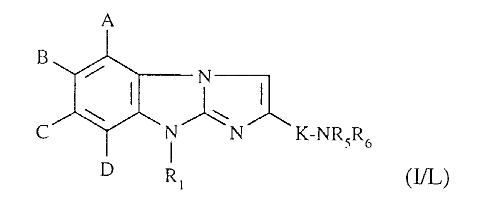 où A, B, D, R₁, R₅ et R₆ ont la même définition que dans la formule (I),
cas particulier des dérivés de formule (I) pour lesquels Y et Z d'une part et X et R₁ d'autre part forment ensemble une liaison, R₄ représente un atome d'hydrogène, n vaut 0 et R₃ représente un groupement K-NR₅R₆ où K représente un groupement alkyle inférieur,
dérivé de formule (I/L) que l'on transforme, si on le désire, par un acide, en un sel pharmaceutiquement acceptable.

De façon plus générale, l'homme du métier pourra utiliser ce procédé utilisant comme matière première les dérivés de formule (XVIII) pour l'obtention d'autres familles de dérivés de formule (I) en utilisant les réactions chimiques usuelles.

Un autre cas particulier concerne les dérivés pour lesquels R₄ représente un groupement CH₂NR₅R₆. Ceux-ci pourront être obtenus en traitant un dérivé de formule (I/A) tel que défini précédemment par une réaction de Mannich utilisant le formol et une amine HNR₅R₆.

Une autre variante du procédé général concerne la synthèse de dérivés de formule (I) pour lesquels R₂ représente un groupement G₁.

De tels dérivés pourront être obtenus en utilisant comme matière première un composé de formule (XX): 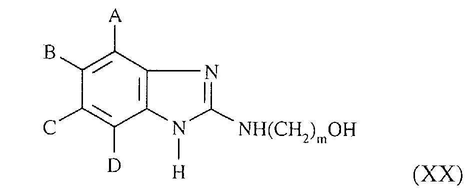 où A, B, C, D et m ont la même définition que précédemment,
que l'on traite par un dérivé de formule (XXI):

Hal-(CH₂)ₙ₊₁-COR₃ (XXI)

où n et R₃ ont la même définition que précédemment et Hal représente un atome d'halogène pour obtenir un dérivé de formule (XXII): 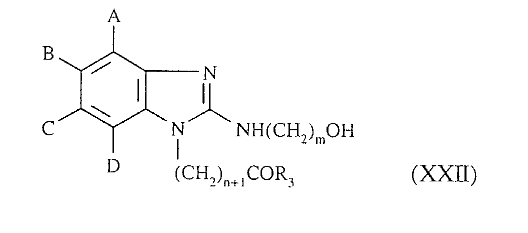 dans laquelle A, B, C, D, R₃ et m ont la même définition que dans la formule (I) qui par chauffage conduit à la formation d'un dérivé de formule (XXIII): 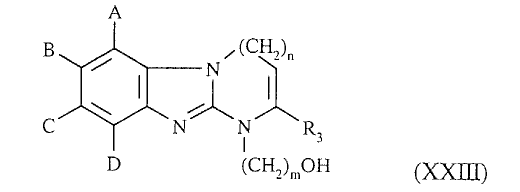 où A, B, C, D, m, n et R₃ ont la même définition que dans la formule (I),
que l'on traite :
a ) par un agent d'halogénation,
b ) par une amine de formule HNR₅R₆,
pour permettre l'obtention d'une dérivé de formule (I/M): 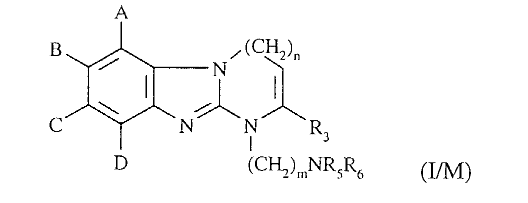 cas particulier des dérivés de formule (I) pour lesquels A, B, C, D, m, n, R₃, R₅ et R₆ ont la même définition que dans la formule (I),
pour lesquels R₁ et X et Y et Z représentent chacun une liaison et R₂ représente un groupement G₁, que l'on purifie si nécessaire et que l'on transforme, si on le désire, par un acide, en sel pharmaceutiquement acceptable.

Les composés de formule (I) possèdent des propriétés pharmacologiques intéressantes.

Les études pharmacologiques préliminaires ont montré que les dérivés de l'invention sont atoxiques et possèdent simultanément une activité hypoglycémiante et une activité antiagrégante plaquettaire.

Les composés de l'invention ont donc tout naturellement comme indication le diabète et ses complications cardiovasculaires (rétinopathies, troubles vasculaires périphériques et cérébraux).

La présente invention a également pour objet les compositions pharmaceutiques renfermant comme principe actif au moins un composé de formule générale (I) ou un de ses d'addition à un acide pharmaceutiquement acceptable ou le cas échéant à une base pharmaceutiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, rectale, per ou trans cutanée, ophtalmique, respiratoire, perlinguale etc..., et notamment les comprimés simples ou dragéifiés, les comprimés sublingaux, les sachets, paquets, les gélules, glossettes, tablettes, suppositoires, crèmes, pommades, gels dermiques, ampoules buvables, injectables, etc...

La posologie varie selon l'âge et le poids du patient, la nature et la sévérité de l'affection ainsi que la voie d'administration. Celle-ci peut être orale, nasale, rectale ou parentérale.

D'une manière générale, la posologie unitaire s'échelonne entre 0,5 et 20 mg par prise et d'une à quatre prises par 24 heures.

Les exemples suivants illustrent l'invention et ne la limitent en aucune façon.

Les spectres de résonance magnétique nucléaire ¹H ont été réalisés en utilisant le TMS (tétraméthylsilane) comme référence interne. Les déplacements chimiques sont exprimés en partie par million (p.p.m.). Les spectres infrarouge ont été effectués par mise en suspension du produit dans de l'huile de paraffine.

### EXEMPLE 1 :

### 9-DIETHYLAMINOETHYL-2,3-DIHYDROIMIDAZO[1,2-a]BENZIMIDAZOLE, DICHLORHYDRATE

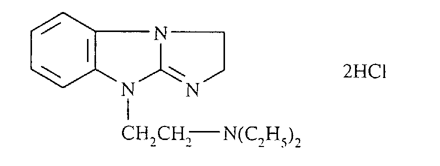

**STADE 1 :2-Amino-1-diéthylaminoéthyl-3-(2-hydroxyéthyl)benzimidazole, chlorhydrate**
Chauffer à 130-135°C pendant 40 minutes environ un mélange de (0,01 mole) de 2-amino-1-[(2-diéthylamino)éthyl]benzimidazole obtenu comme décrit dans J. Med. Chem. 72, 15(9), 9236, et de 3 ml de 2-chloroéthanol. Après refroidissement, le milieu réactionnel est traité par un mélange éther acétone jusque cristallisation. Filtrer et laver le résidu à l'éther. Recristalliser dans un mélange alcool-éther.
*Rendement : 90%*
*Point de fusion : 159°C*

| *Composition centésimale :* | | | | |
|---|---|---|---|---|
| *Calculée* | *C 57,6* | *H 8,1* | *Cl 11,3* | *N 17,9* |
| *Trouvée* | *C 57,4* | *H 8,0* | *Cl 11,0* | *N 17,8* |

**STADE 2 :1-Diéthylaminoéthyl-2-imino-3-(2-chloroéthyl)benzimidazole, dichlorhydrate**
Chauffer à reflux 0,010 mole de produit obtenu au stade 1 et 20 moles de chlorure de thionyle préalablement dissoutes dans 15 ml de chloroforme. Evaporer la solution obtenue, et laver le résidu à l'éther de pétrole pour obtenir le produit du titre. Recristallisation : éthanol - éther.
*Rendement : 100%*
*Point de fusion : 214-215°C*

| *Composition centésimale:* | | | | |
|---|---|---|---|---|
| *Calculée* | *C 49,0* | *H 6,9* | *Cl 28,9* | *N 15,2* |
| *Trouvée* | *C 49,2* | *H 6,7* | *Cl 28,5* | *N 15,6* |

**STADE 3 :9-Diéthylaminoéthyl-2,3-dihydroimidazo[1,2-a]benzimidazole, dichlorhydrate**
A 5 mmol du produit obtenu au stade précédent, ajouter de l'ammoniaque à 10% et extraire au chloroforme ; évaporer et ajouter 10 ml d'o-xylène et porter une heure à reflux. Après refroidissement, filtrer le précipité, le laver à l'éther de pétrole, traiter par une solution de carbonate de soude et extraire à deux reprises au chloroforme (10 ml). L'extrait est élué au chloroforme, sur gel d'alumine. L'éluat est évaporé, le résidu dissous dans l'acétone et la solution ainsi obtenue traitée par une solution d'éther chlorhydrique. Essorer le précipité, laver à l'acétone et à l'éther.
*Rendement : 92% (cristaux hydrates - 1H*_{*2*}*O)*
*Point de fusion : 245-246°C (hydrate) (de l'éthanol)*

| *Composition centésimale :* | | | | |
|---|---|---|---|---|
| *Calculée* | *C 54,4* | *H 7,3* | *Cl 21,4* | *N 16,9* |
| *Trouvée* | *C 54,2* | *H 7,2* | *Cl 21,4* | *N 16,9* |

*Caractéristiques spectrales :*
*Infrarouge : 1665 cm*^{*-1*} *ν C = N*
*Résonance Magnétique Nucléaire :* (Base) (CDCl₃)
δ *:0,96 ppm, triplet, 6H, 2CH*_{*3*}
*δ:6,82 ppm, massif, 4H, aromatiques*

### EXEMPLE 2 :

### 9-MORPHOLINOETHYL-2,3-DIHYDROIMIDAZO[1,2-a]BENZIMIDAZOLE, DICHLORHYDRATE

En procédant comme dans l'exemple 1, mais en utilisant comme matière première le 2-amino-1-(2-morpholinoéthyl)benzimidazole obtenu comme décrit dans Khim. Geterot sikl. Soedin ; 69, (5), 869-873 au lieu du 2-amino-1-(2-diéthylaminoéthyl)benzimidazole, on obtient le produit du titre.
*Point de fusion : 282-83°C*

| *Composition centésimale :* | | | | |
|---|---|---|---|---|
| *Calculée* | *C 52,2* | *H 6,4* | *CI 20,5* | *N 16,2* |
| *Trouvée* | *C 52,0* | *H 6,3* | *Cl 20,3* | *N 16,4* |

### EXEMPLE 3 :

### 9-PHENACYL-2,3-DIHYDROIMIDAZO[1,2-a]BENZIMIDAZOLE, CHLORHYDRATE

**STADE A :2-(2-Hydroxyéthylamino)-1-phénacylbenzimidazole, dibromhydrate**
Chauffer à reflux pendant trois heures 8,95 g (50 mmoles) de 2-(2-hydroxy éthylamino) benzimidazole et 10 g de bromure de phénacyle dans 100 ml de 2-propanol. Laisser refroidir. Essorer le précipité formé et laver à l'acétone. On obtient le produit du titre. *Rendement : 89%*
*Point de fusion : 216-217°C (décomposition)*

| *Composition centésimale :* | | | | |
|---|---|---|---|---|
| *Calculée* | *C 54,3* | *H 4,8* | *Br 21,2* | *N 11,2* |
| *Trouvée* | *C 54,1* | *H 5,0* | *Br 21,0* | *N 11,2* |

*Caractéristiques spectrales :*
*Infrarouge :1702 cm*^{*-1*} ν *CO*
*1675 cm*^{*-1*} *ν C=NH*^{*+*}
**STADE B :2(2-Hydroxyéthylamino)-1-phénacilbenzimidazole (base)**
Ajouter de l'ammoniaque à 22% à 75 ml d'une solution chaude du dibromhydrate obtenu au stade A, dans l'alcool à 95° jusqu'à obtention d'un pH de 10 environ. Le produit du titre précipité ; il est filtré, lavé à l'eau et séché.
*Rendement : 95%*
*Point de fusion : 167-168°C (décomposition)*

| *Composition centésimale :* | | | |
|---|---|---|---|
| *Calculée* | C *69,2* | *H 5,8* | *N 14,2* |
| *Trouvée* | *C 69,0* | *H 5,9* | *N 14,4* |

*Caractéristiques spectrales :*
*Infrarouge: 1525, 1580, 1615 cm*^{*-1*} *ν C=C et C=N*
*1700 cm*^{*-1*} ν *C=O*
**STADE C :1-Phénacyl-2-(2-chloroéthylamino)benzimidazole, chlorhydrate**
Ajouter sous agitation 0,7 ml (10 mmole) de chlorure de thionyle fraîchement distillé à une suspension de 1,5 g (5 mmol) de la base obtenue précédemment dans 25 ml de chloroforme anhydre. Chauffer à reflux 1 heure la solution obtenue. Après refroidissement, le précipité de chlorhydrate de 1-phénacyl-2-(2-chloroéthylamino)benzimidazole est isolé par filtration, lavé à l'éther de pétrole et recristallisé d'un mélange éthanol/éther.
*Rendement : :90%*
*Point de fusion : 207-208°C (décomposition)*

| *Composition centésimale :* | | | | |
|---|---|---|---|---|
| *Calculée* | *C 58,3* | *H 4,9* | *Cl 20,2* | *N 12,0* |
| *Trouvée* | *C 58,0* | *H 5,0* | *Cl 19,9* | *N 12,3* |

**STADE D :2-(2-Chloroéthylamino)-1-phénacylbenzimidazole**
Le chlorhydrate obtenu au stade C est traité par une solution aqueuse d'ammoniaque suivi par l'extraction chloroformique de la base libre formée. Recristalliser le résidu dans le benzène.
*Rendement : 87%*
*Point de fusion : 115-116°C*
*Caractéristiques spectrales :*
*Infrarouge : 1680 cm*^{*-1*}ν *CO*
*3300 cm*^{*-1*}*ν NH*
**STADE E :9-Phénacyl-2,3-dihydroimidazo[1,2-a]benzimidazole**
Porter 0,03 mole de 2-(2-chloroéthylamino)-1-phénacylbenzimidazole à une température de 135-140°C obtenue par un bain d'huile. Après fusion complète, le milieu réactionnel est cristallisé. Le milieu réactionnel est alors refroidi et traité par 10 ml d'une solution aqueuse d'ammoniaque à 22% et extrait à trois reprises au chloroforme. Les phases organiques sont réunies et concentrées. Le résidu organique est élué sur colonne d'alumine et l'éluat, après évaporation du solvant, est contitué du produit du titre.
*Rendement : 88%*
*Point de fusion : 178-179°C*

| *Composition centésimale :* | | | |
|---|---|---|---|
| *Calculée* | *C 73,3* | *H 5,4* | *N 15,2* |
| *Trouvée* | *C 73,3* | *H 5,5* | *N 15,0* |

*Caractéristiques spectrales :*
*Infrarouge : 1505, 1600 cm*^{*-1*}ν (C=C)
*1665 cm*^{*-1*} ν *(C=N)*
*1690 cm*^{*-1*}ν *(C=O)*
*Résonance Magnétique Nucléaire : CDCl*_{*3*}*,* δ = *ppm*
*δ = 5,1 ; singulet ; 2H, CH*_{*2*}*-CO*
*δ = 6,75 ; 7,54 et 7,9, trois multiplet, 9H, aromatiques*
**STADE F :9-phénacyl-2,3-dihydroimidazo[1,2-a]benzimidazole, chlorhydrate**
Dissoudre à chaud 0,55 g de la base obtenue au stade E dans 20 ml d'acétone jusqu'à l'obtention du pH 2-3. Filtrer le précipité obtenu, laver à l'acétone et à l'éther et recristalliser dans l'alcool.
*Rendement : 96%*
*Point de fusion : 257-258°C (décomposition)*

| *Composition centésimale :* | | | | |
|---|---|---|---|---|
| *Calculée* | *C 65,1* | *H 5,1* | *Cl 11,3* | *N 13,4* |
| *Trouvée* | *C 64,9* | *H 5,2* | *Cl 11,0* | *N 13,1* |

### EXEMPLE 4 :

### 9-PHENACYL-2,3-DIHYDROIMIDAZO[1,2-a]BENZIMIDAZOLE (2ème procédé de synthèse)

Ajouter 10 mmol (1,99 g) de bromure de phénacyle à une solution chaude de 1H-2,3-dihydroimidazo[1,2-a]benzimidazole décrit dans J. Med. Chem. 1982, 25(11), 1342-6, dans 10 ml de 2-propanol. Porter deux heures à reflux. Après refroidissement le précipité est recueilli par filtration et traité avec une solution aqueuse d'ammoniaque à 22% pour former le composé du titre.
*Rendement : 95%*
*Point de fusion : 178-179°C*

Ce produit peut être traité selon le protocole de l'exemple 3, stade F pour conduire au chlorhydrate.

### EXEMPLE 5:

### 9-(p-CHLOROPHENACYL)-2,3-DIHYDROIMIDAZO[1,2-a]BENZIMIDAZOLE, CHLORHYDRATE

En procédant comme dans l'exemple 4 ou comme dans l'exemple 3, mais en remplaçant le bromure de phénacyle (exemple 3, stade A) par le bromure de 4'-chloro phénacyle, on obtient le produit du titre.
*Point de fusion : 294-295°C*

| *Composition centésimale :* | | | | |
|---|---|---|---|---|
| *Calculée* | *C 58,7* | *H 4,3* | *Cl 20,4* | *N 12,1* |
| *Trouvée* | *C 58,7* | *H 4,4* | *Cl 19,9* | *N 12,3* |

*Caractéristiques spectrales :*
*Infrarouge: 1500-1600 cm*^{*-1*}*ν CC*
*1660 cm*^{*-1*}*ν C=N*
*1695 cm*^{*-1*}ν *C=O*
*Résonance Magnétique Nucléaire : CDCl*_{*3*}*,* δ = *ppm*
*δ = 5,02 ; singulet ; 2H, CH*_{*2*}*-CO*

### EXEMPLE 6:

### 9-(p-METHOXYPHENACYL)-2,3-DIHYDROIMIDAZO[1,2-a]BENZIMIDAZOLE, CHLORHYDRATE

En procédant comme dans l'exemple 4 ou comme dans l'exemple 3, mais en remplaçant le bromure de phénacyle (exemple 3, stade A) par le bromure de 4'-méthoxyphénacyle, on obtient le produit du titre.
*Point de fusion : 242-243°C*

| *Composition centésimale :* | | | | |
|---|---|---|---|---|
| *Calculée* | *C 62,9* | *H 5,3* | *Cl 10,3* | *N 12,2* |
| *Trouvée* | *C 62,7* | *H 5,2* | *Cl 10,0* | *N 12,4* |

*Caractéristiques spectrales :*
*Infrarouge (base): 1500-1600 cm*^{*-1*}*ν C=C*
*1660 cm*^{*-1*}ν *C=N*
*1695 cm*^{*-1*}*ν C=O*
*Résonance Magnétique Nucléaire : CDCl*_{*3*}*, (base)*
*δ = 3,75 ; 3H ; singulet, OCH*_{*3*}
*δ = 5,03 ; 2H ; singulet, CH*_{*2*}*O*
*δ = 6,75 et 7,90; 8H, 2 multiplets, aromatiques*

### EXEMPLE 7:

### 9-(1-NAPHTOYLMETHYL)-2,3-DIHYDROIMIDAZO[1,2-a]BENZIMIDAZOLE, CHLORHYDRATE

En procédant comme dans l'exmeple 4 ou comme dans l'exemple 3, mais en remplaçant le bromure de phénacyle (exemple 3, stade A) par le bromure de naphtoylméthyle, on obtient le produit du titre.
*Point de fusion : 202°C (décomposition)*

| *Composition centésimale :* | | | | |
|---|---|---|---|---|
| *Calculée* | *C 64,7* | *H 5,7* | *Cl 11,2* | *N 13,3* |
| *Trouvée* | *C 64,9* | *H 5,6* | *Cl 10,9* | *N 13,4* |

*Caractéristiques spectrales :*
*Infrarouge (base): 1665 cm*^{*-1*}ν *CN*
*2700-3055 cm*^{*-1*}ν *OH*

### EXEMPLE 8:

### 9-(2-HYDROXY-2-PHENYL)ETHYL-2,3-DIHYDROIMIDAZO[1,2-a]BENZIMIDAZOLE, CHLORHYDRATE

Ajouter petit à petit 0,3 g (7,5 mmol) de borohydrure de sodium à une suspension de 1,94 g (7 mmol) de 9-phénacyl-2,3-dihydroimidazo[1,2-a]benzimidazole dans 15 ml de méthanol pendant trente minutes à température ambiante. Poursuivre l'agitation pendant quatre heures supplémentaires et abandonner jusqu'au lendemain. Ajouter alors 10 ml d'acide chlorhydrique à 10% jusqu'à l'obtention d'un pH 2-3 et évaporer le méthanol. Traiter le résidu par une solution d'ammoniaque et extraire au chloroforme. Recristalliser le résidu d'extraction dans l'éthanol.
*Rendement : 87%*
*Point de fusion : 192°C*

| *Composition centésimale :* | | | |
|---|---|---|---|
| *Calculée* | *C 73,0* | *H 6,7* | *N 15,0* |
| *Trouvée* | *C 73,2* | *H 5,8* | *N 15,3* |

*Caractéristiques spectrales :*
*Infrarouge (base): 1665 cm*^{*-1*}*ν C=N*
*2700-3055 cm*^{*-1*}*ν OH*

Le chlorhydrate est obtenu par addition d'éther chlorhydrique à une solution éthanolique chaude de la base précédemment obtenue (3 mmol, 0,84 g) jusqu'à l'obtention d'un milieu acide.
Laisser refroidir, essorer, laver à l'éther.
*Rendement : (par rapport à la base) 97,3%*
*Point de fusion : 202°C*

| *Composition centésimale :* | | | | |
|---|---|---|---|---|
| *Calculée* | *C 64,7* | *H 5,7* | *Cl 11,2* | *N 13,3* |
| *Trouvée* | C 64,9 | *H 5,6* | *Cl 10,9* | *N 13,4* |

*Caractéristiques spectrales :*
*Infrarouge (base)*: *1665 cm*^{*-1*}ν *C=N*
*2700-3055 cm*^{*-1*}*ν OH*

### EXEMPLE 9:

### 9-[2-HYDROXY-2-(4'-CHLOROPHENYL)ETHYL]-2,3-DIHYDROIMIDAZO[1,2-a] BENZIMIDAZOLE, CHLORHYDRATE

En procédant comme l'exemple 8, mais en remplaçant le 9-phénacyl-2,3-dihydroimidazo[1,2-a]benzimidazole par le 9-(p-chlorophénacyl)-2,3-dihydroimidazo[1,2-a]benzimidazole obtenu dans l'exemple 5, on obtient le produit du titre.
*Point de fusion (chlorhydrate) : 208-209°C*

| *Composition centésimale :* | | | | |
|---|---|---|---|---|
| *Calculée* | *C 58,3* | *H 4,9* | *Cl 20,2* | *N 12,0* |
| *Trouvée* | *C 58,4* | *H 4,7* | *Cl 20,5* | *N 11,8* |

*Caractéristiques spectrales :*
*Infrarouge (base)*: *1650 cm*^{*-1*}ν *C=N*
*2690-3060 cm*^{*-1*}*ν OH*

### EXEMPLE 10 :

### 9-(2-(1-NAPHTYL)-2-HYDROXYETHYL]-2,3-DIHYDROIMIDAZO[1,2-a]BENZIMIDAZOLE, CHLORHYDRATE

En procédant comme l'exemple 8, mais en remplaçant le 9-phénacyl-2,3-dihydroimidazo[1,2-a]benzimidazole par le 9-(1-naphtoylméthyl)-2,3-dihydroimidazo[1,2-a]benzimidazole obtenu dans l'exemple 7, on obtient le produit du titre.
*Point de fusion (chlorhydrate) 214-215°C*

| *Composition centésimale :* | | | | |
|---|---|---|---|---|
| *Calculée* | *C 68,9* | *H 5,5* | *Cl 9,7* | *N 11,5* |
| *Trouvée* | *C 68,8* | *H 5,7* | *Cl 9,5* | *N 11,4* |

*Caractéristiques spectrales :*
*Infrarouge (base)*: *1650 cm*^{*-1*}ν *C=N*
*2700-3055 cm*^{*-1*}ν *OH*

### EXEMPLE 12:

### 2-TERT.BUTYL-9-(2-MORPHOLINOETHYL)IMIDAZO[1,2-a]BENZIMIDAZOLE, DIBROMHYDRATE

**STADE A : (2-amino-1-(2-morpholinoéthyl)3-pivaloylméthyl)benzimidazolium, bromhydrate**
Une solution chaude de (2-amino-1-morpholino)éthylbenzimidazole dans 25 ml d'éthanol est mélangée avec 1,35 g (10 mmole) de bromopinacolone, portée à reflux 10 minutes, abandonnée 3 heures à température ambiante et diluée deux fois à l'acétone. Le précipité formé est filtré, lavé à l'acétone pour donner le produit du titre.
*Rendement : 87%*
*Point de fusion : 195-196°C (décomposition)*

| *Composition centésimale :* | | | | |
|---|---|---|---|---|
| *Calculée* | *C 53,6* | *H 6,9* | *Br 18,8* | *N 13,2* |
| *Trouvée* | *C 53,5* | *H 6,7* | *Br 19,0* | *N 13,4* |

*Caractéristiques spectrales :*
*Infrarouge (base)*: *1665 cm*^{*-1*}ν *C=N*
*1715 cm*^{*-1*}*ν CO*
**STADE B :2-tert.butyl-9-(morpholinoéthyl)imidazo[1,2-a]benzimidazole, chlorhydrate**
Le bromhydrate obtenu au stade A est chauffé à reflux dans l'acide chlorhydrique concentré pendant 30 minutes. La solution est refroidie et neutralisée avec une solution aqueuse d'ammoniaque. Le milieu réactionnel est extrait au benzène et l'extrait est séché sur sulfate de sodium. Faire passer ensuite un courant d'acide chlorhydrique gazeux jusqu'à l'acidité. Le précipité formé est filtré et recristallisé dans l'éthanol.
*Rendement : 87%*
*Point de fusion : 263-264°C (décomposition)*

| *Composition centésimale :* | | | | |
|---|---|---|---|---|
| *Calculée* | *C 57,1* | *H 7,1* | *Br 17,8* | *N 14,0* |
| *Trouvée* | *C 57,0* | *H 6,9* | *Br 17,5* | *N 14,2* |

*Caractéristiques spectrales :*
*Infrarouge (base): 1505, 1600, 1620 cm*^{*-1*}*ν C=C* ν *C=N*

### EXEMPLE 13:

### 2-TERT.BUTYL-9-(2-MORPHOLINOETHYL)IMIDAZO[1,2-a]BENZIMIDAZOLE, BROMHYDRATE

En procédant comme dans l'exemple 12, mais en remplaçant au stade B le courant d'acide chlohydrique gazeux par le courant d'acide bromhydrique gazeux, on obtient le produit du titre.
*Point de fusion : 294-295°C*

| *Composition centésimale :* | | | | |
|---|---|---|---|---|
| *Calculée* | *C 46,7* | *H 5,8* | *Br 32,4* | *N 11,5* |
| *Trouvée* | *C 46,5* | *H 6,0* | *Br 32,4* | *N 11,6* |

### EXEMPLE 14:

### 2-TERT.BUTYL-9-(3-DIMETHYLAMINOPROPYL)IMIDAZO[1,2-a]BENZIMIDAZOLE, DICHLORHYDRATE

**STADE A :2-Amino-1-(3-diméthylaminopropyl)-3-pivaloylméthyl benzimidazolium**
Mettre à réagir 10 mmol (2,18 g) de 2-amino-1-diméthylaminopropyl benzimidazole et 10 mmol (1,35 ml) de bromopinacoline dans 70 ml d'acétone.
*Recristallisation : éthanol/acétone/éther*
*Rendement : 90%*
*Point de fusion : 197-198°C (décomposition)*

| *Composition centésimale :* | | | | |
|---|---|---|---|---|
| *Calculée* | *C 54,4* | *H 7,4* | *Br 20,1* | *N 14,1* |
| *Trouvée* | *C 54,2* | *H 7,4* | *Br 19,8* | *N 14,1* |

*Caractéristiques spectrales :*
*Infrarouge: 1720 cm*^{*-1*}*ν* CO
*1660 cm*^{*-1*}*ν C=N*
*3050, 3250 cm*^{*-1*}*ν NH*
**STADE B :2-tert.butyl-9-(3-diméthylaminopropyl)imidazo[1,2-a] benzimidazole**
Porter à reflux 3 mmol du bromhydrate obtenu au stade A et 4 g de carbonate de sodium dans 25 ml d'éthanol pendant 25 minutes. Evaporer et traiter le résidu à deux reprises par 10 ml de chloroforme et passer les solutions chloroformiques obtenues sur une colonne d'alumine. Evaporer l'éluat. On obtient le produit du titre sous forme d'huile.
*Rendement : 90%*

| *Composition centésimale :* | | | |
|---|---|---|---|
| *Calculée* | *C72,4* | *H8,8* | *N18,8* |
| *Trouvée* | *C72,2* | *H8,7* | *N18,9* |

*Caractéristiques spectrales :*
*Infrarouge: 1510, 1605, 1625 cm*^{*-1*}*ν C=C* ν *C=N*
**STADE C :2-tert.butyl-9-(3-diméthylaminopropyl)imidazo[1,2-a] benzimidazole, dichlorhydrate**
Dissoudre l'huile obtenue au stade B dans 15 ml d'éther anhydre et acidifier par l'éther chlorhydrique. Filtrer le précipité obtenu et purifier par reprécipitation par l'éther d'une solution éthanolique.
*Rendement : 92%*
*Point de fusion : 258-259°C*

| *Composition centésimale:* | | | | |
|---|---|---|---|---|
| *Calculée* | *C 58,2* | *H 7,6* | *Cl 19,1* | *N 15,1* |
| *Trouvée* | *C 57,9* | *H 7,8* | *Cl 18,8* | *N 15,2* |

### EXEMPLE 15:

### 2-TERT.BUTYL-9-(3-DIMETHYLAMINOPROPYL)IMIDAZO[1,2-a]BENZIMIDAZOLE, DIPERCHLORATE

En procédant comme dans l'exemple 14, mais en remplaçant au stade C l'acide chlohydrique par l'acide perchlorique, on obtient le produit du titre.
*Point de fusion : 236-237°C*

| *Composition centésimale :* | | | | |
|---|---|---|---|---|
| *Calculée* | *C 43,3* | *H 5,7* | *Cl 14,2* | *N 11,2* |
| *Trouvée* | *C 43,2* | *H 5,5* | *Cl 14,0* | *N 11,0* |

### EXEMPLE 16:

### 9-(2-MORPHOLINOETHYL)-2-(4-HYDROXYPHENYL)IMIDAZO[1,2-a]BENZIMIDAZOLE, DICHLORHYDRATE

**Stade A:9-(2-morpholinoéthyl)-2-(4-hydroxyphényl)imidazo[1,2-a] benzimidazole**
Un mélange de 2-amino-1-(2-morpholinoéthyl)benzimidazole (10 mmol ; 2,46 g) et de bromure de 4-hydroxyphénacyle (10 mmol ; 2,15 g) (obtenu par bromuration de la para-hydroxyacétophénone avec du bromure cuivrique dans un mélange chloroforme-acétate d'éthyle) est porté à reflux dans 10 ml de diméthylformamide pendant 1 heure. Après refroidissement, le mélange est versé dans 50 ml d'eau et alcalinisé par de l'ammoniaque. L'huile fournie est séparée, traitée à l'eau. Le résidu cristallisé est filtré, séché et recristallisé dans l'éthanol.
*Rendement : 52%*
*Point de fusion : 228-229°C*

| *Composition centésimale :* | | | |
|---|---|---|---|
| *Calculée* | *C 69,6* | *H 6,1* | *N 15,5* |
| *Trouvée* | *C 69,5* | *H 6,2* | *N 15,5* |

*Caractéristiques spectrales :*
*Infrarouge: 3350-3200 cm*^{*-1*}ν *OH*
*1500, 1600, 1605cm*^{*-1*}*ν C=C* ν *C=N*
*Résonance Magnétique Nucléaire :* ^{*1*}*H, (CDCl*_{*3*}*)*
δ = *2,48 ppm ; triplet, 4H, N(CH*_{*2*}*)*_{*2*} *morpholine*
δ = *2,78 ppm ; triplet, 2H, CH*_{*2*}*-(morpholine)*
*δ = 3,56 ppm ; triplet, 4H, (CH*_{*2*}*)*_{*2*}*O*
δ = *4,26 ppm ; triplet, 2H, N-CH*_{*2*}*-CH*_{*2*}*-morpholine*
δ = *6,85-7,45 ppm ; multiplet, 9H, aromatiques*
**Stade B:9-(2-morpholinoéthyl)-2-(4-hydroxyphényl)imidazo[1,2-a] benzimidazole, dichlorhydrate**
Chauffer 5 mmol (1,81 g) de la base obtenue au stade A dans 10 ml d'éthanol et acidifier par une solution isopropanolique d'acide chlorhydrique. Refroidir, filtrer, laver à l'éthanol et à l'acétone et recristalliser dans un mélange éthanol/eau.
*Rendement : 81%*
*Point de fusion : 276-277°C*

| *Composition centésimale :* | | | | |
|---|---|---|---|---|
| *Calculée* | *C 57,9* | *H 5,6* | *Cl 16,3* | *N 12,9* |
| *Trouvée* | *C 57,7* | *H 5,5* | *Cl 16,0* | *N 12,8* |

Ce produit peut également être obtenu en utilisant le mode opératoire de l'exemple 11, mais en remplaçant au stade A :
- le bromure de 4-méthylphénacyle, par le bromure de 4-méthoxyphénacyle, et le
- 2-amino-1-[(2-diéthylamino)éthyl]benzimidazole, par le 2-amino-1-[(2-morpholino)éthyl]benzimidazole.

La réaction de cyclisation dans l'acide bromhydrique bouillant s'accompagne de la saponification du groupement méthoxy et l'on obtient alors le bromhydrate.

### EXEMPLE 17:

### 9-(2-MORPHOLINOETHYL)-2-(3-HYDROXYPHENYL)IMIDAZO[1,2-a]BENZIMIDAZOLE, DIBROMHYDRATE

En procédant comme dans l'exemple 11, mais en remplaçant au stade A :
- le bromure de 4-méthylphénacyle, par le bromure de 3-méthoxyphénacyle, et le
- 2-amino-1-(2-diéthylaminoéthyl)benzimidazole, par le 2-amino-1-(2-morpholinoéthyl)benzimidazole,
on obtient le produit du titre.
*Point de fusion : 275-277°C*

| *Composition centésimale :* | | | | |
|---|---|---|---|---|
| *Calculée* | *C 48,1* | *H 4,6* | *Br 30,5* | *N 10,7* |
| *Trouvée* | *C 48,2* | *H 4,5* | *Br 30,2* | *N 10,5* |

### EXEMPLES 18 A 24 :

En procédant comme dans l'exemple 11, mais utilisant comme matières premières
- les dérivés du benzimidazole correctement substitués,
- les halogènures d'aroyle méthyle correctement substitués,
on obtient :

### EXEMPLE 18:

### 9-DIETHYLAMINOETHYL-2-(3,4-DIMETHOXYPHENYL)IMIDAZO[1,2-a]BENZIMIDAZOLE, SULFATE

*Point de fusion : 248-249°C (décomposition)*

| *Composition centésimale :* | | | | |
|---|---|---|---|---|
| *Calculée* | *C 56,3* | *H 6,2* | *N 11,4* | *S 6,5* |
| *Trouvée* | *C 56,2* | *H 6,0* | *N 11,5* | *S 6,3* |

*Caractéristiques spectrales :*
*Infrarouge:1510, 1600, 1610cm*^{*-1*}*ν (C=C ν C=N)*
*Résonance Magnétique Nucléaire : (CDCl*_{*3*}*)*
δ = *0,92 ppm ; triplet, 6H, 2(OCH*_{*3*}*)*
δ *= 2,54 ppm ; quadruplet, 4H, 2(CH*_{*2*}*-CH*_{*3*}*)*
δ = *2,88 ppm ; triplet, 2H, CH*_{*2*}*CH*_{*2*}*N(Et)*_{*2*}
δ = *4,20 ppm ; triplet, 2H, CH*_{*2*}*CH*_{*2*}*N(Et)*_{*2*}

### EXEMPLE 19:

### 9-DIETHYLAMINOETHYL-2-(3,4-DIHYDROXYPHENYL)IMIDAZO[1,2-a]BENZIMIDAZOLE, DINITRATE

*Point de fusion : 198-199°C (décomposition)*

| *Composition centésimale :* | | | |
|---|---|---|---|
| *Calculée* | *C 51,4* | *H 5,3* | *N 17,1* |
| *Trouvée* | *C 51,2* | *H 5,4* | *N 17,2* |

*Caractéristiques spectrales :*
*Infrarouge: 1510, 1590, 1605cm*^{*-1*}*ν C=C ν C=N*
*3200-3350cm*^{*-1*}ν *OH*

### EXEMPLE 20 :

### 9-(2-DIETHYLAMINOETHYL)-2-(3,4-DIHYDROXYPHENYL)IMIDAZO[1,2-a] BENZIMIDAZOLE, CHLORHYDRATE

*Point de fusion : 276-278 °C*

| *Composition centésimale :* | | | | |
|---|---|---|---|---|
| *Calculée* | *C 57,7* | *H 6,0* | *Cl 16,2* | *N 12,8* |
| *Trouvée* | *C 57,5* | *H 6,3* | *Cl 16,6* | *N 13,0* |

*Caractéristiques spectrales :*
*Infrarouge: 1510, 1590, 1605cm*^{*-1*}*ν C=C ν C=N*
*3200-3350cm*^{*-1*}ν *OH*

### EXEMPLE 21 :

### 9-(2-PIPERIDINOETHYL)-2-(3,4-DIHYDROXYPHENYL)IMIDAZO[1,2-a]BENZIMIDAZOLE, DIBROMHYDRATE

*Point de fusion : 297-298°C (décomposition)*

| *Composition centésimale :* | | | | |
|---|---|---|---|---|
| *Calculée* | *C 49,1* | *H 4,9* | *Br 29,7* | *N 10,4* |
| *Trouvée* | *C 48,9* | *H 5,2* | *Br 29,5* | *N 10,3* |

### EXEMPLE 22 :

### 9-(2-PIPERIDINOETHYL)-2-(3,4-DIHYDROXYPHENYL)IMIDAZO[1,2-a]BENZIMIDAZOLE, DINITRATE

*Point de fusion : 215-216°C*

| *Composition centésimale :* | | | |
|---|---|---|---|
| *Calculée* | *C 52,6* | *H 5,2* | *N 16,7* |
| *Trouvée* | *C 52,4* | *H 5,2* | *N 16,7* |

*Caractéristiques spectrales :*
*Infrarouge (base): 1500, 1595, 1605cm*^{*-1*} *ν C=C ν C=N*
*3200-3600cm*^{*-1*}ν *OH*

### EXEMPLE 23 :

### 9-(2-PIPERIDINOETHYL)-2-(3,4-DIHYDROXYPHENYL)IMIDAZO[1,2-a]BENZIMIDAZOLE, DICHLORHYDRATE

*Point de fusion : 293-294°C*

| *Composition centésimale :* | | | | |
|---|---|---|---|---|
| *Calculée* | *C 58,8* | *H 5,8* | *CI 15,8* | *N 12,5* |
| *Trouvée* | *C 58,6* | *H 5,8* | *CI 15,7* | *N 12,7* |

### EXEMPLE 24 :

### 9-(2-DIETHYLAMINOETHYL)-2-(1-METHYL-2-BENZIMIDAZOLYL)IMIDAZO[1,2-a] BENZIMIDAZOLE, DIBROMHYDRATE, HYDRATE

*Point de fusion : 280-281°C*

| *Composition centésimale :* | | | | |
|---|---|---|---|---|
| *Calculée* | *C 48,8* | *H 5,3* | *Br 28,2* | *N 14,9* |
| *Trouvée* | *C 48,6* | *H 5,5* | *Br 28,4* | *N 14,7* |

### EXEMPLE 25 :

### 9-(2-DIETHYLAMINOETHYL)-2-(2-THIENYL)IMIDAZO[1,2-a]BENZIMIDAZOLE, DIPERCHLORATE

*Point de fusion : 139-140°C*
*Caractéristiques spectrales : R.M.N* ^{*1*}*H (CF*_{*3*} *COOD)*
*δ = 1,00 ppm ; 6H, triplet, 2CH*_{*3*}
*δ = 3,14 ppm ; 4H, quadruplet, 2 CH*_{*2*}*-CH*_{*3*}
*δ = 3,49 ppm ; 2H, triplet, CH*_{*2*}*N-(CH*_{*2*}*CH*_{*3*}*)*_{*2*}
*δ = 4,60 ppm ; 2H; triplet, CH*_{*2*}*CH*_{*2*}*N(CH*_{*2*}*CH*_{*3*}*)*_{*2*}

### EXEMPLE 26 :

### 9-(2-DIETHYLAMINOETHYL)-2-(2-THIENYL)IMIDAZO[1,2-a]BENZIMIDAZOLE, PHOSPHATE

*Point de fusion : 188-189°C (décomposition)*
*Caractéristiques spectrales : R.M.N* ^{*1*}*H (CF*_{*3*} *COOD)*
*δ = 1,10 ppm ; 6H, triplet, 2CH*_{*3*}
*δ = 3,18 ppm ; 4H, quadruplet, 2 CH*_{*2*}*-CH*_{*3*}
*δ = 3,53 ppm ; 2H, triplet, CH*_{*2*}*N-(CH*_{*2*}*CH*_{*3*}*)*_{*2*}
*δ = 4,70 ppm ; 2H, triplet, CH*_{*2*}*CH*_{*2*}*N(CH*_{*2*}*CH*_{*3*}*)*_{*2*}

### EXEMPLE 27 :

### 9-(2-PIPERIDINOETHYL)-2-(2-THIENYL)IMIDAZO[1,2-a]BENZIMIDAZOLE, DIBROMHYDRATE

*Point de fusion : 295,5-296°C (décomposition)*

| *Composition centésimale :* | | | | | |
|---|---|---|---|---|---|
| *Calculée* | *C 46,9* | *H 4,7* | *Br 31,2* | *N 10,9* | *S 6,3* |
| *Trouvée* | *C 47,0* | *H 4,9* | *Br 30,9* | *N 10,9* | *S 6,3* |

*Caractéristiques spectrales :*
*Infrarouge (base)*: *1500, 1600, 1615cm*^{*-1*} ν *C=C ν C=N*
*3200-3600 cm*^{*-1*}ν *OH*

### EXEMPLE 28 :

### 9-(2-PIPERIDINOETHYL)-2-(2-THIENYL)IMIDAZO[1,2-a]BENZIMIDAZOLE, SULFATE

*Point de fusion : 265-266°C (décomposition)*

| *Composition centésimale :* | | | | |
|---|---|---|---|---|
| *Calculée* | *C 53,5* | *H 5,4* | *N 12,5* | *S 14,3* |
| *Trouvée* | *C 53,4* | *H 5,7* | *N 12,7* | *S 14,5* |

### EXEMPLE 29 :

### 9-(2-PIPERIDINOETHYL)-2-(5-BROMO-2-THIENYL)IMIDAZO[1,2-a]BENZIMIDAZOLE, DICHLORHYDRATE

**STADE A :2-amino-3-(5-bromo-2-thénoylméthyl)-1-(2-pipéridinoéthyl) benzimidazole, bromhydrate**
Ajouter une solution de 5-bromo-2-bromoacétylthiophène (5,68 g, 20 mmol) dans 30 ml d'éther à une solution chaude de 2-amino-1-(2-pipéridinoéthyl) benzimidazole (4,88 g, 20 mmol) dans 150 ml d'acétone. Agiter et maintenir à température ambiante pendant cinq heures. Essorer le précipité, laver à l'acétone puis à l'éther pour obtenir le produit du titre. *Rendement : 82%*
*Point de fusion : 185°C (décomposition)*

| *Composition centésimale :* | | | | | |
|---|---|---|---|---|---|
| *Calculée* | *C 45,4* | *H 4,6* | *Br 30,2* | *N 10,6* | *S 6,1* |
| *Trouvée* | *C 45,3* | *H 4,8* | *Br 30,6* | *N 10,7* | *S 6,5* |

*Caractéristiques spectrales :*
*Infrarouge (base)*: *1680cm*^{*-1*}ν *C=N*
*1695cm*^{*-1*} ν *CO*
*3120, 3315 cm*^{*-1*}*ν NH*_{*2*}
**STADE B :9-(2-piperidinoéthyl)-2-(5-bromo-2-thiènyl)imidazo[1,2-a] benzimidazole, dichlorhydrate**
Le bromhydrate obtenu au stade A est porté à fusion 20 minutes à 190°C. Après refroidissement, le produit obtenu est traité par 10 ml d'ammoniaque et extrait au benzène. Les phases organiques sont réunies, concentrées et passées sur une colonne d'alumine que l'on élue au benzène. Acidifier l'éluant par une solution d'éther chlorhydrique. Le précipité formé est essoré, lavé à l'acétone puis à l'éther et recristallisé dans l'éthanol.
*Rendement : 72%*
*Point de fusion : 240-241°C (décomposition)*

| *Composition centésimale :* | | | | | | |
|---|---|---|---|---|---|---|
| *Calculée* | *C 47,8* | *H 4,6* | *Br 15,9* | *Cl 14,1* | *N 11,2* | *S 6,4* |
| *Trouvée* | *C 47,6* | *H 4,7* | *Br 15,8* | *CI 14,3* | *N 11,0* | *S 6,2* |

*Caractéristiques spectrales :*
*Infrarouge :1503, 1595, 1615cm*^{*-1*}*ν C=C ν C=N*

### EXEMPLE 30 :

### 2-(5-BROMO-2-THIENYL)-9-(2-MORPHOLINOETHYL)IMIDAZO[1,2-a]BENZIMIDAZOLE, DICHLORHYDRATE

**STADE A :Bromhydrate de 2-amino-3-(5-bromo-2-thénoylméthyl)-1-(2-morpholinoéthyl)benzimidazole**
Dissoudre 2,46 g (10 mmol) de 2-amino-1-(2-morpholinoéthyl)benzimidazole dans 120 ml d'acétone sous chauffage. Ajouter ensuite le 5-bromo 2-bromoacétyl thiophène. Après agitation, abandonner à température ambiante 3 à 4 heures. Le précipité de bromhydrate de 2-amino-3-(5-bromo-2-thénoylméthyl)-1-(2-morpholinoéthyl)benzimidazole est filtré, lavé à deux reprises par 20 ml d'acétone et à l'éther (20 ml) pour obtenir 4,7 g du produit du titre.
*Rendement : 88%*
*Point de fusion : 207-208°C*

| *Composition centésimale :* | | | | | |
|---|---|---|---|---|---|
| *Calculée* | *C 43,0* | *H 4,2* | *Br 30,1* | *N 10,6* | *S 6,1* |
| *Trouvée* | *C 43,3* | *H 4,3* | *Br 30,5* | *N 10,5* | *S 5,9* |

*Caractéristiques spectrales :*
*Infrarouge: 1690cm*^{*-1*} *ν C=O*
*1680cm*^{*-1*} ν *C=N*
*3120, 3310 cm*^{*-1*}*ν NH*_{*2*}
**STADE B :2-(5-bromo-2-thiényl)-9-(2-morpholinoéthyl)imidazo[1,2-a] benzimidazole**
Le bromhydrate obtenu au stade A est porté au reflux 9 heures dans 50 ml d'eau en présence de 2,1 g d'hydrogénocarbonate de sodium. L'huile formée est extraite à trois reprises par 15 ml de chloroforme ; les phases organiques sont réunies et concentrées par évaporation puis le résidu est purifié par chromatographie sur colonne d'alumine éluée au chloroforme. La première fraction est constituée du produit du titre.
*Point de fusion : 187°C*

| *Composition centésimale :* | | | | | |
|---|---|---|---|---|---|
| *Calculée* | *C 52,9* | *H 4,4* | *Br 18,5* | *N 13,0* | *S 7,4* |
| *Trouvée* | *C 53,0* | *H 4,5* | *Br 18,2* | *N 13,3* | *S 7,2* |

*Caractéristiques spectrales :*
*Infrarouge: 1500, 1595, 1620cm*^{*-1*} *ν C=C ν C=N*
*Caractéristiques spectrales : R.M.N (CDCl*_{*3*}*)*
*δ = 2,46 ppm ; triplet, 4H, N(CH*_{*2*}*)*_{*2*} *(morpholine)*
*δ = 2,76 ppm ; triplet, 2H, CH*_{*2*}*-Morpholine*
*δ = 3,56 ppm ; triplet, 4H, (CH*_{*2*}*)*_{*2*}*O (morpholine)*
*δ = 4,22 ppm ; triplet, 2H, CH*_{*2*}*CH*_{*2*}*-Morpholine*
**STADE C :Dichlorhydrate de 2-(5-bromo-2-thiényl)-9-(2-morpholinoéthyl) imidazo [1,2-a] benzimidazole**
La base obtenue au stade précédent est dissoute dans l'acétone (6 mmol pour 50 ml) laquelle est acidifié par de l'acide chlorhydrique concentrée. Trente minutes plus tard, le précipité formé est filtré, lavé à l'acétone et à l'éther.
*Rendement : 91%*
*Point de fusion : 237-238°C (décomposition)*

| *Composition centésimale :* | | | | | | |
|---|---|---|---|---|---|---|
| *Calculée* | C 45,3 | *H 4,2* | *Br 15,8* | *CI 14,1* | *N 11,1* | *S 6,4* |
| *Trouvée* | *C 45,0* | *H 4,3* | *Br 15,4* | *CI 14,4* | *N 11,4* | *S 6,2* |

### EXEMPLE 31 :

### DIBROMHYDRATE DE 2-(2-FURYL)-9-(2-N,N-DIETHYLAMINOETHYL)IMIDAZO[1,2-a] BENZIMIDAZOLE

En procédant comme dans l'exemple 30, mais en remplaçant:
1-au stade A
   - le 2-amino 1-(2-morpholinoéthyl)benzimidazole par le 2-amino 1-(2-N,N-diéthylaminoéthyl)benzimidazole
   - et le 5-bromo-2-bromoacétylthiophène par le 2-bromoacétylfurane
2-au stade C
   - l'acide chlorhydrique par l'acide bromhydrique,
   on obtient le produit du titre.
   *Point de fusion : 269-270°C (décomposition)*
   *Caractéristiques spectrales : R.M.N* ^{*1*}*H (CF*_{*3*}*COOH)*
   *δ = 1,04 ppm ; triplet, 6H*, *(2CH*_{*3*}*)*
   *δ = 3,10 ppm ; quadruplet, 4H, (2CH*_{*2*}*)*
   *δ = 3,48 ppm ; triplet, 2H, CH*_{*2*}*N(Et)*_{*2*}
   *δ = 4,90 ppm ; triplet, 2H, CH*_{*2*}*CH*_{*2*}*N(Et)*_{*2*}

### EXEMPLE 32 :

### SULFATE DE 2-(2-FURYL)-9-(2-N,N-DIETHYLAMINOETHYL)IMIDAZO[1,2-a] BENZIMIDAZOLE

En procédant comme dans l'exemple 31, mais en remplaçant au stade C l'acide bromhydrique par l'acide sulfurique, on obtient le produit du titre.
*Point de fusion : 239-240°C (décomposition)*

| *Composition centésimale :* | | | | |
|---|---|---|---|---|
| *Calculée* | *C 54,3* | *H 5,7* | *N 13,3* | *S 7,6* |
| *Trouvée* | *C 54,3* | *H 6,0* | *N 13,5* | *S 8,0* |

*Caractéristiques spectrales :*
*Infrarouge: 1500, 1595, 1620cm*^{*-1*} *ν C=C ν C=N*

### EXEMPLE 33 :

### DIBROMHYDRATE DE 2-(2-FURYL)-9-(2-MORPHOLINOETHYL)IMIDAZO[1,2-a] BENZIMIDAZOLE

En procédant comme dans l'exemple 30, mais en remplaçant au stade A le 5-bromo-2-bromoacétyl thiophène par le 2-bromoacétylfurane et au stade C l'acide chlorhydrique par l'acide bromhydrique, on obtient le produit du titre.
*Point de fusion : 263-264°C (décomposition)*

| *Composition centésimale :* | | | | |
|---|---|---|---|---|
| *Calculée* | *C 45,6* | *H 4,5* | *Br 32,1* | *N 11,3* |
| *Trouvée* | *C 45,8* | *H 4,6* | *Br 31,9* | *N 11,5* |

*Caractéristiques spectrales :*
*Infrarouge: 1500, 1600, 1630cm*^{*-1*} *ν C=C ν C=N*
*Caractéristiques* spectrales : *R.M.N* ^{*1*}*H (CDCl*_{*3*}*)*
*δ = 2,48 ppm ; triplet, 4H, N(CH*_{*2*}*)*_{*2*} *Morpholine*
*δ = 2,79 ppm ; triplet, 2H, CH*_{*2*}*-Morpholine*
*δ = 3,57 ppm ; triplet, 4H, (CH*_{*2*}*)*_{*2*}*O (Morpholine)*
*δ = 4,26 ppm ; triplet, 2H, CH*_{*2*}*CH*_{*2*} *Morpholine*

### EXEMPLE 39 :

### DICHLORHYDRATE DE 2-METHYL-9-(2-MORPHOLINOETHYL)-3-(4-CHLOROBENZOYL) IMIDAZO[1,2-a]BENZIMIDAZOLE

**STADE A :Bromure de 2-amino-4-(4-chlorophénacyl)-1-(2-morpholionoéthyl) benzimidazolium**
Une solution de 2,33 g (10 mmole) de bromure de 4-chlorophénacyle dans 25 ml d'acétone est ajoutée à une solution chaude de 2-amino-1-(2-morpholinoéthyl)benzimidazole (2,46 g ; 10 mmol) dans 75 ml d'acétone. Le mélange est agité, porté à ébullition pendant 5 minutes et maintenu à température ambiante. Trois heures plus tard apparaît un précipité du produit du titre qui est filtré et lavé à l'acétone et à l'éther.
*Rendement : 95%*
*Point de fusion : 193-194°C*

| *Composition centésimale :* | | | | | |
|---|---|---|---|---|---|
| *Calculée* | *C 52,6* | *H 5,0* | *Br 16,6* | *Cl 7,4* | *N 11,7* |
| *Trouvée* | *C 52,5* | *H 5,1* | *Br 16,2* | *Cl 7,7* | *N 11,9* |

*Caractéristiques spectrales :*
*Infrarouge: 1700cm*^{*-1*} *ν C=O*
*1680cm*^{*-1*}*ν C=C*
*3170cm*^{*-1*} ν *NH*_{*2*}
**STADE B :2-Méthyl-9-(2-morpholinoéthyl)-3-(4-chlorobenzoyl)imidazo [1,2-a]benzimidazole**
2,4 g de produit obtenu au stade A sont mis en suspension dans 25 ml d'anhydride acétique et maintenus à température ambiante 24 heures. Le milieu réactionnel est ensuite chauffé au bain-marie jusqu'à dissolution du précipité formé puis porté à reflux 10 minutes. Après refroidissement la solution est versée dans 100 ml d'eau et, après décomposition de l'excès d'anhydride acétique par de l'ammoniaque, extraite à deux reprises au benzène. Les extraits réunis sont concentrés et passés sur une colonne d'alumine (éluant : benzene). L'éluant est évaporé pour donner le produit du titre.
*Rendement : 86%*
*Point de fusion : 147-148°C*

| *Composition centésimale :* | | | | |
|---|---|---|---|---|
| *Calculée* | *C 65,3* | *H 5,5* | *Cl 8,4* | *N 13,3* |
| *Trouvée* | *C 65,4* | *H 5,7* | *Cl 8,2* | *N 13,0* |

*Caractéristiques spectrales :*
*Infrarouge: 1500, 1595, 1625cm*^{*-1*}*ν C=C ν C=N*
*1640cm*^{*-1*}*ν CO*
**STADE C :Dichlorhydrate de 2-méthyl-9-(2-morpholionéthyl)-3-(4-chlorobenzoyl) imidazo[1,2-a]benzimidazole**
1,7 g de base obtenue au stade B est dissous dans 30 ml d'acétone et acidifié par de l'acide chlorhydrique concentré : le précipité du titre est séparé par filtration et recristallisé dans l'éthanol.
*Rendement : 89%*
*Point de fusion : 235-236°C*

| *Composition centésimale :* | | | | |
|---|---|---|---|---|
| *Calculée* | *C 55,7* | *H 5,1* | *Cl 21,5* | *N 11,3* |
| *Trouvée* | *C 55,4* | *H 5,1* | *Cl 21,1* | *N 11,4* |

### EXEMPLE 40 :

### DICHLORHYDRATE DE 2-METHYL-9-(2-PIPERIDINOETHYL)-3-(2-FUROYL)IMIDAZO [1,2-a]BENZIMIDAZOLE

En procédant de la même façon que dans l'exemple 39, mais en remplaçant, au stade A le bromure de 4-chlorophénacyle par le bromure de 2-furoylméthyle, et le 2-amino-1-(2-morpholinoéthyl)benzimidazole par le 2-amino-1-(2-pipéridinoéthyl)benzimidazole, on obtient le produit du titre.
*Point de fusion : 227-228°C*

| *Composition centésimale :* | | | | |
|---|---|---|---|---|
| *Calculée* | *C 58,8* | *H 5,8* | *Cl 15,8* | *N 12,5* |
| *Trouvée* | *C 58,6* | *H 5,7* | *Cl 15,5* | *N 12,7* |

*Caractéristiques spectrales :*
*Infrarouge: 1495, 1600, 1610cm*^{*-1*}*ν C=C ν C=N*
*1625cm*^{*-1*}ν CO

### EXEMPLE 41 :

### DICHLORHYDRATE DE 2-METHYL-9-(2-PIPERIDINOETHYL)-3-(2-THENOYL)IMIDAZO [1,2-a]BENZIMIDAZOLE

**STADE A : Bromure de 2-amino-1-(2-pipéridinoéthyl)-3-(2-thénoylméthyl) benzimidazolium**
Une solution éthérée de 2-bromoacétylthiophène (obtenu par bromuration du 2-acétylthiophène (1,9 g , 15 mmol) par 0,8 ml (15 mmol) de brome dans 10 ml de dioxanne et 15 ml d'éther) est ajoutée à une solution chaude de 2-amino-1-(2-pipéridinoéthyl)benzimidazole (2,44 g ; 10 mmol) dans 100 ml d'acétone. Agiter et maintenir à température ambiante. Le lendemain, le précipité du titre est recueilli par filtration, lavé à l'acétone et à l'éther.
*Rendement : 94%*
*Point de fusion : 198°C (décomposition)*

| *Composition centésimale :* | | | | | |
|---|---|---|---|---|---|
| *Calculée* | *C 53,3* | *H 5,6* | *Br 17,6* | *N 12,5* | *S 7,2* |
| *Trouvée* | *C 53,5* | *H 5,6* | *Br 17,4* | *N 12,3* | *S 7,0* |

*Caractéristiques spectrales :*
*Infrarouge: 1685cm*^{*-1*}*ν C=O*
*1675cm*^{*-1*}*ν C=N*
*3210, 3340cm*^{*-1*}*ν NH*_{*2*}
**STADE B :2-Méthyl-9-(2-pipéridinoéthyl)-3-(2-thénoyl)imidazo[1,2-a] benzimidazole**
Le bromure obtenu au stade A est porté à reflux dans 15 ml d'anhydride acétique jusqu'à dissolution complète. Refroidir et verser dans 50 ml d'eau froide. Neutraliser par une solution de carbonate de sodium et extraire à deux reprises par 10 ml de chloroforme. Réunir les phases organiques, passer sur une colonne d'alumine et évaporer. Recristalliser le résidu dans l'acétate d'éthyle.
*Rendement : 92%*
*Point de fusion : 125-126°C (décomposition)*

| *Composition centésimale :* | | | | |
|---|---|---|---|---|
| *Calculée* | *C 67,5* | *H 6,2* | *N 14,3* | *S 8,2* |
| *Trouvée* | *C 67,6* | *H 6,4* | *N 14,3* | *S 8,0* |

*Caractéristiques spectrales :*
*Infrarouge: 1510, 1590, 1605cm*^{*-1*}*ν C=C ν C=N*
*1625cm*^{*-1*}ν *C=O*
**STADE C :Dichlorhydrate de 2-méthyl-9-(2-pipéridinoéthyl)-3-(2-thénoyl) imidazo [1,2-a]benzimidazole**
Dissoudre 4 mmol du produit obtenu au stade précédent dans 20 ml d'acétone. Acidifier par l'acide chlorhydrique concentré. Filtrer et recristalliser dans l'éthanol.
*Rendement : 87%*
*Point de fusion : 232°C (décomposition)*

| *Composition centésimale :* | | | | | |
|---|---|---|---|---|---|
| *Calculée* | *C 56,8* | *H 5,6* | *Cl 15,2* | *N 12,0* | *S 6,9* |
| *Trouvée* | *C 56,3* | *H 5,5* | *Cl 15,6* | *N 12,2* | *S 7,0* |

### EXEMPLE 42 :

### SULFATE DE 2-METHYL-9-(2-PIPERIDINOETHYL)-3-(2-THENOYL)IMIDAZO[1,2-a] BENZIMIDAZOLE

En procédant comme dans l'exemple 41, mais en remplaçant au stade C l'acide chlorhydrique par l'acide sulfurique, on obtient le produit du titre.
*Point de fusion : 240-241°C (décomposition)*

| *Composition centésimale :* | | | |
|---|---|---|---|
| *Calculée* | *C 53,9* | *H 5,3* | *N 11,4* |
| *Trouvée* | *C 53,8* | *H 5,3* | *N 11,3* |

### EXEMPLE 43 :

### DICHLORHYDRATE DE 2-METHYL-9-(2-MORPHOLINOETHYL)-3-(5-BROMO-2-THENOYL)IMIDAZO[1,2-a]BENZIMIDAZOLE

En procédant comme dans l'exemple 41, mais en remplaçant au stade A :
- le 2-acétylthiophène par le 2-acétyl-5-bromothiophène,
- le 2-amino-1-(2-pipéridinoéthyl)benzimidazole par le 2-amino-1-(2-morpholinoéthyl)benzimidazole,
on obtient le produit du titre.
*Point de fusion : 249°C (décomposition)*

| *Composition centésimale :* | | | | | | |
|---|---|---|---|---|---|---|
| *Calculée* | *C 46,2* | *H 4,2* | *Br 14,6* | *Cl 13,0* | *N 10,3* | *S 5,9* |
| *Trouvée* | *C 46,5* | *H 4,4* | *Br 14,4* | *Cl 13,0* | *N 10,2* | *S 6,0* |

*Caractéristiques spectrales :*
*Infrarouge: 1500, 1600, 1610cm*^{*-1*} *ν C=C ν C=N*
*1625cm*^{*-1*} *ν CO*

### EXEMPLE 44 :

### DICHLORHYDRATE DE 2-TERT.BUTYL-1-(2-MORPHOLINOETHYL)IMIDAZO[1,2-a] BENZIMIDAZOLE

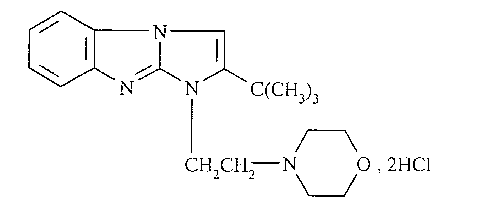

**STADE A :Bromhydrate de 2-(2-hydroxyéthylamino)-1-pivaloylméthyl benzimidazole**
Porter à reflux un mélange de 2-(2-hydroxyéthylamino)benzimidazole (1,77 g ; 10 mmol) et de bromopinacoline (1,35 g ; 10 mmol) dans 50 ml de propanol pendant 3 heures. Puis maintenir à température ambiante. Le lendemain, le précipité du titre est recueilli par filtration et lavé à l'acétone puis à l'éther.
*Rendement : 89%*
*Point de fusion : 212-213°C (décomposition)*

| *Composition centésimale :* | | | | |
|---|---|---|---|---|
| *Calculée* | *C 50,6* | *H 6,2* | *Br 22,1* | *N 11,8* |
| *Trouvée* | *C 50,5* | *H 6,5* | *Br 22,0* | *N 11,7* |

*Caractéristiques spectrales :*
*Infrarouge: 1720cm*^{*-1*}*ν C=O*
*3285, 3365cm*^{*-1*}*ν OH ν NH*
**STADE B :2-tert.butyl-1-(2-hydroxyéthyl)imidazo[1,2-a]benzimidazole**
Le bromhydrate obtenu au stade précédent est placé dans un récipient à 225°C pendant 10 minutes. Le produit obtenu est traité par de l'ammoniaque et extrait à trois reprises par 5 ml de chloroforme. Les phases organiques sont réunies, concentrées et passées sur une colonne d'alumine éluée au chloroforme. L'huile obtenue après évaporation cristallise par trituration dans l'éther de pétrole.
*Rendement : 70%*
*Point de fusion : 157-158°C*

| *Composition centésimale :* | | | |
|---|---|---|---|
| *Calculée* | *C 70,3* | *H 7,6* | *N 16,1* |
| *Trouvée* | *C 70,2* | *H 7,5* | *N 16,3* |

*Caractéristiques spectrales :*
*Infrarouge: 1640cm*^{*-1*} ν *C=N*
*3065, 3205cm*^{*-1*}*ν OH associée*
**STADE C :Chlorhydrate de 2-tert.butyl-1-(2-chloroéthyl)imidazo[1,2-a] benzimidazole**
Ajouter 0,93 ml de chlorure de thionyle à une suspension de 1,85 g (7,1 mmole) de dérivé obtenu au stade B dans 20 ml de chloroforme en agitant vigoureusement. Porter deux heures à reflux et refroidir. Evaporer et triturer le résidu dans l'éther de pétrole. Le précipité obtenu est recueilli par filtration et lavé à l'éther de pétrole.
*Rendement: 100%*
*Point de fusion : 200-201°C (décomposition)*

| *Composition centésimale:* | | | | |
|---|---|---|---|---|
| *Calculée* | *C 57,7* | *H 6,1* | *Cl 22,7* | *N 13,5* |
| *Trouvée* | *C 57,9* | *H 6,0* | *Cl 22,5* | *N 13,6* |

*Caractéristiques spectrales :*
*Infrarouge: 1520, 1620cm*^{*-1*} *ν C=C*
*2300, 2800cm*^{*-1*} ν *-N+H*
**STADE D :2-tert.butyl-1-(2-morpholinoéthyl)imidazo[1,2-a]benzimidazole**
Porter à ébullition 1 g (3,2 mmol) du produit obtenu au stade précédent dans 5 ml de morpholine pendant 6 heures. Refroidir puis verser dans 10 ml d'eau. Extraire à trois reprises au chloroforme. Réunir les phases organiques, laver à l'eau et passer sur une colonne d'alumine en éluant au chloroforme. Evaporer le solvant. On obtient le produit du titre.
*Rendement : 89%*

| *Composition centésimale :* | | | |
|---|---|---|---|
| *Calculée* | *C 69,9* | *H 8,0* | *N 17,2* |
| *Trouvée* | *C 70,0* | *H 7,8* | *N 17,4* |

*Caractéristiques spectrales : R.M.N* ^{*1*}*H*
*δ = 1,41 ppm ; singulet, 9H, C(CH*_{*3*}*)*_{*3*}
*δ = 1,50 ppm ; triplet, 4H,(CH*_{*2*}*)*_{*2*}*N*
*δ = 2,86 ppm ; triplet, 2H, CH*_{*2*}*-Morpholine*
*δ = 3,63 ppm ; triplet, 4H, (CH*_{*2*}*)*_{*2*}*O*
*δ = 4,28 ppm ; triplet, 2H, CH*_{*2*}*CH*_{*2*}*-Morpholine*
**STADE E :Dichlorhydrate de 2-tert.butyl-1-(2-morpholinoéthyl)imidazo [1,2-a]benzimidazole**
L'huile obtenue au stade D est dissoute dans 15 ml d'acétone et la solution obtenue est acidifiée par l'éther chlorhydrique. Filtrer le précipité obtenu. Laver à l'acétone et sécher.
*Rendement : 85%*
*Point de fusion : 288°C (décomposition)*

| *Composition centésimale :* | | | | |
|---|---|---|---|---|
| *Calculée* | *C 57,1* | *H 7,1* | *Cl 17,8* | *N 14,0* |
| *Trouvée* | *C 57,3* | *H 7,2* | *Cl 17,5* | *N 13,8* |

*Caractéristiques spectrales :*
*Infrarouge: 1665cm*^{*-1*} *ν C=N*^{*+*}
*2200-2700 ; 3300-3450cm*^{*-1*} ν *NH*^{*+*}

### EXEMPLE 45 :

### DICHLORHYDRATE DE 2-TERT.BUTYL-1-(2-PIPERIDINOETHYL)IMIDAZO[1,2-a] BENZIMIDAZOLE

En procédant comme dans l'exemple 44, mais en remplaçant au stade D la morpholine par la pipéridine, on obtient le produit du titre.
*Point de fusion : 231°C (décomposition)*

| *Composition centésimale :* | | | | |
|---|---|---|---|---|
| *Calculée* | *C 60,4* | *H 7,6* | *Cl 17,8* | *N 14,1* |
| *Trouvée* | *C 60,6* | *H 7,9* | *Cl 17,5* | *N 14,0* |

*Caractéristiques spectrales :*
*Infrarouge: 1660cm*^{*-1*}*ν C=N*^{*+*}
*2200-2700 ; 3300-3450cm*^{*-1*}*ν NH*^{*+*}

### EXEMPLE 46 :

### DICHLORHYDRATE DE 2-TERT.BUTYL-1-(2-N,N-DIETHYLAMINOETHYL)IMIDAZO [1,2-a]BENZIMIDAZOLE

En procédant comme dans l'exemple 44, mais en remplaçant au stade D la morpholine par la N,N-diéthylamine, on obtient le produit du titre.
*Point de fusion (hydrate) : 240-241°C (décomposition)*

| *Composition centésimale:* | | | | |
|---|---|---|---|---|
| *Calculée* | *C 56,6* | *H 8,0* | *Cl 17,6* | *N 13,9* |
| *Trouvée* | *C 56,6* | *H 7,8* | *Cl 17,3* | *N 14,0* |

*Caractéristiques spectrales : R.M.N* ^{*1*}*H (CDCl*_{*3*}*) Base*
*δ = 0,72 ppm ; triplet, 6H, 2CH*_{*3*}
*δ = 1,41 ppm ; singulet, 9H, C(CH*_{*3*}*)*_{*3*}
*δ = 2,35 ppm ; quadruplet, 4H, N(CH*_{*2*}*)*_{*2*}
*δ = 2,76 ppm ; triplet, 2H, CH*_{*2*}*-N(Et)*_{*2*}
*δ = 4,18 ppm ; triplet, 2H, CH*_{*2*}*-CH*_{*2*}*-N(Et)*_{*2*}
*δ = 6,9 ppm ; singulet, 1H, imidazole*
*δ = 7,12-7,45 ppm ; massif, 4H, aromatiques*

### EXEMPLE 47:

### DICHLORHYDRATE DE 10-(2-DIETHYLAMINOETHYL)-2,3,4,10-TETRAHYDROPYRIMIDINO[1,2-a]BENZIMIDAZOLE (DIHYDRATE)

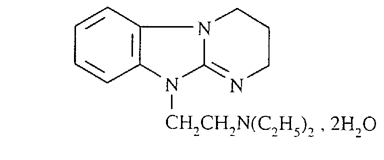

**STADE A : 10-(2-diéthylaminoéthyl)-2,3,4,10-tétrahydropyrimidino[1,2-a] benzimidazole**
Porter à reflux cinq heures un mélange de 2,32 g (10 mmol) de 2-amino-1-diéthylaminoéthylbenzimidazole et de 1,2 ml (10 mmol) de 1-bromo-3-chloropropane dans 10 ml de xylène. Ajouter 50 ml d'eau et agiter jusqu'à l'obtention d'une solution. Séparer la phase aqueuse, alcaliniser par de l'ammoniaque jusqu'à pH 9 et extraire à trois reprises au chloroforme. Réunir les phases organiques, concentrer et éluer au chloroforme sur une colonne d'alumine. Recueillir la première fraction et évaporer le solvant. Sécher.
*Rendement : 86%*

| *Composition centésimale :* | | | |
|---|---|---|---|
| *Calculée* | *C 66,2* | *H 9,0* | *N 19,3* |
| *Trouvée* | *C 66,0* | *H 9,3* | *N 19,5* |

*Caractéristiques spectrales :*
*Infrarouge: 1660cm*^{*-1*}*ν C=N*
**STADE B :Dichlorhydrate de 10-(2-diéthylaminoéthyl)-2,3,4,10-tétrahydropyrimidino[1,2-a]benzimidazole (dihydrate)**
La base obtenue au stade précédent est dissoute dans 20 ml d'éther et la solution est acidifiée par de l'éther chlorhydrique. Filtrer le précipité obtenu, laver à l'éther et sécher. *Rendement : 91,6%*
*Point de fusion : 78-80°C (décomposition)*

| *Composition centésimale (dihydrate):* | | | | |
|---|---|---|---|---|
| *Calculée* | *C 50,4* | *H 7,9* | *Cl 18,6* | *N 14,7* |
| *Trouvée* | *C 50,1* | *H 8,2* | *Cl 18,4* | *N 14,5* |

### EXEMPLE 48 :

### CHLORHYDRATE DE 10-PHENACYL-2,3,4,10-TETRAHYDROPYRIMIDINO[1,2-a] BENZIMIDAZOLE

**STADE A :2-(3-hydroxypropylamino)benzimidazole**
Chauffer à 145-150°C pendant deux heures un mélange de 5 g (25 mmol) d'acide 2-benzimidazole sulfonique et de 5,7 ml (75 mmole) de 3-amino propanol. Refroidir à 90°C et ajouter 15 ml d'eau froide en agitant vigoureusement jusqu'à formation d'un précipité. Maintenir une nuit à température voisine de 4°C, filtrer le précipité du titre, laver à l'eau froide et sécher.
*Rendement : 95%*
*Point de fusion : 139-140°C*

| *Composition centésimale :* | | | |
|---|---|---|---|
| *Calculée* | *C 62,8* | *H 6,9* | *N 22,0* |
| *Trouvée* | *C 62,6* | *H 6,9* | *N 22,3* |

*Caractéristiques spectrales :*
*Infrarouge: 1500, 1580, 1600cm*^{*-1*}*ν C=C*
*1650cm*^{*-1*}*ν C=N*
*3070-3240cm*^{*-1*}*ν OH ν NH*
**STADE B :Bromhydrate de 2-(3-hydroxypropylamino)-1-phénacyl benzimidazole**
Porter 5 à 6 heures au reflux un mélange de 1,91 g (10 mmol) du produit obtenu au stade A et 1,99 g (10 mmol) de bromure de phénacyle dans 20 ml d'isopropanol. Refroidir. Recueillir le précipité du titre par filtration et laver à l'acétone. Recristalliser dans l'éthanol.
*Rendement : 82%*
*Point de fusion : 237-238°C*

| *Composition centésimale :* | | | | |
|---|---|---|---|---|
| *Calculée* | *C 55,4* | *H 5,2* | *Br 20,5* | *N 10,8* |
| *Trouvée* | *C 55,6* | *H 5,1* | *Br 20,0* | *N 10,5* |

*Caractéristiques spectrales :*
*Infrarouge: 1700cm*^{*-1*}ν CO
*1650cm*^{*-1*}*ν C=N*
*3100-3240cm*^{*-1*}ν *OH ν NH*
**STADE C :10-Phénacyl-2,3,4,10-tétrahydropyrimidino[1,2-a]benzimidazole**
Ajouter lentement et sous agitation 0,7 ml (10 mmol) de chlorure de thionyle à une suspension de 1,95 g (5 mmol) du produit obtenu au stade B dans 25 ml de chloroforme. Porter à reflux jusqu'à disparition du produit de départ (suivie par chromatographie couche mince) et de son dérivé chloré. Evaporer le milieu réactionnel, traiter le résidu par de l'ammoniaque et extraire à trois reprises au chloroforme. Réunir les phases organiques, évaporer et chauffer le résidu à 110-120°C pendant 20 minutes ; traiter par l'ammoniaque et au chloroforme. La phase chloroformique est passée sur une colonne d'alumine éluée au chloroforme. Recueillir la fraction de Rf ≈ 0,3. Evaporer le solvant et recristalliser dans l'acétonitrile le produit du titre.
*Rendement : 86,2%*
*Point de fusion : 168-169°C (décomposition)*

| *Composition centésimale:* | | | |
|---|---|---|---|
| *Calculée* | *C 74,2* | *H 5,9* | *N 14,4* |
| *Trouvée* | *C 74,2* | *H 5,7* | *N 14,2* |

*Caractéristiques spectrales :*
*Infrarouge: 1505, 1590, 1605cm*^{*-1*} *ν C=C*
*1660cm*^{*-1*}*ν C=N*
*1690cm*^{*-1*}*ν C=O*
*R.M.N* ^{*1*}*H (CDCl*_{*3*}*) : Base*
*δ = 1,89 ppm ; massif, 2H, CH*_{*2*}*(3)*
*δ = 3,45 ppm ; triplet, 2H, N-CH*_{*2*} *(4)*
*δ = 3,75 ppm ; triplet, 2H, N-CH*_{*2*} *(2)*
*δ = 5,10 ppm ; singulet, 2H, CH*_{*2*}*-CO*
*δ = 6,80 ppm ; massif, 9H, aromatiques*
**STADE D :Chlorhydrate de 10-phénacyl-2,3,4,10-tétrahydropyrimidino [1,2-a] benzimidazole**
Le produit obtenu au stade C est dissous dans l'acétone. On traite la solution obtenue par de l'acide chlorhydrique concentré. Essorer le précipité et recristalliser dans l'éthanol.
*Point de fusion : 288-289°C*

| *Composition centésimale :* | | | | |
|---|---|---|---|---|
| *Calculée* | *C 66,0* | *H 5,5* | *Cl 10,8* | *N 12,8* |
| *Trouvée* | *C 65,7* | *H 5,7* | *Cl 11,0* | *N 12,5* |

*Caractéristiques spectrales :*
*Infrarouge: 1670cm*^{*-1*}*ν (C=N*^{*+*}*-H)*
*1700cm*^{*-1*}*ν (C=O)*

### EXEMPLE 49 :

### 10-PHENACYL-2,3,4,10-TETRAHYDROPYRIMIDINO[1,2-a]BENZIMIDAZOLE

**STADE A :[1H]-1,2,3,4-tétrahydropyrimidino[1,2-a]benzimidazole**
A une solution obtenue en chauffant de 2-(3-hydroxypropylamino)benzimidazole (obtenu dans l'exemple 48, stade A) dans le diméthylformamide, ajouter goutte à goutte, à température ambiante une solution de chlorure de thionyle dans le diméthylformamide. Porter ensuite quatre heures au reflux, refroidir, diluer à l'eau et neutraliser par l'ammonique. Trente minutes plus tard, séparer le produit du titre, laver à l'eau et recristalliser dans l'acétonitrile.
*Rendement : 82%*
*Point de fusion : 210-211°C*

| *Composition centésimale :* | | | |
|---|---|---|---|
| *Calculée* | *C 69,4* | *H 6,5* | *N 24,1* |
| *Trouvée* | *C 69,3* | *H 6,4* | *N 24,3* |

*Caractéristiques spectrales :*
*Infrarouge: 1620cm*^{*-1*}ν *C=N*
*R.M.N* ^{*1*}*H (CDCl*_{*3*}*) :*
*δ = 2,12 ppm ; triplet, 2H, CH*_{*2*} *(3)*
*δ = 3,47 ppm ; triplet, 2H, CH*_{*2*} *(4)*
*δ = 3,91 ppm ; triplet, 2H, CH*_{*2*} *(2)*
*δ = 6,95-7,20 ppm ; 5H, aromatiques*
**STADE B :10-Phénacyl-2,3,4,10-tétrahydropyrimidino[1,2-a]benzimidazole**
1,38 g (8 mmol) de produit obtenu au stade A est dissous dans 50 ml de xylène à 120°C. Refroidir à 50°C et ajouter 1,59 g (8 mmol) de bromure de phénacyle. Agiter vigoureusement jusqu'à dissolution puis porter à ébullition et laisser une heure à reflux. Refroidir, séparer le précipité formé, laver à l'éther de pétrole, sécher et traiter par l'ammoniaque. Séparer le précipité du titre et recristalliser dans l'acétonitrile.
Rendement : 95%
*Point de fusion : 168-169°C (décomposition)*

### EXEMPLE 50 :

### CHLORHYDRATE DE 10-(4'-CHLOROPHENACYL)-2,3,4,10-TETRAHYDROPYRIMIDINO [1,2-a]BENZIMIDAZOLE

En procédant comme dans les exemples 48 et 49, mais en remplaçant aux stades B le bromure de phénacyle par le bromure de 4-chlorophénacyle, on obtient le produit du titre.
*Point de fusion : 270-271°C*

| *Composition centésimale :* | | | | |
|---|---|---|---|---|
| *Calculée* | *C 59,7* | *H 4,7* | *Cl 19,6* | *N 11,6* |
| *Trouvée* | *C 59,5* | *H 4,5* | *Cl 19,3* | *N 11,5* |

*Caractéristiques spectrales :*
*Infrarouge: 1670cm*^{*-1*}ν *C=N*^{*+*}
*1700cm*^{*-1*}*ν C=O*
*R.M.N* ^{*1*}*H (CDCl*_{*3*}*) :*
*δ = 1,89 ppm ; massif, 2H, CH*_{*2*} *(3)*
*δ = 3,45 ppm ; massif 2H, CH*_{*2*} *(4)*
*δ = 3,75 ppm ; massif 2H, CH*_{*2*} *(2)*
*δ = 5,20 ppm ; singulet, 2H, CH*_{*2*}*-CO*

### EXEMPLE 51 :

### CHLORHYDRATE DE 10-(4'-METHOXYPHENACYL)-2,3,4,10-TETRAHYDROPYRIMIDINO [1,2-a]BENZIMIDAZOLE

En procédant comme dans les exemples 48 et 49, mais en remplaçant aux stades B le bromure de phénacyle par le bromure de 4-méthoxyphénacyle, on obtient le produit du titre.
*Point de fusion : 260-262°C (décomposition)*

| *Composition centésimale :* | | | | |
|---|---|---|---|---|
| *Calculée* | *C 63,8* | *H 5,6* | *Cl 9,9* | *N 11,7* |
| *Trouvée* | *C 63,9* | *H 5,5* | *Cl 10,1* | *N 11,5* |

*Caractéristiques spectrales :*
*Infrarouge: 1670cm*^{*-1*} ν *C=N*^{*+*}*H*
*1690cm*^{*-1*}*ν CO*
*R.M.N* ^{*1*}*H (CDCl*_{*3*}*)*:
*δ = 1,89 ppm ; quintuplet, 2H, CH*_{*2*} *(3)*
*δ = 3,48 ppm ; triplet, 2H, CH*_{*2*} *(4)*
*δ* = *3,25 ppm ; triplet, 2H, CH*_{*2*} *(2)*
*δ = 3,78 ppm ; singulet, 3H, OCH*_{*3*}
*δ = 5,10 ppm ; singulet, 2H, CH*_{*2*}*-CO*

### EXEMPLE 52:

### CHLORHYDRATE DE 10-[2-HYDROXY-2-(4-CHLOROPHENYL)ETHYL]-2,3,4,10-TETRAHYDROPYRIMIDINO[1,2-a]BENZIMIDAZOLE

**STADE A :10-[2-hydroxy-2-(4-chlorophényl)éthyl]-2,3,4,10-tétrahydro pyrimidino [1,2-a]benzimidazole**
A une suspension de 3,0 g (9,3 mmol) de 10-(4-chlorophénacyl)-2,3,4,10-tétrahydropyrimidino[1,2-a]benzimidazole (obtenu dans l'exemple 50) dans 20 ml d'éthanol ajouter par petites portions et sous agitation vigoureuse 0,35 g (9,3 mmol) de borohydrure de sodium. Maintenir l'agitation une heure et abandonner 12 heures à température ambiante. Acidifier à l'acide chlorhydrique, évaporer le milieu réactionnel. Traiter le résidu par l'ammoniaque et extraire au chloroforme. La phase organique obtenue est passée sur une colonne d'alumine. Evaporer le solvant. Le résidu est le produit du titre.
*Point de fusion : 146-147°C*

| *Composition centésimale :* | | | | |
|---|---|---|---|---|
| *Calculée* | *C 65,9* | *H 5,5* | *Cl 10,8* | *N 12,8* |
| *Trouvée* | *C 65,6* | *H 5,7* | *Cl 10,5* | *N 12,5* |

*Caractéristiques spectrales :*
*Infrarouge: 1490, 1600cm*^{*-1*}*ν C=C*
*1650cm*^{*-1*}*ν CDCl*_{*3*}
*R.M.N* ^{*1*}*H (CDCl*_{*3*}*) :*
*δ = 1,91 ppm ; quintet, 2H, CH*_{*2*} *(3)*
*δ = 3,51 ppm ; triplet, 2H, CH*_{*2*} *(4)*
δ = 3,74 *ppm ; triplet, 2H, CH*_{*2*} *(2)*
*δ = 3,94 ppm ; triplet, 1H, CH*
δ *= 5,00 ppm ; doublet, 2H, CH*_{*2*}*-CH*
**STADE B :Chlorhydrate de 10-[2-hydroxy-2-(4-chlorophényl)éthyl]- 2,3, 4,10-tétrahydropyrimidino[1,2-a]benzimidazole**
Ajouter lentement de l'isopropanol chlorhydrique à une solution isopropanique de la base obtenue au stade A. Attendre 30 minutes. Essorer, laver à l'acétone.
*Rendement : 90%*
*Point de fusion : 253-254°C (décomposition)*

| *Composition centésimale :* | | | | |
|---|---|---|---|---|
| *Calculée* | *C 59,3* | *H 5,3* | *Cl 19,5* | *N 11,5* |
| *Trouvée* | *C 59,4* | *H 5,5* | *Cl 19,4* | *N 11,3* |

### EXEMPLE 53 :

### CHLORHYDRATE DE 10-[2-HYDROXY-2-(4-METHOXYPHENYL)ETHYL]-2,3,4,10-TETRAHYDROPYRIMIDINO[1,2-a]BENZIMIDAZOLE

En procédant comme dans l'exemple 52, mais en remplaçant au stade A le 10-(4-chlorophénacyl)-2,3,4,10-tétrahydropyrimidino[1,2-a]benzimidazole par le 10-(4-méthoxyphénacyl)-2,3,4,10-tétrahydropyrimidino[1,2-a]benzimida-zole obtenu dans l'exemple 51, on obtient le produit du titre.
*Point de fusion : 210-212°C*

| *Composition centésimale :* | | | | |
|---|---|---|---|---|
| *Calculée* | *C 63,4* | *H 6,2* | *Cl 9,9* | *N 11,7* |
| *Trouvée* | *C 63,2* | *H 6,5* | *Cl 9,5* | *N 11,9* |

*Caractéristiques spectrales :*
*Infrarouge: 1500-1610cm*^{*-1*} *ν C=C*

### EXEMPLE 54 :

### SULFATE DE 1-(2-DIETHYLAMINOETHYL)-1,2,3,4-TETRAHYDROPYRIMIDINO[1,2-a] BENZIMIDAZOLE (TRIHYDRATE)

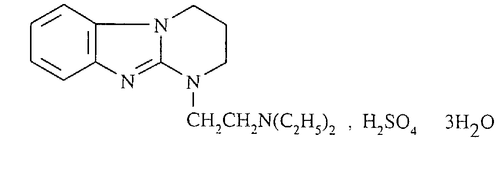

**STADE A :1-(2-diéthylaminoéthyl)-1,2,3,4-tétrahydropyrimido[1,2-a] benzimidazole**
Porter 12 heures à reflux un mélange de 1,16 g (5 mmol) de 2-(2-diéthylaminoéthylamino)benzimidazole et de 0,6 ml (5 mmol) de 1-bromo-3-chloropropane dans 10 ml de xylène. Après refroidissement, le xylène est décanté et l'huile résiduelle est traitée à l'éther de pétrole. Evaporer l'éther de pétrole rémanent et traiter à l'ammoniaque. Extraire au chloroforme. Les phases organiques réunies sont concentrées et passées sur une colonne d'alumine éluée au chloroforme. L'évaporation du solvant permet d'obtenir le produit du titre.
*Rendement : 42%*

| *Composition centésimale :* | | | |
|---|---|---|---|
| *Calculée* | *C 70,5* | *H 8,9* | *N 20,6* |
| *Trouvée* | *C 70,4* | *H 9,1* | *N 20,8* |

*Caractéristiques spectrales :*
*Infrarouge: 1625cm*^{*-1*}*ν (C=N)*
*R.M.N* ^{*1*}*H (CDCl*_{*3*}*):*
*δ* = *0,97 ppm ; triplet, 6H, (2CH*_{*3*}*)*
*δ = 2,16 ppm ; quintuplet, 2H, CH*_{*2*} *(3)*
*δ = 2,55 ppm ; quadruplet, 4H, N(CH*_{*2*}*)*_{*2*}
*δ = 2,72 ppm ; triplet, 2H, CH*_{*2*} *N*
*δ = 3,47 ppm ; triplet, 2H, CH*_{*2*} *(4)*
*δ = 3,66 ppm ; triplet, 2H, CH*_{*2*} *(2)*
*δ = 3,89 ppm ; triplet, 2H, NCH*_{*2*}*CH*_{*2*}*N(Et)*_{*2*}
**STADE B :Sulfate de 1-(2-diéthylaminoéthyl)-1,2,3,4-tétrahydropyri-midino[1,2-a] benzimidazole (trihydrate)**
Dissoudre la base obtenue au stade A dans 10 ml d'acétone et acidifier par l'acétone sulfurique. Triturer et ajouter par petites portions de l'acétone : filtrer ; sécher et recristalliser dans l'acétonitrile.
*Rendement : 98%*
*Point de fusion : 200-202°C*

| *Composition centésimale (trihydrate) :* | | | | |
|---|---|---|---|---|
| *Calculée* | *C 45,5* | *H 7,6* | *N 13,3* | *S 7,6* |
| *Trouvée* | *C 45,5* | *H 7,5* | *N 13,0* | *S 7,4* |

### EXEMPLE 55 :

### DICHLORHYDRATE DE 1-(2-PIPERIDINOETHYL)-1,2,3,4-TETRAHYDROPYRIMIDINO [1,2-a]BENZIMIDAZOLE (TRIHYDRATE)

En procédant comme dans l'exemple 54, mais en remplaçant au stade A le 2-(2-diéthylaminoéthylamino)benzimidazole par le 2-(2-pipéridinoéthylamino) benzimidazole et au stade B l'acide sulfurique par l'acide chlorhydrique, on obtient le produit du titre.
*Point de fusion : 188-190°C (décomposition)*

| *Composition centésimale :* | | | | |
|---|---|---|---|---|
| *Calculée* | *C 49,6* | *H 7,8* | *CI 17,2* | *N 13,6* |
| *Trouvée* | *C 49,6* | *H 7,9* | *CI 16,9* | *N 13,5* |

*Caractéristiques spectrales :*
*Infrarouge: 1620cm*^{*-1*}*ν C=N*
*R.M.N* ^{*1*}*H (CDCl*_{*3*}*):*
*δ* = *1,44 ppm ; massif, 6H, pipéridine*
*δ = 2,15 ppm ; quintuplet, 2H, CH*_{*2*} *(3)*
*δ* = *2,42 ppm ; massif, 4H, pipéridine*
*δ = 2,63 ppm ; triplet, 2H, CH*_{*2*}*-N*
*δ = 3,43 ppm ; triplet, 2H, CH*_{*2*} *(4)*
*δ = 3,68 ppm ; triplet, 2H, CH*_{*2*} *(2)*

### EXEMPLE 56 :

### DICHLORHYDRATE DE 1-(2-MORPHOLINOETHYL)-1,2,3,4-TETRAHYDROPYRIMIDINO [1,2-a]BENZIMIDAZOLE (TRIHYDRATE)

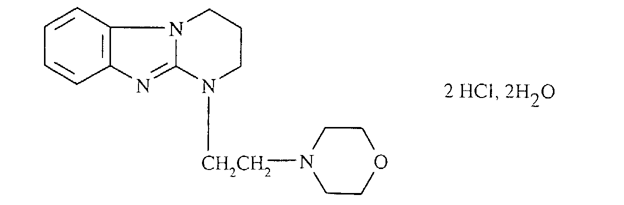

En procédant comme dans l'exemple 55, mais en remplaçant le 2-(2-pipéridinoéthylamino)benzimidazole par le 2-(2-morpholinoéthylamino) benzimidazole, on obtient le produit du titre.
*Point de fusion : 284-285°C (décomposition)*

| *Composition centésimale :* | | | | |
|---|---|---|---|---|
| *Calculée* | *C 48,6* | *H 7,1* | *Cl 17,9* | *N 14,2* |
| *Trouvée* | *C 48,4* | *H 7,0* | *Cl 18,0* | *N 14,2* |

*Caractéristiques spectrales :*
*Infrarouge: 1620cm*^{*-1*}ν *C=N*
*R.M.N* ^{*1*}*H (CDCl*_{*3*}*) :*
*δ = 2,16 ppm ; quintuplet, 2H, CH*_{*2*} *(3)*
*δ = 2,46 ppm ; multiplet, 4H, morpholine N CH*_{*2*} *(2)*
*δ = 2,63 ppm ; triplet, 2H, CH*_{*2*}*-Morpholine*
*δ = 3,43 ppm ; triplet, 2H, CH*_{*2*} *(4)*
*δ = 3,65 ppm ; massif 6H, morpholine et CH*_{*2*} *(2)*
*δ = 3,88 ppm ; triplet, 2H, CH*_{*2*}*-CH*_{*2*}*-Morpholine*

### EXEMPLE 57:

### 9-(2-HYDROXY-2-(4-METHOXYPHENYL)ETHYL]-2,3-DIHYDROIMIDAZO[1,2-a] BENZIMIDAZOLE, CHLORHYDRATE

Ajouter petit à petit 0,3 g (7,5 mmol) de borohydrure de sodium à une suspension de 2,41 g (7 mmol) de 9-(4'-méthoxyphénacyl)-2,3-dihydroimidazo[1,2-a]benzimidazole- décrit dans l'exemple 6 - dans 15 ml de méthanol pendant trente minutes à température ambiante. Poursuivre l'agitation pendant quatre heures supplémentaires et abandonner jusqu'au lendemain. Ajouter alors 10 ml d'acide chlorhydrique à 10% jusqu'à l'obtention d'un pH 2-3 et évaporer le méthanol. Traiter le résidu par une solution d'ammoniaque et extraire au chloroforme. Recristaliser le résidu. On obtient le produit du titre sous forme de base. Dissoudre dans l'éthanol chaud et ajouter de l'éther chlorhydrique. Laisser refroidir, essorer, laver à l'éther. On obtient le produit du titre.
*Point de fusion : 192-193°C*

| *Composition centésimale :* | | | | |
|---|---|---|---|---|
| *Calculée* | *C 62,5* | *H 5,8* | *Cl 10,3* | *N 12,1* |
| *Trouvée* | *C 62,6* | *H 6,0* | *Cl 10,5* | *N 12,4* |

*Caractéristiques spectrales :*
*Infrarouge :1660 cm*^{*-1*}ν *(C* = *N)*
*3100-3600 cm*^{*-1*}ν *(OH)*
*Résonance Magnétique Nucléaire : (1H) (CF*_{*3*}*COOD)*
*δ:3,5 ppm, singulet, 3H, OCH*_{*3*}
*δ:4,9 ppm, triplet, 2H, CH*_{*2*}

### EXEMPLE 58 :

### 10-(3',5'-DITERTBUTYL-4'-HYDROXYPHENACYL)-2,3,4,10-TETRAHYDROPYRIMIDINO [1,2-a]BENZIMIDAZOLE, BROMHYDRATE

*Point de fusion : 283-284°C (sublimation)*

### EXEMPLE 59 :

### 9-(3',5'-DITERTBUTYL-4'-HYDROXYPHENACYLIMIDAZO[1,2-a]BENZIMIDAZOLE, BROMHYDRATE

*Point de fusion : 267-268°C (sublimation)*

### EXEMPLE 60 :

### 10-(3',4'-DIHYDROXYPHENACYL)-2,3,4,10-TETRAHYDROPYRIMIDINO[1,2-a]BENZIMIDAZOLE, BROMHYDRATE

*Point de fusion : 262-263°C (sublimation)*

### EXEMPLE 61 :

### 9-(3',4'-DIHYDROXYPHENACYL)IMIDAZO[1,2-a]BENZIMIDAZOLE, BROMHYDRATE

*Point de fusion : 268-269°C (sublimation)*

### EXEMPLE 62 :

### 10-(4'-HYDROXYPHENACYL)-2,3,4,10-TETRAHYDROPYRIMIDINO[1,2-a]BENZIMIDAZOLE, BROMHYDRATE

*Point de fusion : 294-295°C (sublimation)*

### EXEMPLE 63 :

### 9-(4'-HYDROXYPHENACYL)IMIDAZO[1,2-a]BENZIMIDAZOLE, BROMHYDRATE

*Point de fusion : 297-298°C (sublimation)*

### EXEMPLE 64 :

### 10-(3',4'-DIMETHOXYPHENACYL)-2,3,4,10-TETRAHYDROPYRIMIDINO[1,2-a]BENZIMIDAZOLE, BROMHYDRATE

*Point de fusion : 263-264°C (sublimation)*

### EXEMPLE 65 :

### 9-(3',4'-DIMETHOXYPHENACYL)IMIDAZO[1,2-a]BENZIMIDAZOLE, BROMHYDRATE

*Point de fusion : 253-254°C (sublimation)*

### EXEMPLE 66 :

### 10-(5'-BROMO-2-THENOYLMETHYL)-2,3,4,10-TETRAHYDROPYRIMIDINO[1,2-a]BENZIMIDAZOLE, BROMHYDRATE

*Point de fusion : 260-261°C (sublimation)*

### EXEMPLE 67:

### 9-(5'-BROMO-2-THENOYLMETHYL)IMIDAZO[1,2-a]BENZIMIDAZOLE, BROMHYDRATE

*Point de fusion : 259-260°C (sublimation)*

### EXEMPLE 68 :

### 10-(2-THENOYLMETHYL)-2,3,4,10-TETRAHYDROPYRIMIDINO[1,2-a]BENZIMIDAZOLE, BROMHYDRATE

*Point de fusion : 290-291°C (sublimation)*

### EXEMPLE 69 :

### 9-(2-THENOYLMETHYL)IMIDAZO[1,2-a]BENZIMIDAZOLE, BROMHYDRATE

*Point de fusion : 235-236°C (sublimation)*

### EXEMPLE 70 :

### 10-(3',4'-DICHLOROPHENACYL)-2,3,4,10-TETRAHYDROPYRIMIDINO[1,2-a]BENZIMIDAZOLE, BROMHYDRATE

*Point de fusion : 278-279°C (sublimation)*

### EXEMPLE 71:

### 10-(4'-CHLOROPHENACYL)-2,3,4,10-TETRAHYDROPYRIMIDINO[1,2-a] BENZIMIDAZOLE, BROMHYDRATE

*Point de fusion : 269-270°C (sublimation)*

### ETUDE PHARMACOLOGIQUE DES PRODUITS DE L'INVENTION

### EXEMPLE A :

### ETUDE DE LA TOXICITE AIGUË

La toxicité aiguë a été appréciée après administration orale à des lots de 8 souris (26 ± 2 grammes) de doses croissantes de produits à étudier. Les animaux ont été observés à intervalles réguliers au cours de la première journée et quotidiennement pendant les deux semaines suivant le traitement.
Il apparaît que les composés de l'invention sont très peu toxiques.

### EXEMPLE B :

### ETUDE DE L'ACTIVITE HYPOGLYCEMIANTE

L'activité hypoglycémiante des dérivés de l'invention a été recherchée sur des rats blancs (160-190 g) à raison de cinq animaux par composé étudié, dix huit heures avant le début de l'expérimentation, les animaux sont soumis à la diète hydrique.

Les composés à étudier sont administrés par voie intrépéritonéale à la dose unique de 50 mg.kg⁻¹. La concentration sanguine en glucose est déterminée par des prélèvements réalisés avant l'injection du produit puis 2 heures, 4 heures et 6 heures après l'administration. Le dosage a été réalisé en utilisant la méthode par fermentation à l'aide des kits Bio-La-Tests (Lachema - Tchecoslovaquie). Pour cette manipulation on a utilisé un spectro photomètre KFK-2 (λ = 490 nm).

Les concentrations sanguines en glucose ont été comparées à une solution étalon de glucose (10 mmol/l). Il apparaît que les produits de l'invention ont une activité hypoglycémiante comparable à celle du gliclazide. Leur durée d'action est supérieure à douze heures.

### EXEMPLE C:

### ETUDE DE L'ACTIVITE ANTIAGREGANTE PLAQUETTAIRE

L'étude de l'activité antiagrégante plaquettaire des composés de l'invention a été recherchée selon la méthode G.V.R. BORN (Aggregation of blood platelets by adenosine dyphosphate and its reversal - Nature 1962-v. 194 p 927-929). Pour ces expérimentations on a utilisé un plasma de lapin riche en plaquettes contenant au moins 2,5 × 108 plaquettes par ml. L'ADP (adénosine diphosphate) sert d'inducteur. Avant d'utiliser l'ADP, les composés de l'invention sont mis en présence du plasma riche en plaquettes. Chaque composé a été étudié sur cinq échantillons différents de plasma riche en plaquettes. L'agrégation plaquettaire a été évaluée en pourcentage de diminution de la densité optique du plasma enrichi en plaquettes par enregistrement photoélectrocolorimétrique. La dose inhibitrice 50 des composés de l'invention (dose à laquelle l'agrégation plaquettaire est diminuée de moitié) est inférieure à 10-4M pour les produits de l'invention.

### EXEMPLE D :

### RECHERCHE DE L'ACTIVITE ANTIHYPERTENSIVE

Les animaux sont acclimatés durant une période de six jours avant le début de l'étude. Au début de l'expérience, les rats sont anesthésiés avec 1000 mg.kg⁻¹ d'uréthane administré par voie intrapéritonéale. Dans la veine jugulaire est introduit un cathéter.
Un cathéter relié à un enregistreur de précision est placé dans l'artère carotide. On laisse s'écouler un intervalle de 10 minutes pour permettre la stabilisation de la tension artérielle avant la prise de la première mesure.

Dans un premier temps, on administre le solvant par voie intraveineuse à tous les animaux, la pression artérielle est enregistrée pendant 30 minutes après l'administration et des lectures de pression artérielle sont effectuées 10, 20 et 30 minutes après l'administration. Les composés de l'invention sont administrés en solution saline également par voie intraveineuse. L'enregistrement de la tension artérielle réalisé pendant 30 minutes et des mesures de pression sont effectuées 10, 20 et 30 minutes après l'administration. Les produits de l'invention n'entraînent pas de diminution significative de la pression artérielle.

### EXEMPLE E :

### COMPOSITION PHARMACEUTIQUE

Comprimés destinés au traitement du diabète et de ses complications dosés à 20 mg de dichlorhydrate de 9-(2-diéthylaminoéthyl)2,3-dihydroimidazo [1,2-a]benzimidazole.

| Formule de préparation pour 1000 comprimés : | |
|---|---|
| dichlorhydrate de 9-(2-diéthylaminoéthyl)-2,3-dihydroimidazo[1.2-a] benzimidazole | 20 g |
| Amidon de blé | 15 g |
| Lactose | 65 g |
| Stéarate de magnésium | 2 g |
| Silice | 1 g |
| Hydroxypropyl cellulose 2 g | 2 g |

## Revendications

1. Dérivés de formule générale (I) : 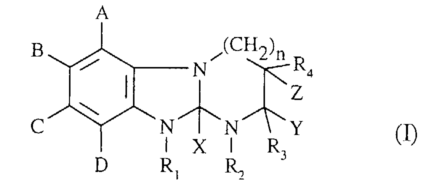 pour lesquels :
ou bien :
**1.**
n = 0
A, B, C, D identiques ou différents représentent un atome d'hydrogène, d'halogène, un groupement alkyle inférieur, alkoxy inférieur, hydroxyle, trifluorométhyle, ou hydroxyalkyle inférieur,
Y et Z représentent chacun un atome d'hydrogène ou forment ensemble une liaison, et alors soit :
**1.A.**
X et R₂ forment ensemble une liaison, et dans ce cas alors :
R₁ représente un groupement G₁,
G₁ représente le groupement 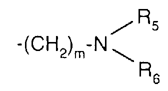 avec m nombre entier compris inclusivement entre 1 et 6 et R₅ et R₆ identiques ou différents représentent :
a/ indépendamment l'un de l'autre un atome d'hydrogène, un groupement alkyle inférieur, un groupement arylalkyle inférieur, ou un groupement arylalkyle inférieur substitué ;
b/ ou R₅ et R₆ forment simultanément avec l'atome d'azote qui les porte un système morpholinique, pipéridine, pyrrolidine ou pipérazine éventuellement substitué sur l'atome d'azote en 4 par un groupement alkyle inférieur, aryle, aryle substitué, arylalkyl inférieur, arylalkyl inférieur substitué,
ou bien
R₁ représente un groupement G₂, G₂ signifiant un groupement (CH₂)ₘCOR₇ ou G₃, G₃ signifiant un groupement (CH₂)ₘCHOHR₇, m ayant la même signification que signifiant un groupement (CH₂)ₘCHOHR₇, m ayant la même signification que précédemment et R₇ signifiant un groupement aryle ou aryle substitué,
R₃ représente un atome d'hydrogène, un groupement alkyle inférieur, un noyau phényl substitué par un groupement hydroxy ou hydroxyalkyle ou par deux à cinq groupements choisis parmi alkyle inférieur, alkoxy inférieur, halogène, trifluorométhyle, hydroxy, hydroxyalkyle, ou bien R₃ représente un noyau naphtyle éventuellement subsitué, ou un groupement hétéroaryale éventuellement substitué,
R₄ représente un atome d'hydrogène, un groupement G₁ où G₁ a la même définition que précédemment, un groupement G₄, c'est-à-dire un groupement de formule :
-COO-G₁
où G₁ a la même définition que précédemment, ou un groupement G₅ c'est-à-dire - COR₉ où R₉ représente un groupement aryle, un groupement aryle substitué, un groupement hétéroaryle, un groupement hétéroaryle substitué,
à l'exception des dérivés pour lesquels simultanément R₄ représente un atome d'hydrogène et R₁ représente un groupement: 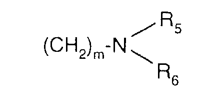 avec m = 2,
et R₅ et R₆ représentent simultanément un groupement éthyle tandis que R₃ représente un groupement alkyle inférieur ou naphtyle,
et des dérivés pour lesquels R₅ et R₆ forment simultanément avec l'atome d'azote qui les porte un système pipéridine, pyrrolidine ou pipérazine éventuellement substitué sur l'azote en 4 par un groupement alkyle inférieur, aryle, arylalkyle inférieur, aryle substitué, arylalkyle inférieur substitué tandis que R₃ représente un atome d'hydrogène.
ou
**1.B**
X et R₁ forment ensemble une liaison,
R₂ représente un groupement G₁, G₂ ou G₃ avec G₁, G₂ ou G₃ ayant la même définition que précédemment, et alors R₃ représente un atome d'hydrogène, un groupement alkyle inférieur, un groupement phényle substitué, un groupement naphtyle, un groupement naphtyle substitué, un groupement hétéroaryle, un groupement hétéroaryle substitué ou un groupement G₁ où G₁ à la même définition que précédemment, tandis que R₄ représente un atome d'hydrogène ou un groupement G₁, G₄ ou G₅ où G₄ et G₅ ont la même définition que précédemment,
étant entendu que lorsque R₂ représente un groupement G₁, G₁ ayant la même définition que précédemment, alors R₃ est différent d'un atome d'hydrogène,
ou
**2.**
n =1,
A, B, C, D identiques ou différents représentent un atome d'hydrogène, d'halogène, un groupement alkyle inférieur ou trifluorométhyle,
avec X et R₁ forment une liaison ou bien X et R₂ forment une liaison l'une au moins de ces deux liaisons devant être présente dans la molécule,
Y et Z représentent chacun un atome d'hydrogène, ou forment ensemble une liaison,
R₁ ou R₂ - selon que X et R₂ ou X et R₁ forment une liaison - représentent alors un groupement méthyle ou G₁ ou G₂ ou G₃, G₁, G₂ et G₃ ayant la même définition que précédemment,
R₃ représente un atome d'hydrogène, un groupement alkyle inférieur, un groupement aryle, un groupement aryle substitué, un groupement hétéroaryle, un groupement hétéroaryle substitué,
et R₄ représente un atome d'hydrogène ou un groupement G₁ ou G₄ ou G₅, G₁, G₄ et G₅ gardant la même définition que précédemment,
leurs isomères, énantiomères, diastéréoisomères, ainsi que leurs sels d'addition à un acide pharmaceutiquement acceptable,
étant entendu que par groupement alkyle inférieur ou alkoxy inférieur, on entend des groupements linéaire ou ramifié comprenant de 1 à 6 atomes de carbone,
que par groupement aryle, on entend un groupement phényle ou naphtyle,
que par groupement hétéroaryle, on entend un groupement furyle, thiényle, pyridyle, pyrrolyle, imidazolyle, benzimidazolyle, benzofuryle, benzothiényle, quinolyle, isoquinolyle, ou indolyle,
que le terme substitué affectant les expressions arylalkyle, aryle, phényle, naphtyle, hétéroaryle signifie, sauf précision contraire, que les groupements concernés sont substitués sur leur partie cyclique par un à trois radicaux choisis parmi halogène, trifluorométhyle, hydroxy, alkoxy inférieur, hydroxy alkyle inférieur, alkyle inférieur,
et que s'il existe dans une même molécule plusieurs groupements G₁ ou groupements R₅ et R₆, ceux-ci peuvent être identiques ou différents.

2. Composés selon la revendication 1 pour laquelle n vaut 0, X et R₂ forment une liaison, Z et Y représentent chacun un atome d'hydrogène, leurs isomères, épimères, diastéréroisomères ainsi que le cas échéant leurs sels d'addition à un acide pharmaceutiquement acceptable.

3. Composés selon la revendication 1 pour lesquels n vaut 0, X et R₂ d'une part et Y et Z d'autre part forment une liaison, leurs isomères, épimères, diastéréroisomères ainsi que leurs sels d'addition à un acide pharmaceutiquement acceptable.

4. Composés selon la revendication 1 pour lesquels R₄ représente un atome d'hydrogène, leurs isomères, épimères, diastéréroisomères ainsi que leurs sels d'addition à un acide pharmaceutiquement acceptable.

5. Composés selon la revendication 1 pour lesquels n vaut 1, Y et Z représentent chacun un atome d'hydrogène, et X et R₂ forment ensemble une liaison, leurs isomères, épimères, diastéréroisomères ainsi que leurs sels d'addition à un acide pharmaceutiquement acceptable.

6. Composés selon la revendication 1 pour lesquels n vaut 1, Y et Z représentent chacun un atome d'hydrogène, et R₁ et X forment ensemble une liaison, leurs isomères, épimères, diastéréroisomères ainsi que leurs sels d'addition à un acide pharmaceutiquement acceptable.

7. Composés selon la revendication 1 pour lesquels n vaut 0, Y et Z forment ensemble une liaison, et X et R₁ forment ensemble une liaison, leurs isomères, épimères, diastéréroisomères ainsi que leurs sels d'addition à un acide pharmaceutiquement acceptable.

8. Composés selon la revendication 1 qui est le 9-(2-diéthylaminoéthyl)-2,3-dihydroimidazo[1,2-a]benzimidazole, ses isomères ainsi que leurs sels d'addition à un acide pharmaceutiquement acceptable.

9. Composés selon la revendication 1 qui sont :
9-MORPHOLINOETHYL-2,3-DIHYDROIMIDAZO[1,2-a]BENZIMIDAZOLE
9-PHENACYL-2,3-DIHYDROIMIDAZO[1,2-a]BENZIMIDAZOLE
9-(p-CHLOROPHENACYL)-2,3-DIHYDROIMIDAZO[1,2-a]BENZIMIDAZOLE
9-(p-METHOXYPHENACYL)-2,3-DIHYDROIMIDAZO[1,2-a]BENZIMIDAZOLE
9-(1-NAPHTOYLMETHYL)-2,3-DIHYDROIMIDAZO[1,2-a]BENZIMIDAZOLE
9-(2-HYDROXY-2-PHENYLETHYL)-2,3-DIHYDROIMIDAZO[1,2-a]BENZIMIDAZOLE
9-[2-HYDROXY-2-(4'-CHLOROPHENYL)ETHYL]-2,3-DIHYDROIMIDAZO[1,2-a] BENZIMIDAZOLE
9-[2-(1-NAPHTYL)-2-HYDROXYETHYL]-2,3-DIHYDROIMIDAZO[1,2-a] BENZIMIDAZOLE,
2-TERT.BUTYL-9-(2-MORPHOLINOETHYL)IMIDAZO[1,2-a]BENZIMIDAZOLE
2-TERT.BUTYL-9-(3-DIMETHYLAMINOPROPYL)IMIDAZO[1,2-a]BENZIMIDAZOLE
9-(2-MORPHOLINOETHYL)-2-(4-HYDROXYPHENYL)IMIDAZO[1,2-a]BENZIMIDAZOLE
9-(2-MORPHOLINOETHYL)-2-(3-HYDROXYPHENYL)IMIDAZO[1,2-a]BENZIMIDAZOLE
9-DIETHYLAMINOETHYL-2-(3,4-DIMETHOXYPHENYL)IMIDAZO[1,2-a] BENZIMIDAZOLE
9-DIETHYLAMINOETHYL-2-(3,4-DIHYDROXYPHENYL)IMIDAZO[1,2-a] BENZIMIDAZOLE
9-(2-PIPERIDINOETHYL)-2-(3,4-DIHYDROXYPHENYL)IMIDAZO[1,2-a] BENZIMIDAZOLE
9-(2-DIETHYLAMINOETHYL)-2-(1-METHYL-2-BENZIMIDAZOLYL)IMIDAZO[1,2-a] BENZIMIDAZOLE
9-(2-DIETHYLAMINOETHYL)-2-(2-THIENYL)IMIDAZO[1,2-a]BENZIMIDAZOLE
9-(2-PIPERIDINOETHYL)-2-(2-THIENYL)IMIDAZO[1,2-a]BENZIMIDAZOLE
9-(2-PIPERIDINOETHYL)-2-(5-BROMO-2-THIENYL)IMIDAZO[1,2-a]BENZIMIDAZOLE
2-(5-BROMO-2-THIENYL)-9-(2-MORPHOLINOETHYL)IMIDAZO[1,2-a]BENZIMIDAZOLE
2-(2-FURYL)-9-(2-N, N-DIETHYLAMINOETHYL)IMIDAZO[1,2-a]BENZIMIDAZOLE
2-(2-FURYL)-9-(2-MORPHOLINOETHYL)IMIDAZO[1,2-a]BENZIMIDAZOLE
2-METHYL-9-(2-MORPHOLINOETHYL)-3-(4-CHLOROBENZOYL)IMIDAZO[1,2-a] BENZIMIDAZOLE
2-METHYL-9-(2-PIPERIDINOETHYL)-3-(2-FUROYL)IMIDAZO[1,2-a]BENZIMIDAZOLE
2-METHYL-9-(2-PIPERIDINO ETHYL)-3-(2-THENOYL)IMIDAZO[1,2-a]BENZIMIDAZOLE
2-METHYL-9-(2-MORPHOLINOETHYL)-3-(5-BROMO-2-THENOYL)IMIDAZO[1,2-a] BENZIMIDAZOLE
2-TERT.BUTYL-1-(2-MORPHOLINOETHYL)IMIDAZQ[1,2-a]BENZIMIDAZOLE
2-TERT.BUTYL-1-(2-PIPERIDINOETHYL)IMIDAZO[1,2-a]BENZIMIDAZOLE
2-TERT.BUTYL-1-(2-N,N DIETHYLAMINOETHYL)IMIDAZO[1,2-a]BENZIMIDAZOLE
10-(2-DIETHYLAMINOETHYL)-2,3,4,10-TETRAHYDROPYRIMIDINO[1,2-a] BENZIMIDAZOLE
10-PHENACYL-2,3,4,10-TETRAHYDROPYRIMIDINO[1,2-a]BENZIMIDAZOLE
10-(4'-CHLOROPHENACYL)-2,3,4,10-TETRAHYDROPYRIMIDINO[1,2-a] BENZIMIDAZOLE
10-(4'-METHOXYPHENACYL)-2,3,4,10-TETRAHYDROPYRIMIDINO[1,2-a] BENZIMIDAZOLE
10-[2-HYDROXY-2-(4-CHLOROPHENYL)ETHYL]-2,3,4,10-ETRAHYDROPYRIMIDINO [1,2-a]BENZIMIDAZOLE
10-[2-HYDROXY-2-(4-METHOXYPHENYL)ETHYL]-2,3,4,10-TETRAHYDROPYRIMIDINO[1,2-a]BENZIMIDAZOLE
1-(2-DIETHYLAMINOETHYL)-1,2,3,4-TETRAHYDROPYRIMIDINO[1,2-a] BENZIMIDAZOLE
1-(2-PIPERIDINOETHYL)-1,2,3,4-TETRAHYDROPYRIMIDINO[1,2-a]BENZIMIDAZOLE
1 -(2-MORPHOLINOETHYL)-1,2,3,4-TETRAHYDROPYRIMIDINO[1,2-a] BENZIMIDAZOLE
9-[2-HYDROXY-2-(4-METHOXYPHENYL)ETHYL]2,3-DIHYDROIMIDAZO[1,2-a] BENZIMIDAZOLE
leurs isomères, épimères, diastéréroisomères ainsi que leurs sels d'addition à un acide pharmaceutiquement acceptable.

10. Procédé de préparation des dérivés de formule générale (I), selon la revendication 1, caractérisé :
lorsque dans le dérivé de formule (I) que l'on souhaite obtenir X et R₂ forment ensemble une liaison, en ce que l'on utilise comme matière première un dérivé de formule (II) : 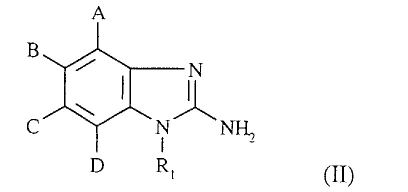 où A, B, C, D et R₁ ont la même définition que précédemment,
que l'on traite :
lorsque dans le dérivé de formule (I) que l'on souhaite obtenir Y et Z représentent chacun un atome d'hydrogène, par un dérivé de formule (III/A) :
Hal_{A}-(CH₂)ₙ-CHR₄-CHR₃-P (III/A)
où Hal_{A} représente un atome d'halogène, n, R₃ et R₄ ont la même définition que précédemment et P représentant un groupe partant choisi parmi halogène ou hydroxyle,
pour conduire à un dérivé de formule (IV/A) : 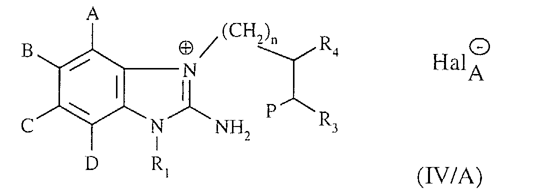 où A, B, C, D, R₁ ont la même définition que dans la formule (I) et n, R₃, R₄ et P ont la même définition que précédemment,
qui, par chauffage, précédé, lorsque P représente un groupement hydroxyle par un traitement par un agent d'halogénation,
conduit à un dérivé de formule (I/B): 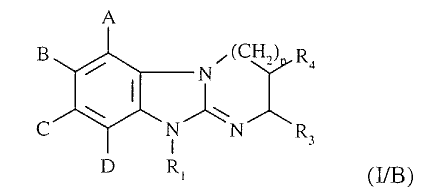 cas particulier des dérivés de formule (I) pour lesquels A, B, C, D, R₁, R₃, R₄ et n ont la même définition que précédemment,
X et R₂ forment ensemble une liaison et Y et Z représentent chacun un atome d'hydrogène,
dérivé de formule (I/B) que l'on purifie si nécessaire et que l'on transforme si on le désire par un acide en un sel pharmaceutiquement acceptable.

11. Procédé de préparation des dérivés de formule générale (I) selon la revendication 1, caractérisé :
lorsque dans le dérivé de formule (I) que l'on souhaite obtenir X et R₂ forment ensemble une liaison, en ce que l'on utilise comme matière première un dérivé de formule (II) : 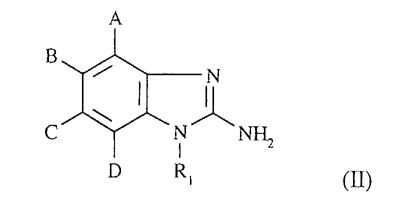 où A, B, C, D et R₁ ont la même définition que précédemment,
que l'on traite :
lorsque dans le dérivé de formule (I) que l'on souhaite obtenir Y et Z forment ensemble une liaison,
par un dérivé de formule (III) : 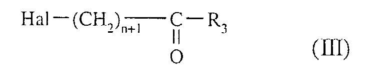 où n a la même définition que précédemment et R₃ la même définition que dans la formule (I) en fonction des valeurs de R₁ et Hal représente un atome d'halogène,
pour conduire à un dérivé de formule (IV) : 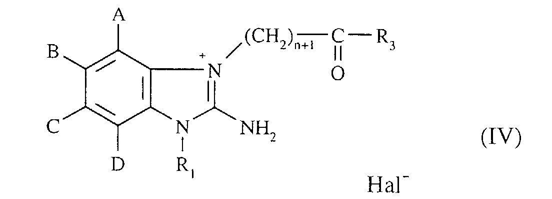 où Hal, A, B, C, D, R₁, n, R₃ ont la même définition que précédemment,
qui, par chauffage, conduit à un dérivé de formule (I/A) : 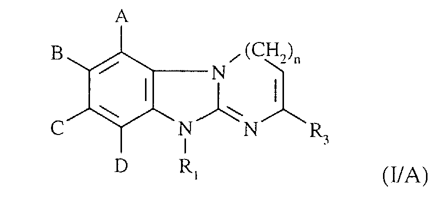 cas particulier des dérivés de formule (I) pour lesquels A, B, C, D, R₁, R₃ et n ont la même définition que précédemment, et X et R₂ d'une part, et Y et Z d'autre part forment ensemble une liaison et R₄ représente un atome d'hydrogène,
dérivé de formule (I/A) qui, lorsque dans le dérivé de formule (I) que l'on souhaite obtenir, R₄ est différent de l'hydrogène est traité :
- lorsque dans le dérivé de formule (I) que l'on souhaite obtenir, R₄ représente un groupement G₄ et Y et Z représentent une liaison,
par un dérivé de formule (V/A) :
Hal'₃-C-CO-Hal" (V/A)
où Hal' et Hal" identiques ou différents représentent chacun un atome d'halogène, pour conduire à un dérivé de formule (VI) : 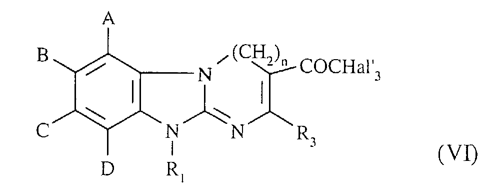 où A, B, C, D, R₁, n, R₃ et Hal'₃ ont la même définition que précédemment, dérivé de formule (VI) que l'on traite par un composé de formule (VII) : 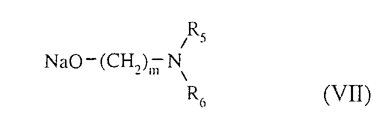 où m, R₅ et R₆ ont la même signification que dans la formule du groupement G₄ tel que défini dans la formule (I),
pour conduire à un dérivé de formule (I/C): 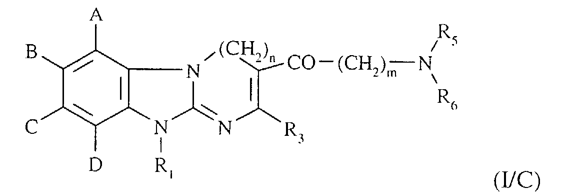 où A, B, C, D, R₁, R₃, R₅ et R₆ et n ont la même définition que dans la formule (I),
Y et Z d'une part et X et R₂ d'autre part forment ensemble une double liaison et R₄ représente un groupement G₄,
dérivé de formule (I/A) ou (I/C) que l'on purifie si nécessaire et que l'on transforme si on le désire, par un acide en un sel pharmaceutiquement acceptable.

12. Procédé de préparation des dérivés de formule générale (I) selon la revendication 1, caractérisé :
lorsque dans le dérivé de formule (I) que l'on souhaite obtenir X et R₂ forment ensemble une liaison, en ce que l'on utilise comme matière première un dérivé de formule (II) : 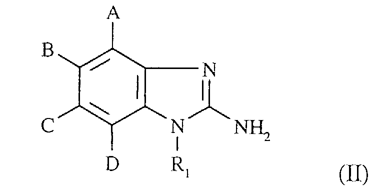 où A, B, C, D et R₁ ont la même définition que précédemment,
que l'on traite :
lorsque dans le dérivé de formule (I) que l'on souhaite obtenir R₄ représente un groupement G₄, Y et Z représentent une liaison par un dérivé de formule (VIII) :
Hal'''-(CH₂)ₘ-Hal₄ (VIII)
ou Hal"' et Hal₄ identiques ou différents représentent un atome d'halogène et m a la même définition que précédemment, pour conduire à un dérivé de formule (IX) : 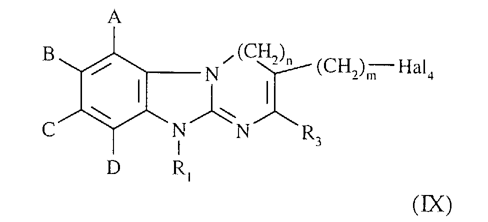 ou A, B, C, D, R₁, n, p, R₃ et Hal₄ ont la même définition que précédement,
que l'on traite alors par un dérivé de formule (X) : 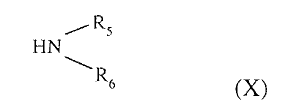 où R₅ et R₆ ont la même définition que précédemment,
pour conduire à un dérivé de formule (I/D) : 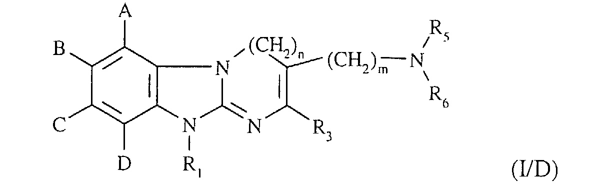 cas particulier des dérivés de formule (I) où A, B, C, D, R₁, n ont la même définition que dans la formule (I) et X et R₂ d'une part, et Y et Z d'autre part, forment ensemble une liaison et R₄ forme un groupement G₁ tel que définis dans la formule (II),
dérivé de formule (I/D) que l'on purifie si nécessaire et que l'on transforme, si on le désire, par un acide en un sel pharmaceutiquement acceptable.

13. Procédé de préparation des dérivés de formule générale (I) selon la revendication 1, caractérisé :
lorsque dans le dérivé de formule (I) que l'on souhaite obtenir X et R₂ forment ensemble une liaison, en ce que l'on utilise comme matière première un dérivé de formule (II) : 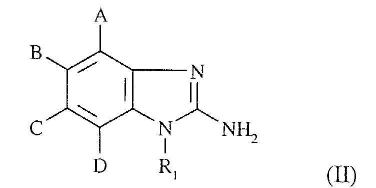 où A, B, C, D et R₁ ont la même définition que précédemment,
que l'on traite :
lorsque dans le dérivé de formule (I) que l'on souhaite obtenir R₄ représente un groupement G₅ ou G₇ et Y et Z représentent une liaison, par un dérivé de formule (XI) :
Cl-CO-R₉ ou Cl-GO-R₁₀ (XI)
selon le dérivé que l'on souhaite obtenir où R₉ et R₁₀ ont la même définition que précédemment,
pour conduire à un dérivé de formule (I/E): 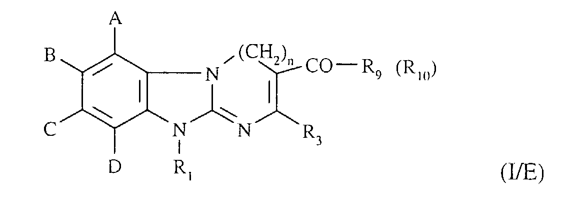 cas particulier des dérivés de formule (I) pour lesquels A, B, C, D, n, R₃ ont la même définition que précédemment, X et R₂ d'une part, et Y et Z d'autre part, forment ensemble une liaison et R₄ représente un groupement G₅ ou G₇ tels que défini précédemment,
dérivé de formule (I/E) que l'on purifie si nécessaire et que l'on transforme, si on le désire, par un acide en un sel pharmaceutiquement acceptable.

14. Procédé de préparation des dérivés de formule (I), selon la revendication 1, caractérisé, lorsque dans le dérivé de formule (I) que l'on souhaite obtenir X et R₁ forment ensemble une liaison,
en ce que l'on utilise comme matière première un dérivé de formule (XII) : 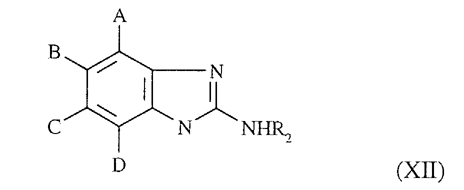 où A, B, C, D et R₂ ont la même définition que précédemment,
* que l'on traite lorsque dans le dérivé de formule (I) que l'on souhaite obtenir Y et Z représentent chacun un atome d'hydrogène,
par un dérivé de formule (III/A) tel que défini précédemment pour conduire après chauffage à un dérivé de formule (I/G): 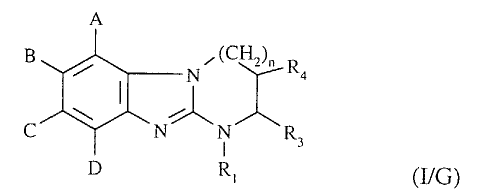 cas particulier des dérivés de formule (I) où A, B, C, D, n, R₁, R₃, R₄ ont la même définition que précédemment,
X et R₁ formant ensemble une liaison et Y et Z représentent chacun un atome d'hydrogène,
* lorsque dans le dérivé de formule (I) que l'on souhaite obtenir Y et Z forment une liaison,
par un dérivé de formule (III) telle que défini précédemment,
pour conduire après chauffage à un dérivé de formule (I/F): 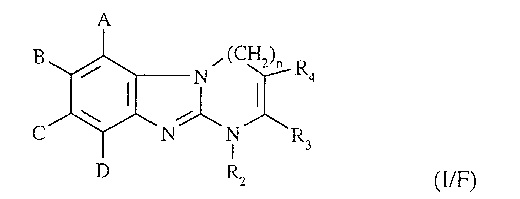 cas particulier des dérivés de formule (I) pour lesquels A, B, C, D, n, R₂, R₃ ont la même définition que précédemment,
X et R₁ d'une part et Y et Z d'autre part forment ensemble une liaison et R₄ représente un atome d'hydrogène,
dérivé de formule (I/F) que l'on peut en fonction de la valeur de R₄ dans le dérivé de formule (I) que l'on souhaite obtenir traiter :
- successivement comme indiqué plus haut par un dérivé de formule (V/A) tel que défini précédemment, puis par un dérivé de formule (VII) tel que défini précédemment,
- successivement comme indiqué plus haut par un dérivé de formule (VIII) tel que défini précédemment puis par un dérivé de formule (X) tel que définit précédemment,
- ou bien encore par un dérivé de formule (XI) tel que défini précédemment,
pour obtenir un dérivé de formule (I/H) : 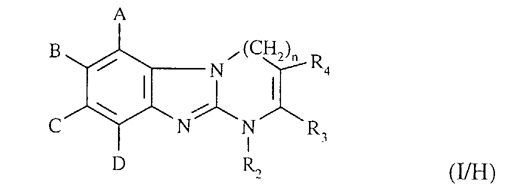 cas particulier des dérivés de formule (I) pour lesquels A, B, C, D, n, R₂, R₃, R₄ ont la même définition que dans la formule (I),
X et R₁ d'une part et Y et Z d'autre part formant chacun une liaison,
dérivés de formule (I), (I/F), (I/G), (I/H) que l'on purifie, si nécessaire, par une technique choisie parmi chromatographie et/ou cristallisation et que l'on transforme, si on le désire, par un acide en un sel pharmaceutiquement acceptable.

15. Procédé de préparation de composé de formule (I/B), selon la revendication 14, caractérisé en ce que l'on soumet à réduction par un hydrure mixte de métal alcalin un dérivé de formule (IV/B) : 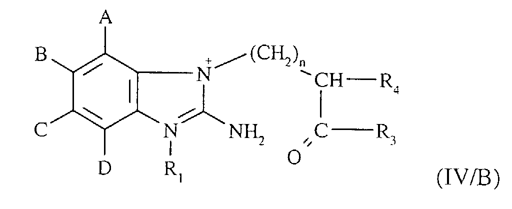 obtenu par condensation d'un dérivé de formule (VII) sur un dérivé de formule (VIII) 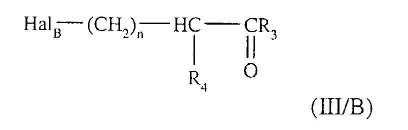 où HalB représente un atome d'halogène et n, R₃, R₄ ont la même définition que précédemment,
en dérivé de formule (IV/A) tel que défini précédemment pour lequel le groupement P représente un groupement hydroxyle, lequel traité par un agent d'halogénation puis chauffé conduit à un dérivé de formule (I/B) tel que défini précédemment que l'on purifie, si nécessaire, et que l'on transforme, si on le désire, par un acide en un sel pharmaceutiquement acceptable.

16. Procédé de préparation de dérivés de formule (I/B), selon la revendication 14, caractérisé en ce que l'on on utilise comme matière première un dérivé de formule (XIII) : 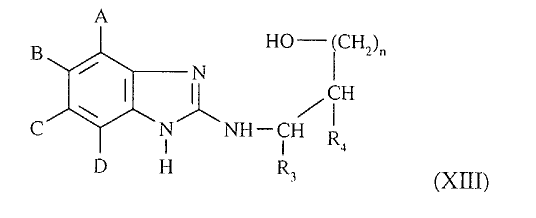 où A, B, C, D et n ont la même définition que précédemment,
que l'on traite :
ou bien par un agent d'halogénation puis par chauffage,
pour conduire à un dérivé de formule (XIV): 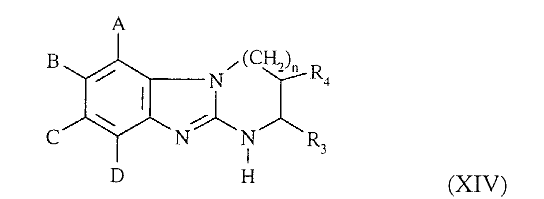 dans laquelle A, B, C, D, R₃, R₄ et n ont la même définition que précédemment,
que l'on traite par un dérivé de formule (XV): 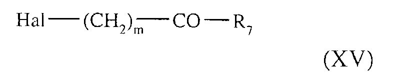 où m et R₇ ont la même définition que dans la formule (I) et Hal représente un atome d'halogène,
pour conduire à un dérivé de formule (I/J) : 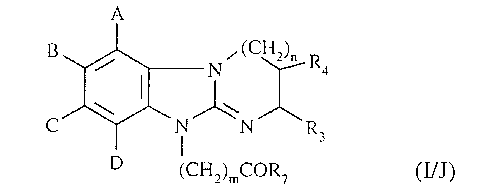 dans lequel A, B, C, D, m, n, R₃, R₄ et R₇ ont la même définition que précédemment, Y et Z représentant un atome d'hydrogène, et X et R₂ formant une liaison et R₁ représentant un groupement G₂,
dérivé de formule (I/J) que l'on peut soumettre à l'action d'un hydrure mixte de métal alcalin pour conduire à un dérivé de formule (I/K): 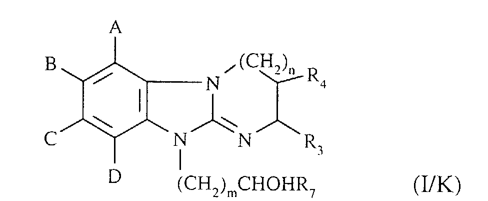 où A, B, C, D, m, n, R₃, R₄ et R₇ ont la même définition que précédemment, Y et Z représentent chacun un atome d'hydrogène et X et R₂ forment ensemble une liaison, et R₁ représente un groupement G₃, ou bien par le dérivé de formule (XV) tel que défini précédemment pour permettre l'obtention d'un dérivé de formule (XVI) : 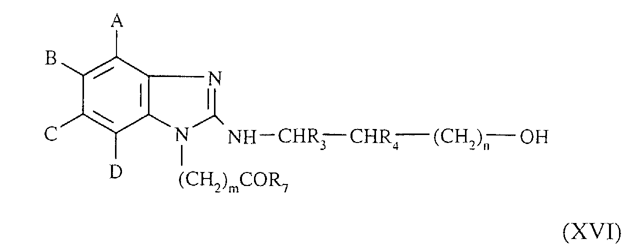 où A, B, C, D, R₃, R₄, n, m et R₇ ont la même définition que précédemment,
qui traite par un agent d'halogénation et par chauffage conduit à un dérivé de formule (I/J) tel que défini précédemment,
dérivés de formule (I/J) ou (I/K) que l'on purifie si nécessaire et que l'on transforme, si on le désire, par un acide en un sel pharmaceutiquement acceptable.

17. Procédé de préparation des dérivés de formules (I/J) ou (I/K) tels que définis selon la revendication 16 qui consiste à utiliser comme matière première un dérivé de formule (XIV) que l'on traite comme décrit dans la revendication 13.

18. Procédé de préparation des dérivés de formule (I/K) telle que définie selon la revendication 16 qui consiste à traiter le dérivé de formule (XIV) telle que défini dans la revendication 16 par un dérivé de formule (XVII) :
Hal - (CH₂)ₘ - CHOH - R₇ (XVII)
où Hal, m et R₇ ont la même définition que dans la formule (I), et si nécessaire à purifier le dérivé obtenu que l'on transforme, si on le désire par un acide en un sel pharmaceutiquement acceptable.

19. Procédé de préparation des dérivés de formule (I) selon la revendication 16 caractérisé en ce que l'on utilise comme matière première les dérivés de formule (XVIII): 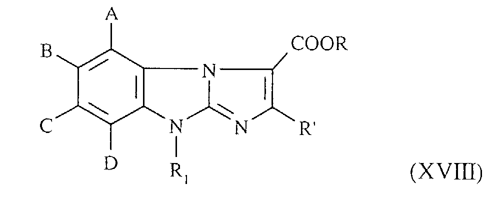 où A, B, C, D, R₁ ont la même définition que précédemment, R un groupement alkyle inférieur ou un atome d'hydrogène et R' un groupement alkyle inférieur éventuellement substitué par un atome d'halogène ou un groupement hydroxyle.

20. Procédé de synthèse des dérivés de formule (I) pour lesquels R₄ représente un groupement CH₂NR₅R₆ où R₅ et R₆ sont tels que définis dans la revendication 1 caractérisé en ce que l'on traite un dérivé de formule (I/A) telle que défini dans la revendication 14 par une réaction de Mannich utilisant le formol et une amine HNR₅R₆ que l'on purifie, si nécessaire, et que l'on transforme par un acide en un sel pharmaceutiquement accpetable.

21. Procédé de synthèse de dérivés de formule (I) pour lesquels R₂ représente un groupement G₁ tels que défini dans la revendication 1 caractérisé en ce que l'on utilise comme matière première un composé de formule (XX): 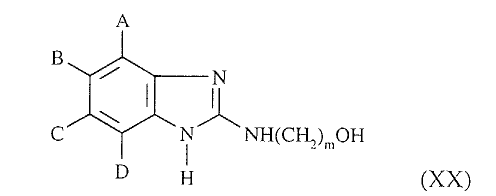 où A, B, C, D et m ont la même définition que précédemment,
que l'on traite par un dérivé de formule (XXI):
Hal-(CH₂)ₙ₊₁ - COR₃ (XXI)
où n et R₃ ont la même définition que précédemment et Hal représente un atome d'halogène pour obtenir un dérivé de formule (XXII) : 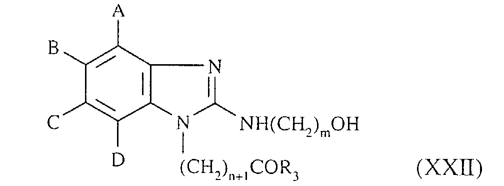 dans laquelle A, B, C, D, R₃ et m ont la même définition que dans la formule (I) qui par chauffage conduit à la formation d'un dérivé de formule (XXIII): 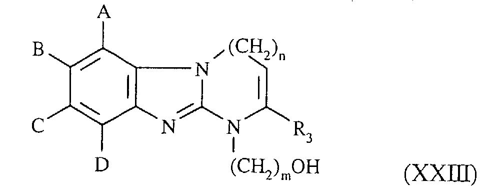 où A, B, C, D, m, n et R₃ ont la même définition que dans la formule (I),
que l'on traite :
a ) par un agent d'halogénation,
b ) par une amine de formule HNR₅R₆,
pour permettre l'obtention d'une dérivé de formule (I/M) : 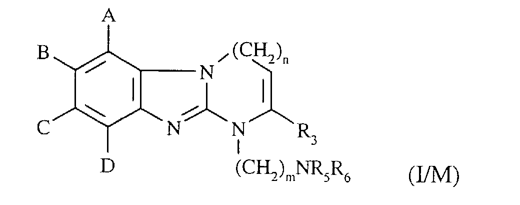 cas particulier des dérivés de formule (I) pour lesquels A, B, C, D, m, n, R₃, R₅ et R₆ ont la même définition que dans la formule (I),
pour lesquels R₁ et X, et Y et Z représentent chacun une liaison et R₂ représente un groupement G₁, que l'on purifie, si nécessaire et que l'on transforme, si on le désire, par un acide en un sel pharmaceutiquement acceptable.

22. Composition pharmaceutique contenant comme principe actif au moins un composé selon l'une des revendication 1 à 9 en combinaison avec un ou plusieurs excipients ou véhicules pharmaceutiquement acceptables.

23. Composition pharmaceutique selon la revendication 22 utilisable dans le traitement du diabète et/ou de ses complications cardiovasculaires.

## Patentansprüche

1. Derivate der allgemeinen Formel (I): 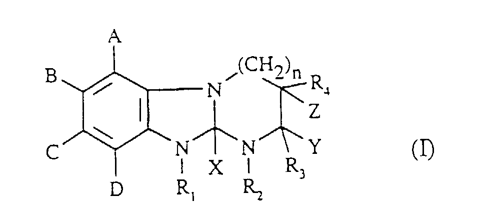 worin:
entweder:
**1.**
n = 0,
A, B, C und D, die gleichartig oder verschieden sind, ein Wasserstoffatom, ein Halogenatom, eine Niedrigalkylgruppe, Niedrigalkoxygruppe, Hydroxylgruppe, Trifluormethylgruppe oder Niedrighydroxyalkylgruppe darstellen,
Y und Z jeweils ein Wasserstoffatom darstellen oder gemeinsam eine Bindung bilden, und
dann entweder:
**1.A.**
X und R₂ gemeinsam eine Bindung bilden und in diesem Fall:
R₁ eine Gruppe G₁ darstellt,
G₁ die Gruppe 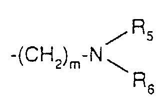 bedeutet, worin m eine ganze Zahl zwischen 1 und 6 einschließlich darstellt und R₅ und R₆, die gleichartig oder verschieden sind:
a/ unabhängig voneinander ein Wasserstoffatom, eine Niedrigalkylgruppe, eine Arylniedrigalkylgruppe oder eine substituierte Arylniedrigalkylgruppe darstellen;
b/ oder R₅ und R₆ gemeinsam mit dem sie tragenden Stickstoffatom ein Morpholin-. Piperidin-, Pyrrolidin- oder Piperazinsystem bilden, welches gegebenenfalls am Stickstoffatom in der 4-Stellung durch eine Niedrigalkylgruppe, Arylgruppe, substituierte Arylgruppe, Arylniedrigalkylgruppe oder substituierte Arylniedrigalkylgruppe substituiert ist,
oder
R₁ eine Gruppe G₂ darstellt worin G₂ eine Gruppe (CH₂)ₘCOR₇ oder G₃ darstellt, worin G₃ eine Gruppe (CH₂)ₘCHOHR₇ bedeutet, worin m die oben angegebene Bedeutung besitzt und R₇ für eine Arylgruppe oder eine substituierte Arylgruppe steht,
R₃ ein Wasserstoffatom, eine Niedrigalkylgruppe, ein Phenylkern, der durch eine Hydroxygruppe oder Hydroxyalkylgruppe oder durch zwei bis fünf Gruppen ausgewählt aus Niedrigalkylgruppen, Niedrigalkoxygruppen, Halogenatomen, Trifluormethylgruppen, Hydroxylgruppen oder Hydroxyalkylgruppen substituiert ist, oder R₃ einen gegebenenfalls substituierten Naphthylkern oder eine gegebenenfalls substituierte Heteroarylgruppe bedeutet,
R₄ ein Wasserstoffatom, eine Gruppe G₁, worin G₁ die oben angegebenen Bedeutungen besitzt, eine Gruppe G₄, d. h. eine Gruppe der Formel:
-COO - G₁
in der G₁ die oben angegebenen Bedeutungen besitzt, oder eine Gruppe G₅, d. h. COR₉, in der R₉ für eine Arylgruppe, eine substituierte Arylgruppe, eine Heteroarylgruppe oder eine substituierte Heteroarylgruppe steht, bedeutet,
mit Ausnahme der Derivate, bei denen gleichzeitig R₄ ein Wasserstoffatom und R₁ eine Gruppe bedeuten: 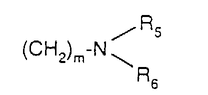 in der m = 2 ist
und R₅ und R₆ gleichzeitig eine Ethylgruppe bedeuten, während R₃ eine Niedrigalkylgruppe oder Naphthylgruppe darstellt,
und der Derivate, bei denen R₅ und R₆ gemeinsam mit dem sie tragenden Stickstoffatom ein Piperidin-, Pyrrolidin- oder Piperazinsystem bilden, welches gegebenenfalls am Stickstoffatom in der 4-Stellung durch eine Niedrigalkylgruppe, Arylgruppe, Arylniedrigalkylgruppe, substituierte Arylgruppe oder substituierte Arylniedrigalkylgruppe substituiert ist und R₃ ein Wasserstoffatom bedeutet,
oder
**1.B.**
X und R₁ gemeinsam eine Bindung bilden,
R₂ eine Gruppe G₁, G₂ oder G₃ darstellt, worin G₁, G₂ oder G₃ die oben angegebenen Bedeutungen besitzen und in diesem Fall R₃ ein Wasserstoffatom, eine Niedrigalkylgruppe, eine substituierte Phenylgruppe, eine Naphthylgruppe, eine substituierte Naphthylgruppe, eine Heteroarylgruppe, eine substituierte Heteroarylgruppe oder eine Gruppe G₁ bedeutet, worin G₁ die oben angegebenen Bedeutungen besitzt, während R₄ ein Wasserstoffatom oder eine Gruppe G₁, G₄ oder G₅ darstellt, worin G₄ und G₅ die oben angegebenen Bedeutungen besitzen,
wobei es sich versteht, daß, wenn R₂ eine Gruppe G₁ darstellt und G₁ die oben angegebenen Bedeutungen besitzt, R₃ von einem Wasserstoffatom verschieden ist,
oder
**2.**
n = 1 ist,
A, B, C und D, die gleichartig oder verschieden sind, ein Wasserstoffatom, ein Halogenatom, eine Niedrigalkylgruppe oder eine Trifluormethylgruppe bedeuten,
X und R₁ eine Bindung bilden oder X und R₂ eine Bindung bilden, wobei mindestens eine dieser beiden Bindungen in dem Molekül vorhanden ist,
Y und Z jeweils ein Wasserstoffatom bedeuten oder gemeinsam eine Bindung bilden,
R₁ oder R₂- in Abhängigkeit davon, ob X und R₂ oder X und R₁ eine Bindung bilden - dann eine Methylgruppe oder G₁ oder G₂ oder G₃ darstellen, worin G₁, G₂ und G₃ die oben angegebenen Bedeutungen besitzen,
R₃ ein Wasserstoffatom, eine Niedrigalkylgruppe, eine Arylgruppe, eine substituierte Arylgruppe, eine Heteroarylgruppe oder eine substituierte Heteroarylgruppe darstellt und
R₄ ein Wasserstoffatom oder eine Gruppe G₁ oder G₄ oder G₅ darstellt, worin G₁, G₄ und G₅ die oben angegebenen Bedeutungen besitzen,
deren Isomere, Enantiomere, Diastereoisomere sowie deren Additionssalze mit einer pharmazeutisch annehmbaren Säure.
wobei es sich versteht, daß unter einer Niedrigalkylgruppe oder Niedrigalkoxygruppe geradkettige oder verzweigte Gruppen mit 1 bis 6 Kohlenstoffatomen zu verstehen sind,
daß unter einer Arylgruppe eine Phenylgruppe oder eine Naphthylgruppe zu verstehen ist,
daß unter einer Heteroarylgruppe eine Furyl-, Thienyl-, Pyridyl-, Pyrrolyl-, Imidazolyl-, Benzimidazolyl-, Benzofuryl-, Benzothienyl-, Chinolyl-, Isochinolyl- oder Indolyl-gruppe zu verstehen ist,
wobei der Begriff substituiert, unter Bezugnahme auf die Begriffe Arylalkyl, Aryl, Phenyl, Naphthyl oder Heteroaryl bedeutet, wenn nichts anderes angegeben ist, daß die betreffenden Gruppen an ihrem cyclischen Teil durch eine bis drei Gruppen ausgewählt aus Halogenatomen, Trifluormethylgruppen, Hydroxylgruppen, Niedrigalkoxygruppen, Hydroxyniedrigalkylgruppen oder Niedrigalkylgruppen substituiert sind,
und daß, wenn in einem Molekül mehrere Gruppen G₁ oder Gruppe R₅ und R₆ vorhanden sind, diese gleichartig oder verschieden sein können.

2. Verbindungen nach Anspruch 1, worin n 0 bedeutet, X und R₂ eine Bindung bilden und Z und Y jeweils ein Wasserstoffatom darstellen, deren Isomere, Epimere, Diastereoisomere sowie gegebenenfalls deren Additionssalze mit einer pharmazeutisch annehmbaren Säure.

3. Verbindungen nach Anspruch 1, worin n 0 bedeutet, X und R₂ einerseits und Y und Z andererseits eine Bindung bilden, deren Isomere, Epimere, Diastereoisomere sowie deren Additionssalze mit einer pharmazeutisch annehmbaren Säure.

4. Verbindungen nach Anspruch 1, worin R₄ ein Wasserstoffatom darstellt, deren Isomere, Epimere, Diastereoisomere sowie deren Additionssalze mit einer pharmazeutisch annehmbaren Säure.

5. Verbindungen nach Anspruch 1, worin n 1 bedeutet, Y und Z jeweils ein Wasserstoffatom darstellen und X und R₂ gemeinsam eine Bindung bilden, deren Isomere, Epimere, Diastereoisomere sowie deren Additionssalze mit einer pharmazeutisch annehmbaren Säure.

6. Verbindungen nach Anspruch 1, worin n den Wet 1 besitzt, Y und Z jeweils ein Wasserstoffatom darstellen und R₁ und X gemeinsam eine Bindung bilden, deren Isomere, Epimere, Diastereoisomere sowie deren Additionssalze mit einer pharmazeutisch annehmbaren Säure.

7. Verbindungen nach Anspruch 1, worin n den Wert 0 besitzt, Y und Z gemeinsam eine Bindung bilden und X und R₁ gemeinsam eine Bindung darstellen, deren Isomere, Epimere, Diastereoisomere sowie deren Additionssalze mit einer pharmazeutisch annehmbaren Säure.

8. Verbindungen nach Anspruch 1, nämlich 9-(2-Diethylaminoethyl)-2,3-dihydroimidazo[1,2-a]benzimidazol, dessen Isomere sowie dessenAdditionssalze mit einer pharmazeutisch annehmbaren Säure.

9. Verbindungen nach Anspruch 1, nämlich:
9-Morpholinoethyl-2,3-dihydroimidazo[1,2-a]benzimidazol,
9-Phenacyl-2,3-dihydroimidazo[1,2-a]benzimidazol,
9-(p-Chlorphenacyl)-2,3-dihydroimidazo[1,2-a]benzimidazol,
9-(p-Methoxyphenacyl)-2,3-dihydroimidazo[1,2-a]benzimidazol,
9-(1-Naphthoylmethyl)-2,3-dihydroimidazo[1,2-a]benzimidazol,
9-(2-Hydroxy-2-phenylethyl)-2,3-dihydroimidazol[1,2-a]benzimidazol,
9-[2-Hydroxy-2-(4'-chlorphenyl)-ethyl]-2,3-dihydroimidazol[1,2-a]benzimidazol,
9-[2-(1-Naphthyl)-2-hydroxyethyl]-2,3-dihydroimidazo[1,2-a]benzimidazol,
2-tert.-Butyl-9-(2-morpholinoethyl)-imidazo[1,2-a]benzimidazol,
2-tert.-Butyl-9-(3-dimethylaminopropyl)-imidazo[1,2-a]benzimidazol,
9-(2-Morpholinoethyl)-2-(4-hydroxyphenyl)-imidazo[1,2-a]benzimidazol,
9-(2-Morpholinoethyl)-2-(3-hydroxyphenyl)-imidazo[1,2-a]benzimidazol,
9-Diethylaminoethyl-2-(3,4-dimethoxyphenyl)-imidazo[1,2-a]benzimidazol,
9-Diethylaminoethyl-2-(3,4-dihydroxyphenyl)-imidazol[1,2-a]benzimidazol,
9-(2-Piperidinoethyl)-2-(3,4-dihydroxyphenyl)-imidazo[1,2-a]benzimidazol,
9-(2-Diethylaminoethyl)-2-(1-methyl-2-benzimidazolyl)-imidazo[1,2-a]benzimidazol,
9-(2-Diethylaminoethyl)-2-(2-thienyl)-imidazo[1,2-a]benzimidazol,
9-(2-Piperidinoethyl)-2-(2-thienyl)-imidazo[1,2-a]benzimidazol,
9-(2-Piperidinoethyl)-2-(5-brom-2-thienyl)-imidazo[1,2-a]benzimidazol,
2-(5-Brom-2-thienyl)-9-(2-morpholinoethyl)-imidazo[1,2-a]benzimidazol,
2-(2-Furyl)-9-(2-N.N-diethylamlnoethyl) -imidazol[1,2-a]benzimidazol,
2-(2-Furyl)-9-(2-morpholinoethyl)-imidazo[1,2-a]benzimidazol,
2-Methyl-9-(2-morpholinoethyl)-3-(4-chlorbenzoyl)-imidazo[1,2-a]benzimidazol,
2-Methyl-9-(2-piperidinoethyl)-3-(2-furoyl)-imidazo[1,2-a]benzimidazol,
2-Methyl-9-(2-piperidinoethyl)-3-(2-thenoyl)-imidazo[1,2-a]benzimidazol,
2-Methyl-9-(2-morpholinoethyl)-3-(5-brom-2-thenoyl)-imidazo[1,2-a]benzimidazol,
2-tert.-Butyl-1-(2-morpholinoethyl)-imidazo[1,2-a]benzimidazol,
2-tert.-Butyl-1-(2-piperidinoethyl)-imidazo[1,2-a]benzimidazol,
2-tert.-Butyl-1-(2-N,N-diethylaminoethyl)-imidazo[1,2-a]benzimidazol,
10-(2-Diethylaminoethyl)-2,3,4,10-tetrahydropyrimidino[1,2-a]benzimidazol,
10-Phenacyl-2,3,4,10-tetrahydropyrimidino[1,2-a]benzimidazol,
10-(4'-Chlorphenacyl)-2,3,4,10-tetrahydropyrimidino[1,2-a]benzimidazol,
10-(4'-Methoxyphenacyl)-2,3,4,10-tetrahydropyrimidino[1,2-a]benzimidazol,
10-[2-Hydroxy-2-(4-chlorphenyl)-ethyl]-2,3,4,10-tetrahydropyrimidino[1,2-a]-benzimidazol,
10-[2-Hydroxy-2-(4-methoxyphenyl)-ethyl]-2,3,4,10-tetrahydropyrimidino[1,2-a]-benzimidazol,
1-(2-Diethylaminoethyl)-1,2,3,4-tetrahydropyrimidino[1,2-a]benzimidazol,
1-(2-Piperidinoethyl)-1,2,3,4-tetrahydropyrimidino[1,2-a]benzimidazol,
1-(2-Morpholinoethyl)-1,2,3,4-tetrahydropyrimidino[1,2-a]benzimidazol,
9-[2-Hydroxy-2-(4-methoxyphenyl)-ethyl]-2,3-dihydroimidazo[1,2-a]benzimidazol,
deren Isomere, Epimere, Diastereosiomere sowie deren Additionssalze mit einer pharmazeutisch annehmbaren Säure.

10. Verfahren zur Herstellung der Derivate der allgemeinen Formel (I) nach Anspruch 1, dadurch gekennzeichnet, daß man:
wenn bei dem herzustellenden Derivat der Formel (I) X und R₂ gemeinsam eine Bindung bilden. man als Ausgangsmaterial ein Derivat der Formel (II) verwendet: 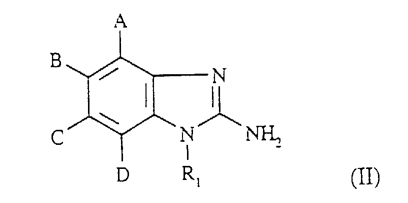 in der A, B, C, D und R₁ die oben angegebenen Bedeutungen besitzen, welches man:
wenn bei dem herzustellenden Derivat der Formel (I) Y und Z jeweils ein Wasserstoffatom darstellen, mit einem Derivat der Formel (III/A) behandelt:
Hal_{A}-(CH₂)ₙ-CHR₄-CHR₃-P (III/A)
in der Hal_{A} ein Halogenatom darstellt, n, R₃ und R₄ die oben angegebenen Bedeutungen besitzen und P eine aus Halogen oder Hydroxy ausgewählte austretende Gruppe darstellt,
so daß man ein Derivat der Formel (IV/A) erhält: 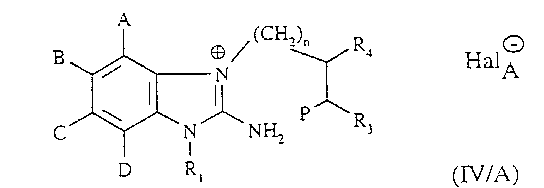 in der A, B, C, D, R₁ die bezüglich der Formel (I) angegebenen Bedeutungen besitzen und n, R₃, R₄ und P die oben angegebenen Bedeutungen besitzen,
welches durch Erhitzen, dem, wenn P eine Hydroxylgruppe darstellt. einer Behandlung mit einem Halogenierungsmittel vorausgeht, zu einem Derivat der Formel (I/B) führt: 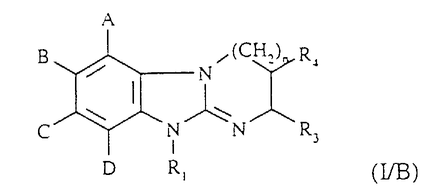 einem Sonderfall der Derivate der Formel (I), in der A, B, C, D, R₁, R₃, R₄ und n die oben angegebenen Bedeutungen besitzen,
X und R₂ gemeinsam eine Bindung bilden und Y und Z jeweils ein Wasserstoffatom darstellen,
welches Derivat der Formel (I/B) man erforderlichenfalls reinigt und gewünschtenfalls mit einer Säure in ein pharmazeutisch annehmbares Salz umwandelt.

11. Verfahren zur Herstellung der Derivate der allgemeinen Formel (I) nach Anspruch 1, dadurch gekennzeichnet, daß
wenn in dem herzustellenden Derivat der Formel (I) X und R₂ gemeinsam eine Bindung bilden, man als Ausgangsmaterial ein Derivat der Formel (II) verwendet: 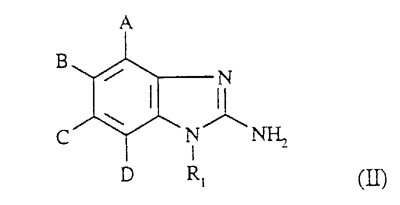 in der A, B, C, D und R₁ die oben angegebenen Bedeutungen besitzen, welches man:
wenn bei dem herzustellenden Derivat der Formel (I) Y und Z gemeinsam eine Bindung bilden,
mit einem Derivat der Formel (III) behandelt: 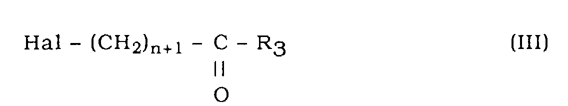 in der n die oben angegebene Bedeutung besitzt, R₃ in Abhängigkeit von den Bedeutungen von R₁ die bezüglich der Formel (I) angegebenen Bedeutungen besitzt und Hal ein Halogenatom darstellt,
zur Bildung eines Derivats der Formel (IV): 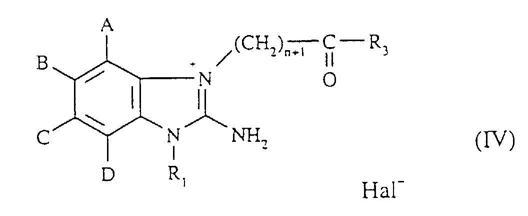 in der Hal, A, B, C, D, R₁, n und R₃ die oben angegebenen Bedeutungen besitzen, welches durch Erhitzen zu einem Derivat der Formel (I/A) führt: 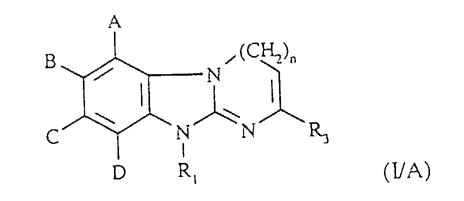 einem Sonderfall der Derivate der Formel (I), in derA, B, C, D, R₁, R₃ und n die oben angegebenen Bedeutungen besitzen und X und R₂ einerseits und Y und Z andererseits gemeinsam eine Bindung bilden und R₄ Wasserstoffatom darstellt,
welches Derivat der Formel (I/A) man, wenn bei dem herzustellenden Derivat der Formel (1) R₄ von Wasserstoff verschieden ist:
- wenn bei dem herzustellenden Derivat der Formel (I) R₄ eine Gruppe G₄ und Y und Z eine Bindung bilden,
mit einem Derivat der Formel (V/A) behandelt:
Hal'₃- C - CO - Hal'' (V/A)
in der Hal' und Hal", die gleichartig oder verschieden sind, jeweils ein Halogenatom darstellen, so daß man ein Derivat der Formel (VI) erhält: 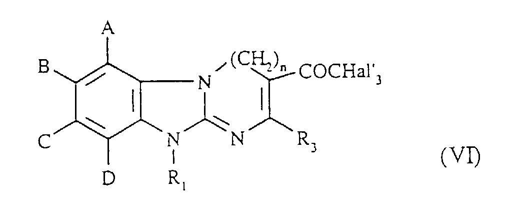 in der A, B, C, D, R₁, n, R₃ und Hal'₃ die oben angegebenen Bedeutungen besitzen, welches Derivat der Formel (VI) man mit einer Verbindung der Formel (VII) behandelt: 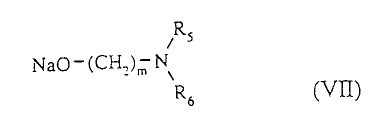 in der m, R₅ und R₆ die bezüglich der Formel der Gruppe G₄, wie sie bezüglich der Formel (I) definiert worden ist, angegebenen Bedeutungen besitzen,
zur Bildung eines Derivats der Formel (I/C): 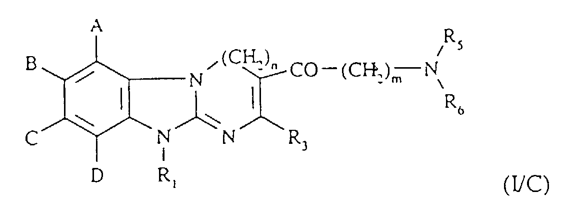 in der A, B, C, D, R₁, R₃, R₅, R₆ und n die bezüglich der Formel (I) angegebenen Bedeutungen besitzen,
Y und Z einerseits und X und R₂ andererseits gemeinsam eine Doppelbindung bilden und R₄ eine Gruppe G₄ darstellt,
welches Derivat der Formel (I/A) oder (I/C) man erforderlichenfalls reinigt und gewünschtenfalls mit einer Säure in ein pharmazeutisch annehmbares Salz umwandelt.

12. Verfahren zur Herstellung der Derivate der allgemeinen Formel (I) nach Anspruch 1, dadurch gekennzeichnet, daß man:
wenn bei dem herzustellenden Derivat der Formel (I) X und R₂ gemeinsam eine Bindung bilden, man als Ausgangsmaterial ein Derivat der Formel (II) verwendet: 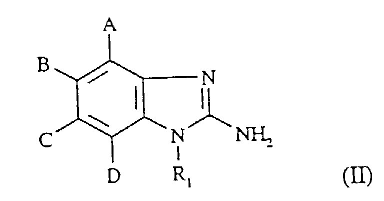 in der A, B, C, D und R₁ die oben angegebenen Bedeutungen besitzen, welches man:
wenn bei dem herzustellenden Derivat der Formel (I) R₄ eine Gruppe G₄ darstellt und Y und Z eine Bindung bilden, mit einem Derivat der Formel (VIII) behandelt:
Hal'''-(CH₂)ₘ-Hal₄ (VIII)
in der Hal''' und Hal₄, die gleichartig oder verschieden sind, ein Halogenatom bedeuten und m die oben angegebene Bedeutung besitzt, zur Bildung eines Derivats der Formel (IX): 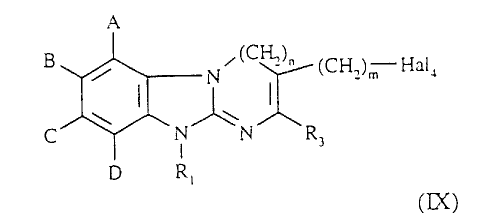 in der A, B, C, D, R₁, n, m, R₃ und Hal₄ die oben angegebenen Bedeutungen besitzen,
welches man dann mit einem Derivat der Formel (X) behandelt: 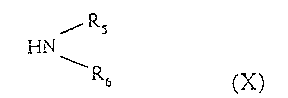 in der R₅ und R₆ die oben angegebenen Bedeutungen besitzen,
zur Bildung eines Derivats der Formel (I/D): 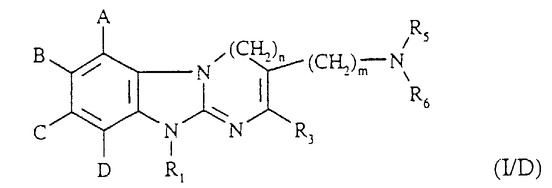 einem Sonderfall der Derivate der Formel (I), in der A, B, C, D, R₁ und n die bezüglich der Formel (I) angegebenen Bedeutungen besitzen und X und R₂ einerseits und Y und Z andererseits gemeinsam eine Bindung bilden und R₄ eine Gruppe G₁ darstellt, wie sie bezüglich der Formel (II) definiert worden ist,
welches Derivat der Formel (I/D) man erforderlichenfalls reinigt und gewünschtenfalls mit einer Säure in ein pharmazeutisch annehmbares Salz umwandelt.

13. Verfahren zur Herstellung der Derivate der allgemeinen Formel (I) nach Anspruch 1, dadurch gekennzeichnet, daß man:
wenn bei dem herzustellenden Derivat der Formel (I) X und R₂ gemeinsam eine Bindung bilden, als Ausgangsmaterial ein Derivat der Formel (II) verwendet: 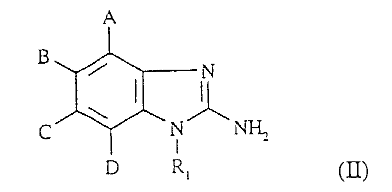 in der A, B, C, D und R₁ die oben angegebenen Bedeutungen besitzen,
welches man:
wenn bei dem herzustellenden Derivat der Formel (I) R₄ eine Gruppe G₅ oder G₇ darstellt und Y und Z eine Bindung bilden, in Abhängigkeit von dem herzustellenden Derivat mit einem Derivat der Formel (XI) behandelt:
Cl - CO - R₉ oder Cl - CO - R₁₀ (XI)
worin R₉ und R₁₀ die oben angegebenen Bedeutungen besitzen,
zur Bildung eines Derivats der Formel (I/E): 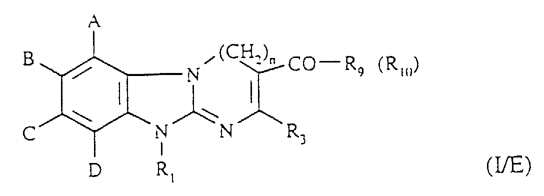 einem Sonderfall der Derivate der Formel (I), worin A, B, C, D, n und R₃ die oben angegebenen Bedeutungen besitzen, X und R₂ einerseits und Y und Z andererseits gemeinsam eine Bindung bilden und R₄ eine Gruppe G₅ oder G₇ darstellen, wie sie oben definiert worden sind,
welches Derivat der Formel (I/E) man erforderlichenfalls reinigt und gewünschtenfalls mit einer Säure in ein pharmazeutisch annehmbares Salz umwandelt.

14. Verfahren zur Herstellung der Derivate der Formel (I) nach Anspruch 1, dadurch gekennzeichnet, daß bei dem herzustellenden Derivat der Formel (I) X und R₁ gemeinsam eine Bindung bilden,
man als Ausgangsmaterial ein Derivat der Formel (XII) verwendet: 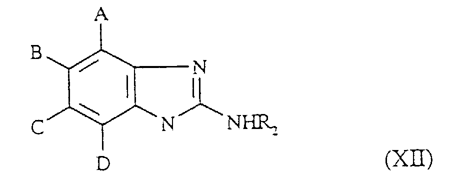 in der A, B, C, D und R₂ die oben angegebenen Bedeutungen besitzen,
* welches man, wenn bei dem herzustellenden Derivat der Formel (I) Y und Z jeweils ein Wasserstoffatom darstellen,
mit einem Derivat der Formel (III/A), wie es oben definiert worden ist, behandelt, so daß man nach dem Erhitzen ein Derivat der Formel (I/G) erhält: 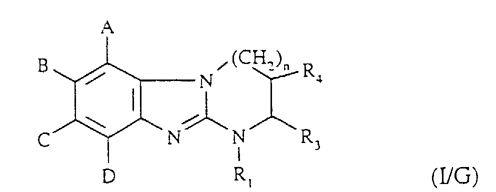 einem Sonderfall der Derivate der Formel (I), in der A, B, C, D, n, R₁, R₃ und R₄ die oben angegebenen Bedeutungen besitzen,
X und R₁ gemeinsam eine Bindung bilden und Y und Z jeweils ein Wasserstoffatom darstellen,
* wenn bei dem herzustellenden Derivat der Formel (I) Y und Z eine Bindung bilden,
mit einem Derivat der Formel (III), wie sie oben definiert worden ist, behandelt, so daß man nach dem Erhitzen ein Derivat der Formel (I/F) erhält: 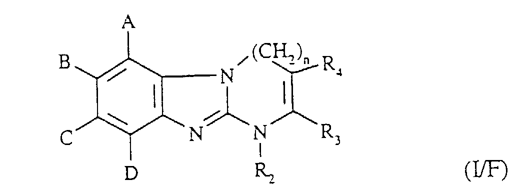 einem Sonderfall der Derivate der Formel (I), in der A, B, C, D, n, R₂ und R₃ die oben angegebenen Bedeutungen besitzen,
X und R₁ einerseits und Y und Z andererseits gemeinsam eine Bindung bilden und R₄ ein Wasserstoffatom darstellt,
welches Derivat der Formel (I/F) man in Abhängigkeit von der Bedeutung von R₄ von dem herzustellenden Derivat der Formel (I):
- nacheinander, wie es oben angegeben worden ist, mit einem Derivat der Formel (V/A), wie es oben definiert worden ist, und dann mit einem Derivat der Formel (VII), wie es oben definiert worden ist, behandelt,
- nacheinander, wie es oben angegeben ist, mit einem Derivat der Formel (VIII), wie es oben definiert worden ist, und dann mit einem Derivat der Formel (X), wie es oben definiert worden ist, behandelt,
- oder mit einem Derivat der Formel (XI), wie es oben definiert worden ist, behandelt,
zur Bildung eines Derivats der Formel (I/H): 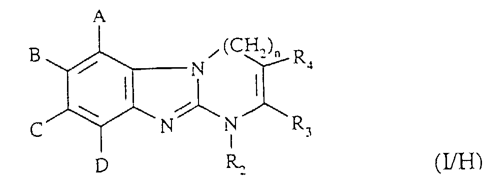 einem Sonderfall der Derivate der Formel (I), in der A, B, C, D, n, R₂, R₃ und R₄ die bezüglich der Formel (I) angegebenen Bedeutungen besitzen,
X und R₁ einerseits und Y und Z andererseits jeweils eine Bindung bilden,
welche Derivate der Formeln (I), (I/F), (I/G) und (I/H) man erforderlichenfalls mit einer aus Chromatographie und/oder Kristallisation ausgewählten Methode reinigt und gewünschtenfalls mit einer Säure in ein pharmazeutisch annehmbares Salz umwandelt.

15. Verfahren zur Herstellung der Derivate der Formel (I/B) nach Anspruch 14, dadurch gekennzeichnet, daß man ein Derivat der Formel (IV/B): 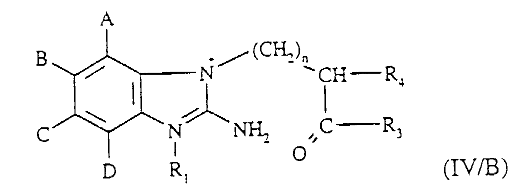 welches man durch Kondensation eines Derivats der Formel (II) mit einem Derivat der Formel (III/B) erhalten hat: 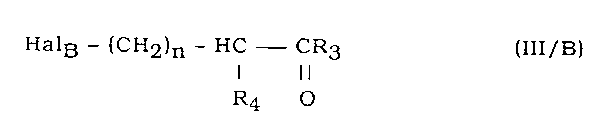 in der Hal_{B} ein Halogenatom darstellt und n, R₃ und R₄ die oben angegebenen Bedeutungen besitzen,
einer Reduktion mit einem gemischten Alkalimetallhydrid unterwirft zur Bildung eines Derivates der Formel (IV/A), wie es oben definiert worden ist, bei der die Gruppe P eine Hydroxylgruppe darstellt, welches durch Behandlung mit einem Halogenierungsmittel und durch Erhitzen zu einem Derivat der Formel (I/B), wie sie oben definiert worden ist, führt, welches man erforderlichenfalls reinigt und gewünschtenfalls mit einer Säure in ein pharmazeutisch annehmbares Salz umwandelt.

16. Verfahren zur Herstellung der Derivate der Formel (I/B) nach Anspruch 14, dadurch gekennzeichnet, daß man als Ausgangsmaterial ein Derivat der Formel (XIII) verwendet: 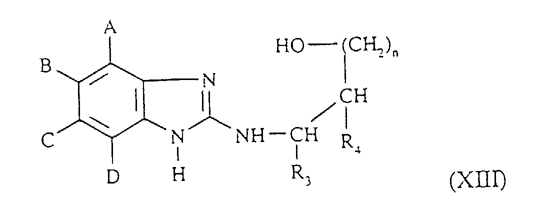 in der A, B, C, D und n die oben angegebenen Bedeutungen besitzen, welches man:
entweder mit einem Halogenierungsmittel behandelt und dann erhitzt unter Bildung eines Derivates der Formel (XIV): 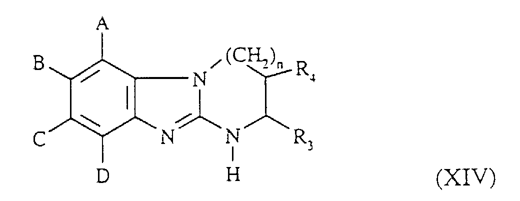 in der A, B, C, D, R₃, R₄ und n die oben angegebenen Bedeutungen besitzen, welches man mit einem Derivat der Formel (XV) behandelt:
Hal - (CH₂)ₘ-CO - R₇ (XV)
in der m und R₇ die bezüglich der Formel (I) angegebenen Bedeutungen besitzen und Hal ein Halogenatom darstellt,
zur Bildung eines Derivats der Formel (I/J): 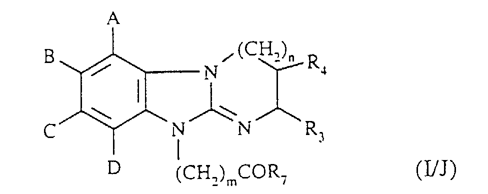 in der A, B, C, D, m, n, R₃, R₄ und R₇ die oben angegebenen Bedeutungen besitzen, Y und Z ein Wasserstoffatom bedeuten und X und R₂ gemeinsam eine Bindung bilden und R₁ eine Gruppe G₂ darstellt,
welches Derivat der Formel (I/J) man der Einwirkung eines gemischten Alkalimetallhydrids unterwerfen kann zur Bildung eines Derivats der Formel (I/K): 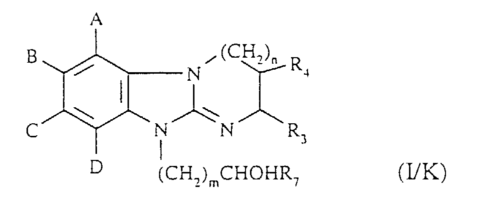 in der A, B, C, D, m, n, R₃, R₄ und R₇ die oben angegebenen Bedeutungen besitzen, Y und Z jeweils ein Wasserstoffatom darstellen, X und R₂ gemeinsam eine Bindung bilden und R₁ eine Gruppe G₃ darstellt,
- oder mit dem Derivat der Formel (XV), wie es oben definiert worden ist, behandelt zur Bildung eines Derivats der Formel (XVI): 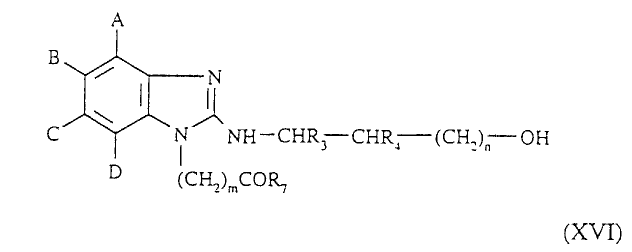 in der A, B, C, D, R₃, R₄, n, m und R₇ die oben angegebenen Bedeutungen besitzen, welches durch Behandlung mit einem Halogenierungsmittel und durch Erhitzen zu einem Derivat der Formel (I/J), wie sie oben definiert worden ist, führt,
welche Derivate der Formeln (I/J) und (I/K) man erforderlichenfalls reinigt und gewünschtenfalls mit einer Säure in ein pharmazeutisch annehmbares Salz umwandelt.

17. Verfahren zur Herstellung der Derivate der Formeln (I/J) oder (I/K), wie sie in Anspruch 16 definiert sind, welches darin besteht, als Ausgangsmaterial ein Derivat der Formel (XIV) einzusetzen, welches man nach der in Anspruch 13 beschriebenen Weise behandelt.

18. Verfahren zur Herstellung der Derivate der Formel (I/K), wie sie in Anspruch 16 definiert worden sind, welches darin besteht, das Derivat der Formel (XIV), wie es in Anspruch 16 definiert worden ist, mit einem Derivat der Formel (XVII) zu behandeln:
Hal-(CH₂)ₘ- CHOH-R₇ (XVII)
in der Hal, m und R₇ die bezüglich der Formel (I) angegebenen Bedeutungen besitzen, und man das erhaltene Derivat erforderlichenfalls reinigt und gewünschtenfalls mit einer Säure in ein pharmazeutisch annehmbares Salz umwandelt.

19. Verfahren zur Herstellung der Derivate der Formel (I) nach Anspruch 16, dadurch gekennzeichnet, daß man als Ausgangsmaterial Derivate der Formel (XVIII) verwendet: 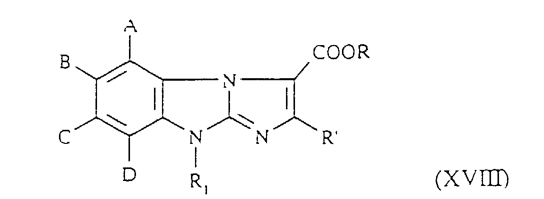 in der A, B, C, D und R₁ die oben angegebenen Bedeutungen besitzen, R eine Niedrigalkylgruppe oder ein Halogenatom darstellt und R' eine gegebenenfalls durch ein Halogenatom oder eine Hydroxylgruppe substituierte Niedrigalkylgruppe bedeutet.

20. Verfahren zur Synthese der Derivate der Formel (I), in der R₄ eine Gruppe CH₂NR₅R₆ darstellt, in der R₅ und R₆ die in Anspruch 1 angegebenen Bedeutungen besitzen, dadurch gekennzeichnet, daß man ein Derivat der Formel (I/A), wie es in Anspruch 14 definiert worden ist, einer Mannich-Reaktion unterwirft unter Verwendung von Formaldehyd und eines Amins HNR₅R₆, worauf man das Produkt erforderlichenfalls reinigt und mit einer Säure in ein pharmazeutisch annehmbares Salz umwandelt.

21. Verfahren zur Synthese der Derivate der Formel (I), in der R₂ eine Gruppe G₁, wie sie in Anspruch 1 definiert worden ist, darstellt, dadurch gekennzeichnet, daß man als Ausgangsmaterial eine Verbindung der Formel (XX) verwendet: 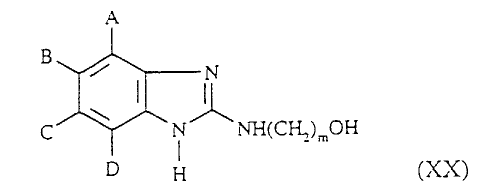 in der A, B, C, D und m die oben angegebenen Bedeutungen besitzen, welches man mit einem Derivat der Formel (XXI) behandelt:
Hal - (CH₂)ₙ₊₁-COR₃ (XXI)
in der n und R₃ die oben angegebenen Bedeutungen besitzen und Hal ein Halogenatom darstellt, zur Bildung eines Derivats der Formel (XXII): 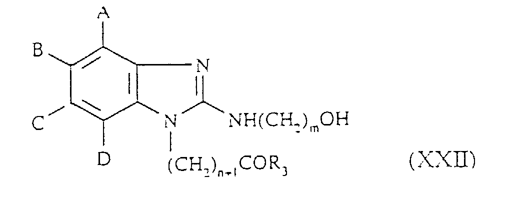 in der A, B, C, D, R₃ und m die bezüglich der Formel (I) angegebenen Bedeutungen besitzen, welches durch Erhitzen zur Bildung eines Derivats der Formel (XXIII) führt: 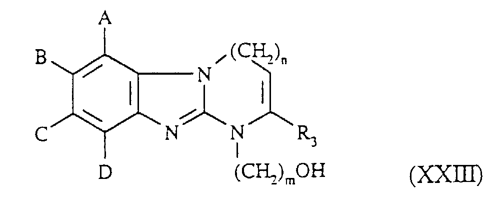 in der A, B, C, D, m, n und R₃ die bezüglich der Formel (I) angegebenen Bedeutungen besitzen,
welches man:
a) mit einem Halogenierungsmittel,
b) mit einem Amin der Formel HNR₅R₆ behandelt
zur Bildung eines Derivats der Formel (I/M): 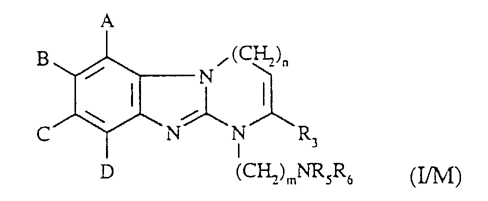 einem Sonderfall der Derivate der Formel (I), in der A, B, C, D, m, n, R₃, R₅ und R₆ die bezüglich der Formel (I) angegebenen Bedeutungen besitzen,
bei denen R₁ und X und Y und Z jeweils eine Bindung darstellen und R₂ eine Gruppe G₁ bedeutet, welche man erforderlichenfalls reinigt und gewünschtenfalls mit einer Säure in ein pharmazeutisch annehmbares Salz umwandelt.

22. Pharmazeutische Zubereitung enthaltend als Wirkstoff mindestens eine Verbindung nach einem derAnsprüche 1 bis 9 in Kombination mit einem oder mehreren pharmazeutisch annehmbaren Trägermaterialien oder Bindemitteln.

23. Pharmazeutische Zubereitung nach Anspruch 22 zur Behandlung des Diabetes und/oder seiner kardiovaskulären Komplikationen.

## Claims

1. Compounds of the general formula (I) : 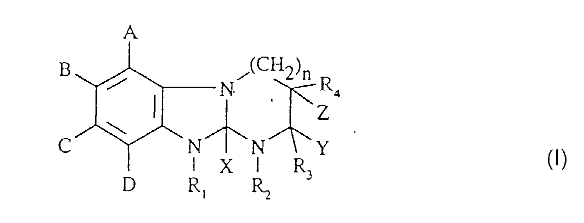 wherein :
**1.**
n = 0,
A, B, C and D are the same or different and represent a hydrogen atom, a halogen atom, or a lower alkyl, lower alkoxy, hydroxy, trifluoromethyl or hydroxy-lower alkyl group, and
Y and Z each represent a hydrogen atom or together form a bond, in which case either:
**1.A.**
X and R₂ toaether form a bond, and in that case :
R₁ represents a grouping G₁,
G₁ represents the grouping 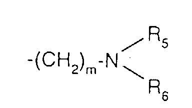 wherein m is an integer of from 1 to 6 inclusive and R₅ and R₆ are the same or different and represent :
a/ independently of one another, a hydrogen atom, a lower alkyl group, an aryl-lower alkyl group or a substituted aryl-lower alkyl group;
b/ or R₅ and R₆ form, simultaneously with the nitrogen atom carrying them, a morpholine, piperidine, pyrrolidine or piperazine system that is optionally substituted on the nitrogen atom in the 4 position by a lower alkyl, aryl, substituted aryl, aryl-lower alkyl or substituted aryl-lower alkyl group,
or
R₁ represents a grouping G₂, G₂ denoting a (CH₂)ₘCOR₇ grouping, or a grouping G₃, G₃ denoting a (CH₂)ₘCHOHR₇ grouping, m being as defined above and R₇ denoting an aryl or substituted aryl group,
R₃ represents a hydrogen atom, a lower alkyl group, a phenyl nucleus substituted by a hydroxy or hydroxyalkyl group or by from two to five groups selected from lower alkyl, lower alkoxy, halogen, trifluoromethyl, hydroxy and hydroxyalkyl, or R₃ represents an optionally substituted naphthyl nucleus or an optionally substituted heteroaryl group,
R₄ represents a hydrogen atom, a G₁ grouping, G₁ being as defined above, a grouping G₄, that is a grouping of formula :
-COO-G₁
wherein G₁ is as defined above, or a grouping G₅, that is -COR₉, wherein R₉ represents an aryl group, a substituted aryl group, a heteroaryl group or a substituted heteroaryl group,
with the exception of compounds wherein simultaneously R₄ represents a hydrogen atom and R₁ represents a grouping : 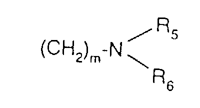 wherein m = 2,
and R₅ and R₆ simultaneously represent an ethyl group while R₃ represents a lower alkyl or naphthyl group,
and with the exception of compounds wherein R₅ and R₆ form simultaneously with the nitrogen atom carrying them a piperidine, pyrrolidine or piperazine system that is optionally substituted on the nitrogen atom in the 4 position by a lower alkyl, aryl, aryl-lower alkyl, substituted aryl or substituted aryl-lower alkyl group while R₃ represents a hydrogen atom;
or
**1.B**
X and R₁ together form a bond,
R₂ represents a G₁, G₂ or G₃ grouping, G₁, G₂ and G₃ being as defined above, and in that case R₃ represents a hydrogen atom, a lower alkyl group, a substituted phenyl group, a naphthyl group, a substituted naphthyl group, a heteroaryl group or a substituted heteroaryl group, or R₂ represents a G₁ grouping, G₁ being as defined above, while R₄ represents a hydrogen atom or a G₁, G₄ or G₅ grouping, G₄ and G₅ being as defined above,
provided that when R₂ represents a G₁ grouping, G₁ being as defined above, R₃ is other than a hydrogen atom,
or
**2.**
n = 1,
A, B, C and D are the same or different and represent a hydrogen atom, a halogen atom, a lower alkyl group or a trifluoromethyl group,
X and R₁ form a bond or X and R₂ form a bond, it being necessary for at least one of those two bonds to be present in the molecule,
Y and Z each represent a hydrogen atom or together form a bond,
R₁ or R₂- according to whether X and R₂ or X and R₁ form a bond - represents a methyl or G₁ or G₂ or G₃ grouping, G₁, G₂ and G₃ being as defined above,
R₃ represents a hydrogen atom, a lower alkyl group, an aryl group, a substituted aryl group, a heteroaryl group or a substituted heteroaryl group, and
R₄ represents a hydrogen atom or a G₁ or G₄ or G₅ grouping, G₁, G₄ and G₅ having the same definition as given above,
their isomers, enantiomers and diastereoisomers, and also the addition salts thereof with a pharmaceutically acceptable acid,
wherein
there is understood by lower alkyl or lower alkoxy group a linear or branched group having from 1 to 6 carbon atoms,
there is understood by aryl group a phenyl or naphthyl group,
there is understood by heteroaryl group a furyl, thienyl, pyridyl, pyrrolyl, imidazolyl, benzimidazolyl, benzofuryl, benzothienyl, quinolyl, isoquinolyl or indolyl group,
the term "substituted" relating to the terms arylalkyl, aryl, phenyl, naphthyl and heteroaryl denotes, unless specified to the contrary, that the groups in question are substituted on their cyclic moiety by from one to three radicals selected from halogen, trifluoromethyl, hydroxy, lower alkoxy, hydroxy-lower alkyl and lower alkyl,
and more than one G₁ grouping or R₅ and R₆ groupings may be present in the same molecule, it being possible for those groupings to be the same or different.

2. Compounds according to claim 1, wherein n is 0, X and R₂ form a bond, and Z and Y each represent a hydrogen atom, their isomers, epimers and diastereoisomers, and also, where appropriate, the addition salts thereof with a pharmaceutically acceptable acid.

3. Compounds according to claim 1, wherein n is 0, and X and R₂ on the one hand, and Y and Z on the other hand, form a bond, their isomers, epimers and diastereoisomers, and also the addition salts thereof with a pharmaceutically acceptable acid.

4. Compounds according to claim 1, wherein R₄ represents a hydrogen atom, their isomers, epimers and diastereoisomers, and also the addition salts thereof with a pharmaceutically acceptable acid.

5. Compounds according to claim 1, wherein n is 1, Y and Z each represent a hydrogen atom, and X and R₂ together form a bond, their isomers, epimers and diastereoisomers, and also the addition salts thereof with a pharmaceutically acceptable acid.

6. Compounds according to claim 1, wherein n is 1, Y and Z each represent a hydrogen atom, and R₁ and X together form a bond, their isomers, epimers and diastereoisomers, and also the addition salts thereof with a pharmaceutically acceptable acid.

7. Compounds according to claim 1, wherein n is 0, Y and Z together form a bond, and X and R₁ together form a bond, their isomers, epimers and diastereoisomers, and also the addition salts thereof with a pharmaceutically acceptable acid.

8. Compound according to claim 1, which is 9-(2-diethylaminoethyl)-2,3-dihydroimidazo[1,2-a]benzimidazole, its isomers, and also the addition salts thereof with a pharmaceutically acceptable acid.

9. Compounds according to claim 1 which are :
9-morpholinoethyl-2,3-dihydroimidazo[1,2-a]benzimidazole
9-phenacyl-2,3-dihydroimidazo[1,2-a]benzimidazole
9-(p-chlorophenacyl)-2,3-dihydroimidazo[1,2-a]benzimidazole
9-(p-methoxyphenacyl)-2,3-dihydroimidazo[1,2-a]benzimidazole
9-(1-naphthoylmethyl)-2,3-dihydroimidazo[1,2-a]benzimidazole
9-(2-hydroxy-2-phenylethyl)- 2,3-dihydroimidazo[1,2-a]benzimidazole
9-[2-hydroxy-2-(4'-chlorophenyl)ethyl]-2,3-dihydroimidazo[1,2-a]benzimidazole
9-[2-(1-naphthyl)-2-hydroxyethyl]-2,3-dihydroimidazo[1,2-a]benzimidazole
2-tert-butyl-9-(2-morpholinoethyl)imidazo[1,2-a]benzimidazole
2-tert-butyl-9-(3-dimethylaminopropyl)imidazo[1,2-a]benzimidazole
9-(2-morpholinoethyl)-2-(4-hydroxypheny)imidazo[1,2-a]benzimidazole
9-(2-morpholinoethyl)-2-(3-hydroxyphenyl)imidazo[1,2-a]benzimidazole
9-diethylaminoethyl-2-(3,4-dimethoxyphenyl)imidazo[1,2-a]benzimidazole
9-diethylaminoethyl-2-(3,4-dihydroxyphenyl)imidazo[1,2-a]benzimidazole
9-(2-piperidinoethyl)-2-(3,4-dihydroxyphenyl)imidazo[1,2-a]benzimidazole
9-(2-diethylaminoethyl)-2-(1-methyl-2-benzimidazolyl)imidazo[1,2-a]benzimidazole
9-(2-diethylaminoethyl)-2-(2-thienyl)imidazo[1,2-a]benzimidazole
9-(2-piperidinoethyl)-2-(2-thienyl)imidazo[1,2-a]benzimidazole
9-(2-piperidinoethyl)-2-(5-bromo-2-thienyl)imidazo[1,2-a]benzimidazole
2-(5-bromo-2-thienyl)-9-(2-morpholinoethyl)imidazo[1,2-a]benzimidazole
2-(2-furyl)-9-(2-N,N-diethylaminoethyl)imidazo[1,2-a]benzimidazole
2-(2-furyl)-9-(2-morpholinoethyl)imidazo[1,2-a]benzimidazole
2-methyl-9-(2-morpholinoethyl)-3-(4-chlorobenzoyl)imidazo[1,2-a]benzimidazole
2-methyl-9-(2-piperidinoethyl)-3-(2-furoyl)imidazo[1,2-a]benzimidazole
2-methyl-9-(2-piperidinoethyl)-3-(2-thenoyl)imidazo[1,2-a]benzimidazole
2-methyl-9-(2-morpholinoethyl)-3-(5-bromo-2-thenoyl)imidazo[1,2-a]benzimidazole
2-tert-butyl-1-(2-morpholinoethyl)imidazo[1,2-a]benzimidazole
2-tert-butyl-1-(2-piperidinoethyl)imidazo[1,2-a]benzimidazole
2-tert-butyl-1-(2-N,N-diethylaminoethyl)imidazo[1,2-a]benzimidazole
10-(2-diethylaminoethyl)-2,3,4,10-tetrahydropyrimidino[1,2-a]benzimidazole
10-phenacyl-2,3,4,10-tetrahydropyrimidino[1,2-a]benzimidazole
10-(4'-chlorophenacyl)-2,3,4,10-tetrahydropyrimidino[1,2-a]benzimidazole
10-(4'-methoxyphenacyl)-2,3,4,10-tetrahydropyrimidino[1,2-a]benzimidazole
10-[2-hydroxy-2-(4-chlorophenyl)ethyl)-2,3,4,10-tetrahydropyrimidino[1,2-a]benzimidazole
10-[2-hydroxy-2-(4-methoxyphenyl)ethyl]-2,3,4,10-tetrahydropyrimidino[1,2-a]benzimidazole
1-(2-diethylaminoethyl)-1,2,3,4-tetrahydropyrimidino[1,2-a]benzimidazole
1-(2-piperidinoethyl)-1,2,3,4-tetrahydropyrimidino[1,2-a]benzimidazole
1-(2-morpholinoethyl)-1,2,3,4-tetrahydropyrimidino[1,2-a]benzimidazole
9-[2-hydroxy-2-(4-methoxyphenyl)ethyl]-2,3-dihydroimidazo[1,2-a]benzimidazole
their isomers, epimers and diastereoisomers, and also the addition salts thereof with a pharmaceutically acceptable acid.

10. Process for the preparation of compounds of the general formula (I) according to claim 1, characterised in that :
when X and R₂ together form a bond in the compound of formula (I) it is desired to obtain, there is used as starting material a compound of formula (II) : 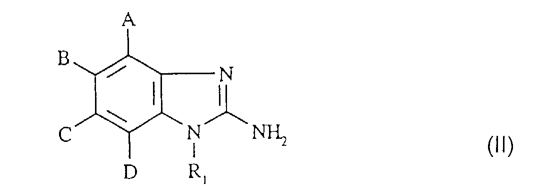 wherein A, B, C, D and R₁ are as defined hereinabove,
which is treated :
when Y and Z each represent a hydrogen atom in the compound of formula (I) it is desired to obtain, with a compound of formula (III/A) :
Hal_{A}-(CH₂)ₙ-CHR₄-CHR₃-P (III/A)
wherein Hal_{A} represents a halogen atom, n, R₃ and R₄ are as defined hereinabove and P represents a leaving group selected from halogen and hydroxy,
to yield a compound of formula (IV/A) : 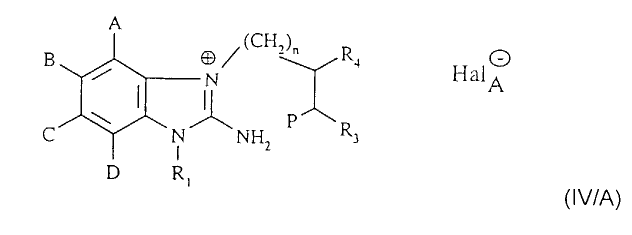 wherein A, B, C, D and R₁ are as defined for formula (I) and n, R₃, R₄ and P are as defined hereinabove,
which on heating, preceded when P represents a hydroxy group by treatment with a halogenation agent,
yields a compound of formula (I/B): 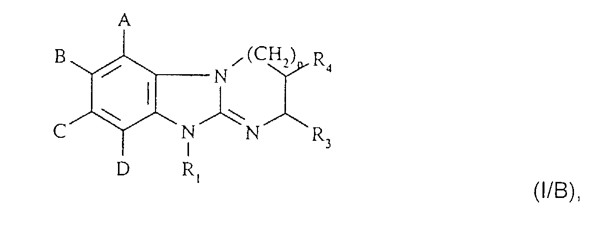 a particular case of the compounds of formula (I) wherein A, B, C, D, R₁, R₃, R₄ and n are as defined hereinabove,
X and R₂ together form a bond and Y and Z each represent a hydrogen atom,
which compound of formula (I/B) is, if necessary, purified and, if desired, converted with an acid into a pharmaceutically acceptable salt.

11. Process for the preparation of compounds of the general formula (I) according to claim 1, characterised in that :
when X and R₂ together form a bond in the compound of formula (I) it is desired to obtain, there is used as starting material a compound of formula (II) : 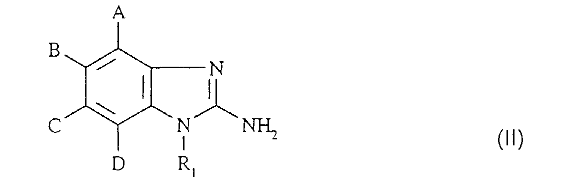 wherein A, B, C, D and R₁ are as defined hereinabove,
which is treated :
when Y and Z together form a bond in the compound of formula (I) it is desired to obtain, with a compound of formula (III): 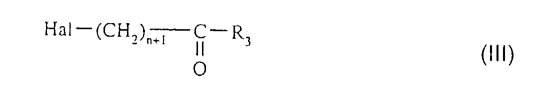 wherein n is as defined hereinabove and R₃ is as defined for formula (I) as a function of the meanings R₁, and Hal represents a halogen atom
to yield a compound of formula (IV): 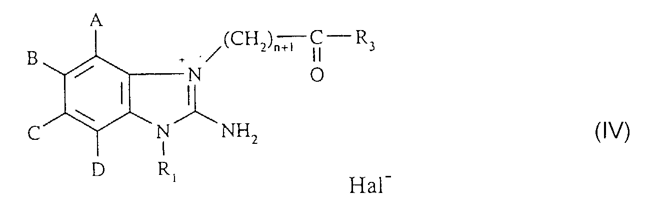 wherein Hal, A, B, C, D, R₁, n and R₃ are as defined hereinabove,
which, on heating, yields a compound of formula (I/A) : 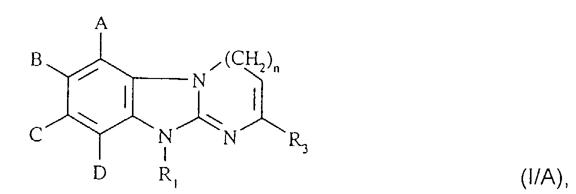 a particular case of the compounds of formula (I) wherein A, B, C, D, R₁, R₃ and n are as defined hereinabove, and X and R₂ on the one hand, and Y and Z on the other hand, together form a bond, and R₄ represents a hydrogen atom,
which compound of formula (I/A), when R₄ is other than hydrogen in the compound of formula (I) it is desired to obtain, is treated :
- when R₄ represents a G₄ grouping and Y and Z represent a bond in the compound of formula (I) it is desired to obtain,
with a compound of formula (V/A) :
Hal'₃-C - CO - Hal" (VA)
wherein Hal' and Hal" are the same or different and each represent a halogen atom, to yield a compound of formula (VI) : 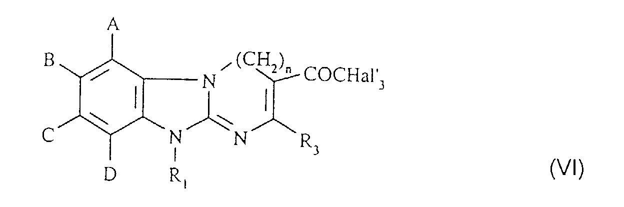 wherein A, B, C, D, R₁, n, R₃ and Hal'₃ are as defined hereinabove,
which compound of formula (VI) is treated with a compound of formula (VII) : 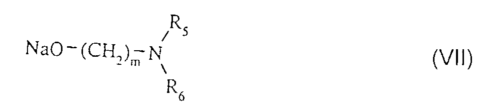 wherein m, R₅ and R₆ are as defined for the formula of the G₄ grouping as defined for formula (I),
to yield a compound of formula (I/C): 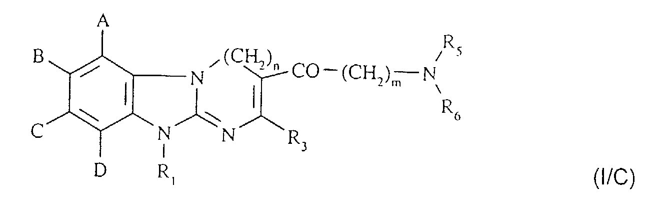 wherein A, B, C, D, R₁, R₃, R₅ and R₆ are as defined for formula (I),
Y and Z on the one hand, and X and R₂ on the other hand, together form a double bond and R₄ represents a G₄ grouping,
which compound of formula (I/A) or (I/C) is, if necessary, purified and, if desired, converted with an acid into a pharmaceutically acceptable salt.

12. Process for the preparation of compounds of the general formula (I) according to claim 1, characterised in that :
when X and R₂ together form a bond in the compound of formula (I) it is desired to obtain, there is used as starting material a compound of formula (II) : 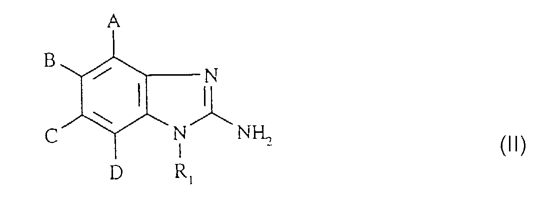 wherein A, B, C, D and R₁ are as defined hereinabove,
which is treated :
when R₄ represents a G₄ grouping and Y and Z represent a bond in the compound of formula (I) it is desired to obtain, with a compound of formula (VIII) :
Hal'''-(CH₂)ₘ-Hal₄ (VIII)
wherein Hal"' and Hal₄ are the same or different and each represent a halogen atom and m is as defined hereinabove, to yield a compound of formula (IX) : 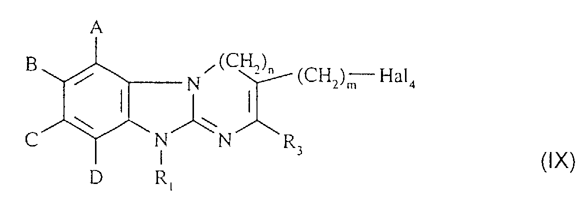 wherein A, B, C, D, R₁, n, p, R₃ and Hal₄ are as defined hereinabove,
which is then treated with a compound of formula (X) : 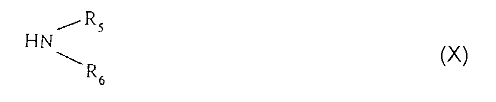 wherein R₅ and R₆ are as defined hereinabove,
to yield a compound of formula (I/D): 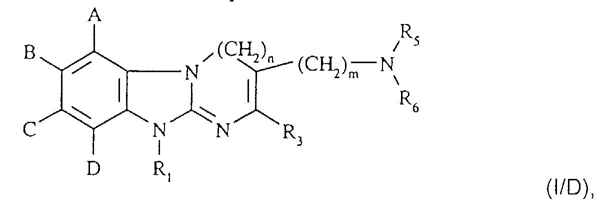 a particular case of the compounds of formula (I) wherein A, B, C, D, R₁ and n are as defined for formula I, and X and R₂ on the one hand, and Y and Z on the other hand, together form a bond and R₄ forms a G₁ grouping as defined for formula (II),
which compound of formula (I/D) is, if necessary, purified and, if desired, converted with an acid into a pharmaceutically acceptable salt.

13. Process for the preparation of compounds of the general formula (I) according to claim 1, characterised in that :
when X and R₂ together form a bond in the compound of formula (I) it is desired to obtain, there is used as starting material a compound of formula (II) : 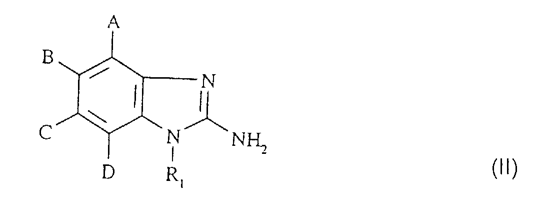 wherein A, B, C, D and R₁ are as defined hereinabove,
which is treated :
when R₄ represents a G₅ or G₇ grouping and Y and Z represent a bond in the compound of formula (I) it is desired to obtain, with a compound of formula (XI) :
Cl-CO-R₉ or Cl-CO-R₁₀ (XI),
according to the compound it is desired to obtain, wherein R₉ and R₁₀ are as defined hereinabove,
to yield a compound of formula (I/E): 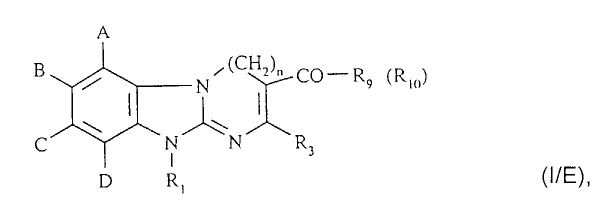 a particular case of the compounds of formula (I) wherein A, B, C, D, n and R₃ are as defined hereinabove, X and R₂ on the one hand, and Y and Z on the other hand, together form a bond and R₄ represents a G₅ or G₇ grouping as defined hereinabove,
which compound of formula (I/E) is, if necessary, purified and, if desired, converted with an acid into a pharmaceutically acceptable salt.

14. Process for the preparation of compounds of the general formula (I) according to claim 1, characterised in that when X and R₁ together form a bond in the compound of formula (I) it is desired to obtain,
there is used as starting material a compound of formula (XII) : 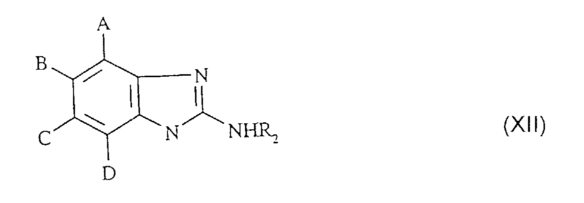 wherein A, B, C, D and R₂ are as defined hereinabove,
which :
* when Y and Z each represent a hydrogen atom in the compound of formula (I) it is desired to obtain, is treated
with a compound of formula (III/A) as defined hereinabove to yield, after heating, a compound of formula (I/G): 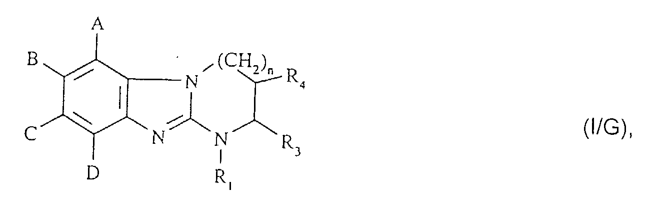 a particular case of the compounds of formula (I) wherein A, B, C, D, n, R₁, R₃ and R₄ are as defined hereinabove,
X and R₁ together form a bond and Y and Z each represent a hydrogen atom,
* when Y and Z form a bond in the compound of formula (I) it is desired to obtain, is treated
with a compound of formula (III) as defined hereinabove
to yield, after heating, a compound of formula (I/F): 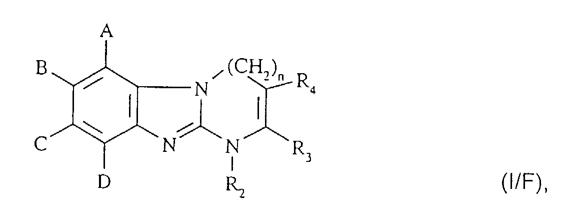 a particular case of the compounds of formula (I) wherein A, B, C, D, n, R₂ and R₃ are as defined hereinabove,
X and R₁ on the one hand, and Y and Z on the other hand, together form a bond and R₄ represents a hydrogen atom,
which compound of formula (I/F) may, as a function of the meaning of R₄ in the compound of formula (I) it is desired to obtain, be treated :
- in succession, as indicated hereinbefore, with a compound of formula (V/A) as defined hereinabove, then with a compound of formula (VII) as defined hereinabove,
- in succession, as indicated hereinbefore, with a compound of formula (VIII) as defined hereinabove, then with a compound of formula (X) as defined hereinabove,
- or with a compound of formula (Xl) as defined hereinabove,
to obtain a compound of formula (I/H) 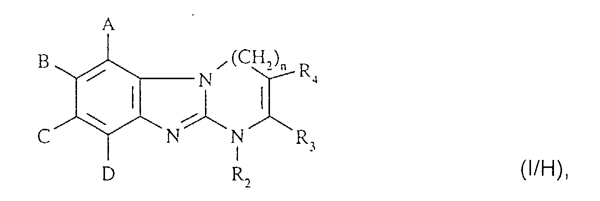 a particular case of the compounds of formula (I) wherein A, B, C, D, n, R₂ , R₃ and R₄ are as defined for formula (I),
X and R₁ on the one hand, and Y and Z on the other hand, each forming a bond,
which compounds of formula (I), (I/F), (I/G) and (I/H) are, if necessary, purified by a technique selected from chromatography and/or crystallisation and, if desired, converted with an acid into a pharmaceutically acceptable salt.

15. Process for the preparation of a compound of formula (I/B) according to claim 14, characterised in that a compound of formula (IV/B): 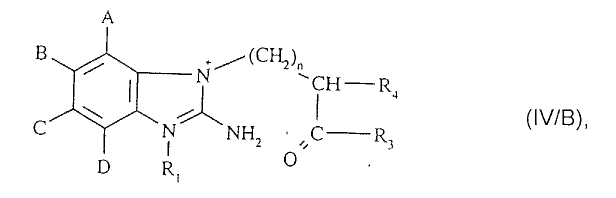 obtained by condensation of a compound of formula (II) with a compound of formula (III/B): 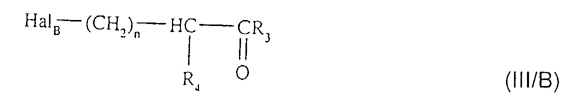 wherein HalB represents a halogen atom and n, R₃ and R₄ are as defined hereinabove, is subjected to reduction with an alkali metal mixed hydride
to form a compound of formula (IV/A) as defined hereinabove wherein the grouping P represents a hydroxy group, which is treated with a halogenation agent and then heated to yield a compound of formula (I/B) as defined hereinabove which, if necessary, is purified and, if desired, is converted with an acid into a pharmaceutically acceptable salt.

16. Process for the preparation of compounds of formula (I/B) according to claim 14, characterised in that there is used as starting material a compound of formula (XIII): 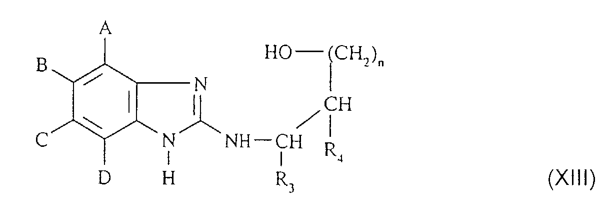 wherein A, B, C, D and n are as defined hereinabove,
which is treated :
either with a halogenation agent and then by heating
to yield a compound of formula (XIV): 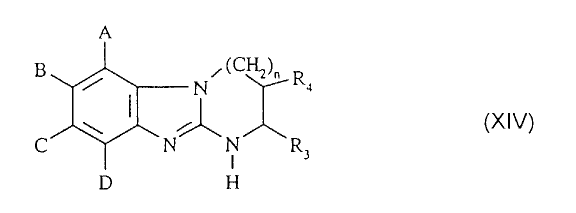 wherein A, B, C, D, R₃, R₄ and n are as defined hereinabove,
which is treated with a compound of formula (XV):
Hal-(CH₂)ₘ-CO-R₇ (XV)
wherein m and R₇ are as defined for formula (I) and Hal represents a halogen atom,
to yield a compound of formula (I/J) : 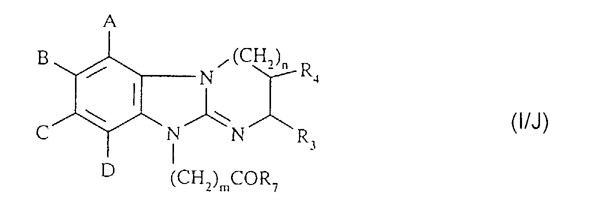 wherein A, B, C, D, m, n, R₃, R₄ and R₇ are as defined hereinabove, Y and Z representing a hydrogen atom, X and R₂ forming a bond and R₁ representing a G₂ grouping,
which compound of formula (I/J) may be subjected to the action of an alkali metal mixed hydride to yield a compound of formula (I/K): 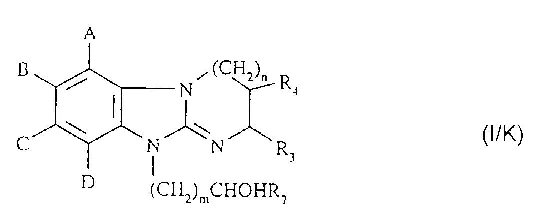 wherein A, B, C, D, m, n, R₃, R₄ and R₇ are as defined hereinabove, Y and Z each represent a hydrogen atom and X and R₂ together form a bond, and R₁ represents a G₃ grouping,
or with a compound of formula (XV) as defined hereinabove to enable a compound of formula (XVI) : 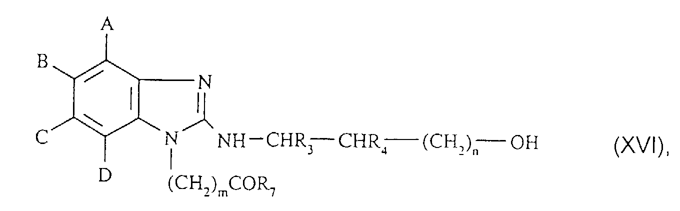 wherein A, B, C, D, R₃, R₄, n, m and R₇ are as defined hereinabove, to be obtained,
which is treated with a halogenation agent and by heating to yield a compound of formula (I/J) as defined hereinabove,
which compounds of formula (I/J) or (I/K) are, if necessary, purified and, if desired, converted with an acid into a pharmaceutically acceptable salt.

17. Process for the preparation of compounds of formula (I/J) or (I/K) as defined in accordance with claim 16, which comprises using as starting material a compound of formula (XIV), which is treated as described in claim 13.

18. Process for the preparation of compounds of formula (I/K) as defined in accordance with claim 16, which comprises treating a compound of formula (XIV) as defined in claim 16 with a compound of formula (XVII) :
Hal-(CH₂)ₘ-CHOH-R₇ (XVII)
wherein Hal, m and R₇ are as defined for formula (I), and, if necessary, purifying the resulting compound which, if desired, is converted with an acid into a pharmaceutically acceptable salt.

19. Process for the preparation of compounds of formula (I) according to claim 16, characterised in that there are used as starting materials compounds of formula (XVIII): 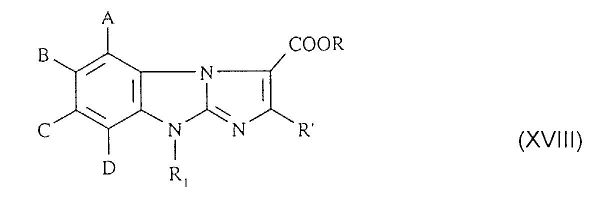 wherein A, B, C, D and R₁ are as defined hereinabove, R represents a lower alkyl group or a hydrogen atom and R' represents a lower alkyl group optionally substituted by a halogen atom or a hydroxy group.

20. Process for the synthesis of compounds of formula (I) wherein R₄ represents a CH₂NR₅R₆ grouping in which R₅ and R₆ are as defined in claim 1, characterised in that a compound of formula (I/A) as defined in claim 14 is treated by a Mannich reaction, using formaldehyde and an amine HNR₅R₆, which compound of formula (I) is purified, if necessary, and is converted with an acid into a pharmaceutically acceptable salt.

21. Process for the synthesis of compounds of formula (I) wherein R₂ represents a G₁ grouping as defined in claim 1, characterised in that there is used as starting material a compound of formula (XX): 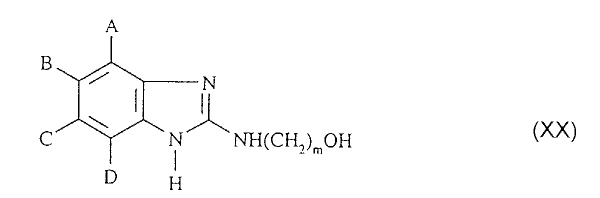 wherein A, B, C, D and m are as defined hereinabove,
which is treated with a compound of formula (XXI):
Hal-(CH₂)ₙ₊₁-COR₃ (XXI)
wherein n and R₃ are as defined hereinabove and Hal represents a halogen atom, to obtain a compound of formula (XXII): 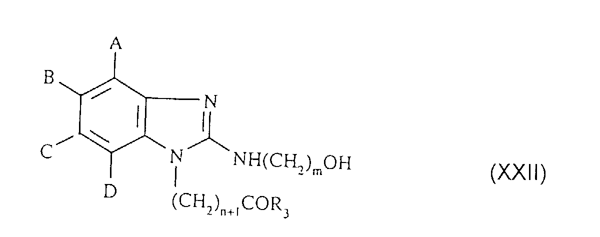 wherein A, B, C, D, R₃ and m are as defined for formula (I) which, on heating, results in the formation of a compound of formula (XXIII): 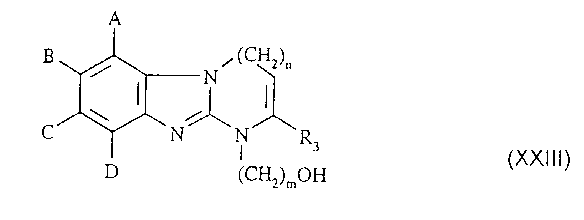 wherein A, B, C, D, m, n and R₃ are as defined for formula (I),
which is treated :
a) with a halogenation agent,
b) with an amine of formula HNR₅R₆,
to enable a compound of formula (I/M): 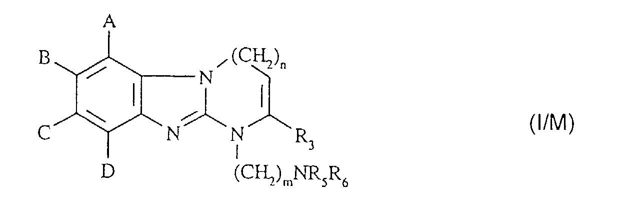 to be obtained, a particular case of the compounds of formula (I) wherein A, B, C, D, m, n, R₃, R₅ and R₆ are as defined for formula (I),
wherein R₁ and X, and Y and Z, each represent a bond and R₂ represents a G₁ grouping,
which, if necessary, is purified and, if desired, is converted with an acid into a pharmaceutically acceptable salt.

22. Pharmaceutical composition comprising as active ingredient at least one compound according to any one of claims 1 to 9 in combination with one or more pharmaceutically acceptable excipients or carriers.

23. Pharmaceutical composition according to claim 22 for use in the treatment of diabetes and/or its cardiovascular complications.
